(19) Europäisches Patentamt
European Patent Office
Office européen des brevets

(11) Veröffentlichungsnummer: **0 075 169**
**B1**

# (12) EUROPÄISCHE PATENTSCHRIFT

(45) Veröffentlichungstag der Patentschrift: **01.06.88**

(51) Int. Cl.[4]: **C 07 D 498/04**, A 61 K 31/535 // (C07D498/04, 265:00, 205:00)

(21) Anmeldenummer: **82108186.6**

(22) Anmeldetag: **06.09.82**

(54) **Beta-Lactam Antibiotica, Verfahren zu ihrer Herstellung sowie ihre Verwendung als Arzneimittel.**

(30) Priorität: **17.09.81 DE 3137038**

(43) Veröffentlichungstag der Anmeldung: **30.03.83 Patentblatt 83/13**

(45) Bekanntmachung des Hinweises auf die Patenterteilung: **01.06.88 Patentblatt 88/22**

(84) Benannte Vertragsstaaten: **AT BE CH DE FR GB IT LI LU NL SE**

(56) Entgegenhaltungen:
**DE - A - 2 633 317**
**DE - A - 2 658 718**
**DE - A - 2 720 579**
**DE - A - 2 720 580**
**DE - A - 2 739 448**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Boberg, Michael, Dr., Bergerheide 101a, D-5600 Wuppertal 1 (DE)**
Erfinder: **Häbich, Dieter, Dr., Fuhlrott Strasse 19, D-5600 Wuppertal 1 (DE)**
Erfinder: **Metzger, Karl Georg, Dr., Pahlkestrasse 15, D-5600 Wuppertal 1 (DE)**
Erfinder: **Naab, Paul, Dr., Schenkstrasse 32 b, D-5600 Wuppertal 21 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 075 169 B1

ACTORUM AG

**Beschreibung**

Die vorliegende Erfindung betrifft β-Lactam-Antibiotica, ein Verfahren zu ihrer Herstellung sowie ihre Verwendung als Arzneimittel, insbesondere als antibakterielle Mittel und als Mittel zur Förderung des Wachstums und zur Verbesserung der Futterverwertung bei Tieren.

Es ist bereits bekannt geworden, dass bestimmte α-(Imidazolidin-2-oxo-1-yl-carbonylamino)-benzylpenicilline und entsprechende Cephalosporine mit einer Imidazolidin-2-oxo-1-yl-carbonyl-amino-Seitenkette antibakteriell wirksam sind (DE-OS 2 528 078, 2 525 541 und 2 732 283).

Cephalosporin-Analoge mit einem Sauerstoffatom anstelle des Schwefelatoms im Sechsring sind im Journal of Heterocyclic Chemistry, Bd. 5 (1968), S. 779, Canadian Journal of Chemistry, Bd. 52 (1974), S. 3996 und J. Am. Chem. Soc., Bd. 96 (1974), S. 7582 beschrieben.

Überraschenderweise zeigen die Oxacepheme der Formel I überlegene Eigenschaften hinsichtlich Wirkung im Vergleich zu den in den obengenannten Offenlegungsschriften konkret beschriebenen Penicillinen und Cephalosporinen.

Die vorliegende Erfindung betrifft daher β-Lactam-Antibiotika der Formel

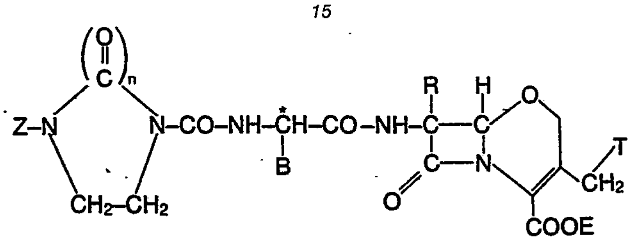

(I)

in welcher

R für Wasserstoff oder Methoxy steht,

n 1 und 2 ist

Z für die Gruppe 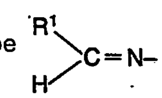 steht,

worin $R^1$ für gegebenenfalls durch einen oder mehrere, gleiche oder verschiedene Reste aus der Gruppe bestehend aus Halogen, Amino, Mono-$C_1$–$C_6$-alkylamino, Di-$C_1$–$C_6$-alkylamino, Pyrrolidyl, Piperidyl, HCO–NH-, $C_1$–$C_6$-Alkyl-CO–NH-, H–CO–N($C_1$–$C_6$-alkyl)-, $C_1$–$C_6$-Alkyl-CO–N($C_1$–$C_6$-alkyl)-, ($C_1$–$C_6$-Alkyl)$_2$C = N-, $C_1$–$C_6$-Alkyl–$SO_2$–NH-, HO–$SO_2$–NH-, HO–$SO_2$–N($C_1$–$C_6$-alkyl)-, Amidino, ($C_1$–$C_6$-Alkyl)$_2$–N–CH = N-,

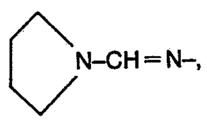

Guanido, Nitro, Azido, Hydroxy, $C_1$–$C_6$-Alkyloxy-, H–CO–O-, $C_1$–$C_6$-Alkyl-CO–O-, $C_1$–$C_6$-Alkyl-O–CO–O-, $H_2$N–CO–O-, $C_1$–$C_6$-Alkyl-NH–CO–O-, ($C_1$–$C_6$-Alkyl)$_2$N–CO–O-,

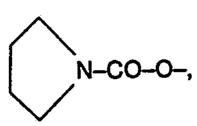

$H_2$N–$SO_2$–O-, $C_1$–$C_6$-Alkyl-NH–$SO_2$–O-, ($C_1$–$C_6$-Alkyl)$_2$N–$SO_2$–O-, HOOC-, $H_2$N–CO-, ($C_1$–$C_6$-Alkyl)$_2$N–CO-, OHC-, HO–$SO_2$–O-, HS-, $C_1$–$C_6$-Alkyl-S-, $C_1$–$C_6$-Alkyl-S-,
O

$HO_3$S-, $C_1$–$C_6$-Alkyl-$SO_2$-, $H_2$N–$SO_2$-, $C_1$–$C_6$-Alkyl-NH–$SO_2$-, ($C_1$–$C_6$-Alkyl)$_2$-N–$SO_2$-, HO–$SO_2$–S-, geradkettiges oder verzweigtes Alkyl mit 1 bis 6 Kohlenstoffatomen, Phenyl und Phenoxy, substituiertes Aryl mit 6 bis 10 Kohlenstoffatomen im Arylteil oder gegebenenfalls durch einen oder mehrere, gleiche oder verschiedene Reste aus der Gruppe bestehend aus $C_1$–$C_6$-Alkyl, Trifluormethyl, Halogen, Amino, $C_1$–$C_6$-Alkylamino, Di-$C_1$–$C_6$-alkylamino, Formylamino, Acetylamino, $CH_3$–O–CO–NH-, $C_2H_5$O–CO–NH-, $CH_3$–$SO_2$–NH-, Hydroxy, Methoxy, Ethoxy, Methylthio, Ethylthio, $CH_3$–$SO_2$-, $CH_3$–SO-, HOOC-, $HO_3$S-, HCO-, $C_1$–$C_6$-Alkyl-CO-, $C_1$–$C_6$-Alkyl-O–CO- und CN am Ringkohlenstoff oder gegebenenfalls durch Reste aus der Gruppe bestehend aus $C_1$–$C_6$-Alkyl, –C≡N, –CHO, –COO–$C_1$–$C_6$-Alkyl, –CO–$NH_2$, –CO–NH–$C_1$–$C_6$-Alkyl und –CO-alkyl, am Ringstickstoff substituierte heteroparaffinische, heteroaromatische oder heteroolefinische 5- bis 7-gliedrige Ringe mit 1 bis 3 gleichen oder verschiedenen Heteroatomen aus der Gruppe bestehend aus Sauerstoff, Schwefel oder Stickstoff steht,

oder wenn n = 1 ist,

Z für Cycloalkyl mit 3 bis 7 Ringkohlenstoffatomen oder für Furyl- Pyryl, 1-Methylpyrryl-2 oder -3, Thienyl, Oxazolyl (gebunden in 2-, 4- oder 5-Stellung), Thiazolyl (gebunden in 2-, 4- oder 5-Stellung), Isoxazolyl (gebunden in 3-, 4- oder 5-Stellung), Isothiazolyl (gebunden in 3-, 4- oder 5-Stellung), 1,2,5-Oxadiazolyl-3-, 1,2,5-Thiadiazolyl-3-, 1,3,4-Oxadiazolyl-2-, 1,3,4-Thiadiazolyl-2-, 1,2,4-Oxadiazolyl (gebunden in 3- oder 5-Stellung), 1,2,4-Thiadiazolyl (gebunden in 3- oder 5-Stellung), Pyrazolyl (gebunden in 3-, 4- oder 5-Stellung), 1,2,4-Triazolyl (gebunden in 3- oder 5-Stellung), Tetrazolyl, Pyridyl (gebunden in 2-, 3- oder 4-Stellung), Pyridazinyl (gebunden in 3- oder 4-Stellung), Pyrimidinyl (gebunden in 2-, 4- oder 5-Stellung, α-Pyronyl (gebunden in 3-, 4-, 5- oder 6-Stellung), γ-Pyronyl (gebunden in 2- oder 3-Stellung) und Benzthiazolyl-2- steht, wobei Z gegebenenfalls durch $C_1$–$C_6$-Alkyl, $C_1$–$C_6$-Alkyliden, Vinyl, Propenyl, Allyl, Isopropenyl, $C_1$–$C_6$-Alkoxymethyl, $C_1$–$C_6$-Alkylthiomethyl, Trifluormethyl, Hydroxymethyl, Formyl, $C_1$–$C_6$-Alkanoyl, $C_1$–$C_6$-Alkanoyloxymethyl, Benzyl, Phenyl, Cyanomethyl, $CH_3$–NH–CO–$CH_2$-, $(CH_3)_2$N–CO–$CH_2$-, $C_1$–$C_6$-Alkoxycarbonyl, Carboxy, Cyano, Hydroxy, $C_1$–$C_6$-

Alkanoyloxy, $C_1$–$C_6$-Alkoxy, Benzyloxy, Halogen, Mercapto, $C_1$–$C_6$-Alkylthio, $C_1$–$C_6$-Alkylsulfinyl, $C_1$–$C_6$-Alkylsulfonyl, $CH_3$–CO–NH-, $CH_3$–CO–N($CH_3$)-, $CH_3$–$SO_2$–NH- und $CH_3$–$SO_2$–N($CH_3$)– mono-di- oder trisubstituiert sein kann,

B für gegebenenfalls durch Halogen, Hydroxy, Alkyl mit 1 bis 6 Kohlenstoffatomen, Cyano und Methylsulfonyl substituiertes Phenyl oder Cyclohexadienyl steht, oder für einen heterocyclischen Rest aus der Gruppe bestehend aus Pyrazolyl, Imidazolyl, Oxazolyl, Oxadiazolyl, 2-Amimo- und 2-Oxo-$\Delta^4$-thiazolinyl, Tetrazolyl, Sydnonyl sowie Furyl, Thienyl-, Pyrrolyl, Thiazolyl-, Isothiazolyl-, Isoxazolyl- und Thiadiazolyl steht, wobei der heterocyclische Rest ein- oder mehrfach durch Halogen, Alkyl mit 1 bis 6 Kohlenstoffatomen, Cyano, Sulfo und Methylsulfonyl substituiert sein kann,

T $C_1$–$C_4$-Alkyl-CO–O-, Pyridinium, Aminopyridinium, Carbamoylpyridinium, Carbamoyloxy, die Gruppe –S-Phenyl, welche durch Halogen, Amino, $C_1$–$C_6$-Alkylamino, Di-$C_1$–$C_6$-alkylamino, $C_1$–$C_6$-Alkyl, Cycloalkyl mit 3 bis 7 Kohlenstoffatomen, $C_1$–$C_6$-Alkyloxy, Trifluormethyl, Phenyl, Benzyl und Acylamino mit 2 bis 5 Kohlenstoffatomen substituiert sein kann, oder die Gruppe –S-Het bedeutet, in welcher Het für einen in der β-Lactamchemie üblichen heterocyclischen 5- oder 6-gliedrigen Ring steht,

E für Wasserstoff, eine pharmazeutisch verwendbare Estergruppierung, ein salzbildendes Kation oder eine geeignete Schutzgruppe steht, oder eine negative Ladung bedeutet, wenn in T ein quartärer Stickstoff enthalten ist,
wobei die Verbindungen der Formel I bezüglich des Chiralitätszentrums C̊ in den beiden möglichen R- und S-Konfigurationen sowie als Gemische der daraus resultierenden Diastereomeren vorliegen können, und wobei die Verbindungen der Formel I, falls Z für die Gruppe

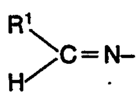

steht, bezüglich der Iminogruppe sowohl in der syn-Form als auch in der anti-Form vorliegen können und wobei die Verbindungen der Formel I auch in den verschiedenen Hydratformen vorliegen können, und die nichttoxischen, pharmazeutisch verträglichen Salze dieser Verbindungen.

Bevorzugt sind Verbindungen der Formel I, in der

R für Methoxy steht

Z für die Gruppe

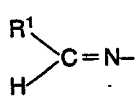

steht, worin

$R^1$ gegebenenfalls durch Halogen (insbesondere Fluor, Chlor und Brom), Alkyl mit 1 bis 4 Kohlenstoffatomen (insbesondere Methyl), Alkoxy mit 1 bis 4 Kohlenstoffatomen, (insbesondere Methoxy), Nitro, 1 oder 2 Hydroxygruppen, S-Alkyl mit 1 bis 4 Kohlenstoffatomen (insbesondere S-Methyl), Alkylsulfonyl mit 1 bis 4 Kohlenstoffatomen (insbesondere Methylsulfonyl) oder $CH_3OOC$- substituiertes Phenyl bedeutet oder gegebenenfalls durch Halogen (insbesondere Chlor oder Brom), Alkyl oder Alkoxycarbonyl mit 1 bis 4 Kohlenstoffatomen oder S-Alkyl ($C_1$–$C_4$), vorzugsweise in der 4- oder 5-Stellung substituiertes Furyl oder Thienyl bedeutet, wobei der Furyl- und Thienylring bevorzugt in 2- und 3-Stellung gebunden ist; oder Pyridyl (vorzugsweise Pyridyl-3); oder

Z für Cyclopropyl, Furyl; Pyridyl; Thienyl, Benzthiazolyl-2- oder für 1,3,4-Thiadiazolyl-2-, welches in 5-Stellung durch sec.-Butyl, Trifluormethyl, Methylthio, i-Propylthio oder Methylsulfonyl substituiert sein kann, steht,

B für Phenyl, Hydroxyphenyl oder Cyclohexadienyl oder für Furyl, Thienyl, Pyrrolyl, Imidazolyl, Thiazolyl, Isothiazolyl, Isoxazolyl, Thiadiazolyl steht und

n 1 oder 2 ist,

T für –$OCOCH_3$, für gegebenenfalls durch Alkyl mit 1 bis 4 Kohlenstoffatomen oder $CF_3$ oder $CH_2COOH$ substituiertes Thiadiazolylthio oder Tetrazolylthio, für Pyridinium, Aminopyridinium, Carbamoylpyridinium oder Carbamoyloxy oder 1,2,5,6-Tetrahydro-2-methyl-5,6-dioxo-as-triazin-3-yl-thio, steht und

C* in der D- = R-Konfiguration vorliegt.

Weiterhin wurde gefunden, dass man die β-Lactam-Antibiotica der Formel I erhält, wenn man Verbindungen der Formel II

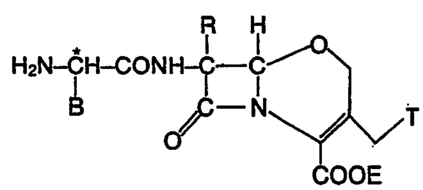

(II)

in welcher R, B, C*, T und E die oben angegebene Bedeutung haben und die auch an der Carboxyl- und Aminogruppe oder nur der Carboxylgruppe silyliert sein können, mit Verbindungen der Formel III

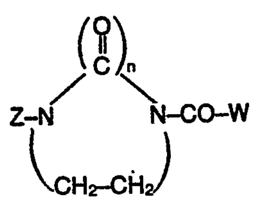

(III)

in welcher

Z und n die oben angegebene Bedeutung haben und

W für Halogen, Azid oder eine andere nukleofuge Abgangsgruppe steht,

in Gegenwart eines Lösungsmittels und gegebenenfalls eines Säurebindemittels bei Temperaturen von etwa −70 °C bis etwa +50 °C umsetzt und die erhaltenen β-Lactam-Antibiotica gegebenenfalls in ihre nichttoxischen, pharmazeutisch verträglichen Salze überführt oder aus den erhaltenen Salzen die freien Säuren herstellt.

Die erfindungsgemässen Verbindungen der Formel (I), in welcher R für Methoxy steht, sind ebenfalls herstellbar durch Alkoxylierung der entsprechenden Wasserstoffderivate (R = H), wobei es vorteilhaft ist, bei der Alkoxylierung solche Verbindungen zu verwenden, in denen E eine geeignete Schutzgruppe bedeutet.

Weiterhin kann man die erfindungsgemässen Verbindungen der Formel (I) herstellen in dem man eine Verbindung der Formel (V)

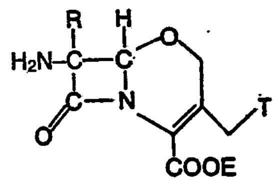

(V)

in der R, T und E die oben angegebene Bedeutung haben mit einem Acylierungsmittel der Formel VI umsetzt

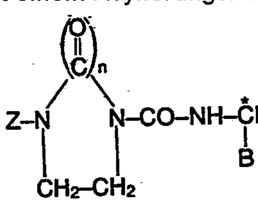

(VI)

in der Z, n, C* und B die oben angegebene Bedeutung haben und W' OH oder eine reaktive Abgangs-Gruppe ist.

Die 7-Aminogruppe der Verbindung V kann vor der Reaktion z.B. in Form einer Isocyan-, Isocyanat-, 1-Halogenalkylidenamino-, 1-Alkoxyalkylidenamino-, Silylamino- oder Enamingruppe aktiviert werden. Als Verbindung (VI) eignen sich z.B. freie Säuren, Säurehalogenide, Säureanhydride, aktive Ester, aktive Amide und Ketene mit dem gewünschten Acylrest. Die Acylierung kann gegebenenfalls in Gegenwart einer Base, wie Triethylamin, Pyridin oder Natriumhydrogencarbonat, Molekularsieben, Carbodiimiden, z.B. Dicyclohexylcarbodiimid, Epoxiden, z.B. Propylenoxid oder Butylenoxid, oder Enzymen, durchgeführt werden. Die Umsetzung kann nach der Säurechlorid-, Säureanhydrid-, Carbodiimid- oder Aktivestermethode erfolgen.

Ein weiteres Verfahren zur Herstellung der erfindungsgemässen Verbindungen der Formel (I) besteht darin, dass man eine Verbindung der Formel VII

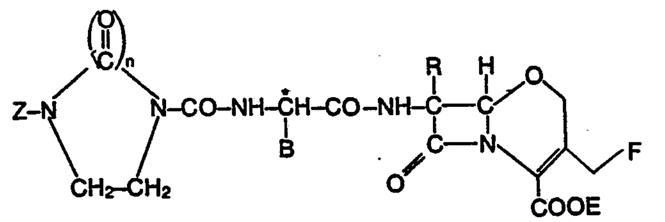

(VII)

in der Z, n, C*, B, R und E die oben angegebene Bedeutung haben und F eine abspaltbare Gruppe darstellt, mit einer geeigneten Substanz der allgemeinen Formel VIII $R^2M$ (VIII) oder mit einem tertiären Amin so umsetzt, dass die in Anspruch 1 genannten Verbindungen entstehen.

In der Formel (VII) bedeutet F einen Substituenten, welcher leicht durch ein nucleophiles Mittel ersetzt werden kann, z.B. ein Halogenatom, sowie Formyloxy, Acetoxy, Propionyloxy, Butyryloxy, Pivaloyloxy, $-OCOCHCl_2$ oder dgl.; eine Acylcarbonyloxygruppe, wie Benzoyloxy, Naphthoyloxy, oder dgl.; eine Arylcarbonylthiogruppe, wie Benzoylthio, Naphthoylthio oder dgl.; eine Carbamoylgruppe; eine heteroaromatische Amin-N-oxidthio-Gruppe mit einer Mercaptogruppe an dem der N-oxid-Gruppe benachbarten Kohlenstoffatom des Moleküls, wie Pyridin-1-oxid-2-ylthio, Pyridazin-1-oxid-6-ylthio oder dgl. Jede der Gruppen kann durch einen oder mehrere Substituenten, z.B. Halogen, Nitro, Alkyl, Alkoxy, Alkylthio, Acyl oder dgl. substituiert sein.

In der Formel (VIII) bedeutet $R^2$ eine organische Gruppe, welche über O, N oder S angeknüpft ist.

In der Formel (VIII) bedeutet M ein Wasserstoffatom, ein Alkalimetall oder ein Erdalkalimetall. Das eingesetzte tertiäre Amin kann Pyridin, Chinolin, Isochinolin, Pyrimidin oder dgl. sein. Diese tertiären Amine können durch einen oder mehrere Substituenten, wie Halogen, niederes Alkyl, Carbamoyl oder dgl. substituiert sein.

In den allgemeinen Formeln steht als gegebenenfalls substituiertes Aryl $R^1$ Aryl mit 6 bis 10 Kohlenstoffatomen im Arylteil. Beispielhaft seien gegebenenfalls substituiertes Phenyl oder Naphthyl genannt. Substituenten im Phenylring stehen in o-, m- oder p-Stellung.

Als gegebenenfalls substituiertes Heterocyclyl $R^1$ steht heteroparaffinische, heteroaromatische und heteroolefinische 5- bis 7-gliedrige, vorzugsweise 5- oder 6-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Als Beispiele seien gegebenenfalls substituiertes Thienyl, Furyl, Oxazolyl, Isoxazolyl, Thiazolyl, Isothiazolyl, Pyrrolyl, Imidazolyl, Pyrazolyl, Oxidiazolyl, Thiadiazolyl, Triazolyl, Oxtriazolyl, Thiatriazolyl, Tetrazolyl, Pyridyl, Pyrazinyl, Pyrimidinyl, Tetrahydrofuranyl, Dioxanyl, Pyrrolidinyl, Piperidinyl, Morpholinyl, Pyronyl-2 und -4.

Aryl $R^1$ können einen oder mehrere, vorzugsweise 1 bis 3, insbesondere 1 oder 2 gleiche oder verschiedene Reste $R^3$ tragen. Ganz besonders bevorzugt sind die genannten Reste $R^1$ unsubstituiert oder enthalten einen Substituenten $R^3$.

Heterocyclyl $R^1$ kann einen oder mehrere, vorzugsweise 1 bis 3, insbesondere 1 oder 2 gleiche

oder verschiedene Reste $R^4$ tragen. Ganz besonders bevorzugt ist Heterocyclyl $R^1$ unsubstituiert oder enthält einen Substituenten $R^4$.

Bei den folgenden Darlegungen bedeutet der Ausdruck «Niederalkyl» überall, auch in Verbindung mit anderen Atomen oder Gruppen (z.B. Niederalkoxy, HCON-(Niederalkyl), usw.) geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n- und i-propyl, n-, i- und t-Butyl genannt. «Niederalkyl» kann durch 1 bis 5, insbesondere 1 bis 3 gleiche oder verschiedene Halogenatome, wobei als Halogenatome vorzugsweise Fluor, Chlor und Brom, insbesondere Fluor steht, substituiert sein. Beispielhaft seien Trifluormethyl, Chlor-di-fluormethyl, Brommethyl, 2,2,2-Tri-fluorethyl und Pentafluorethyl genannt.

$R^3$ bedeutet vorzugsweise Fluor, Chlor, Brom und Jod, insbesondere Fluor, Chlor und Brom; Methylamino, Ethylamino, insbesondere Methylamino, Dimethylamino, Diethylamino, insbesondere Dimethylamino, $CH_3-CO-NH-$; $H-CO-N(CH_3)-$, $H-CO-N(C_2H_5)-$, $CH_3-CO-N(CH_3)-$, $CH_3-SO_2-NH-$, $C_2H_5-SO_2-NH-$, $HO-SO_2-N(CH_3)-$, $HO-SO_2-N(C_2H_5)-$, insbesondere $(CH_3)_2N-CH=N-$, $CH_3-O-$, $C_2H_5-O-$, insbesondere $CH_3O-$, $CH_3-CO-O$, $C_2H_5-CO-O-$, $(CH_3)_3C-CO-O$, $CH_3-O-CO-O-$, $C_2H_5-O-CO-O-$, $(CH_3)_3C-O-CO-O$, $CH_3-NH-CO-O-$, $C_2H_5-NH-CO-O-$, $(CH_3)_2N-CO-O-$, $(C_2H_5)_2N-CO-O-$, $CH_3-NH-SO_2-O-$, $C_2H_5-NH-SO_2-O-$, $(CH_3)_2N-SO_2-O-$, $(C_2H_5)_2N-SO_2-O-$, $(CH_3)_2N-CO$, $(C_2H_5)_2N-CO-$, $CH_3-S-$, $CF_3-S-$, $C_2H_5-S-$, $(CH_3)_2-CH-S-$, $CH_3-\underset{\overset{\|}{O}}{S}-$, $C_2H_5-\underset{\overset{\|}{O}}{S}-$,

$HO_3S-$, $CH_3-SO_2-$, $CF_3SO_2-$, $C_3H_5-SO_2-$, $CH_3-NH-SO_2-$, $C_2H_5-NH-SO_2-$, $(CH_3)_2N-SO_2-$, $(C_2H_5N-SO_2-$, $N-SO_2-$, Methyl, Ethyl, Propyl, Isopropyl, n-Butyl, sek.-Butyl oder tert.-Butyl, insbesondere Methyl.

Im Falle, dass $R^4$ an einem oder mehreren Kohlenstoffatomen im Heterocyclyl $R^1$ steht, bedeutet $R^4$ vorzugsweise Methyl, Ethyl, Isopropyl, insbesondere Methyl, Fluor, Chlor, Brom, $CH_3-NH-$, $C_2H_5-NH-$, $(CH_3)_2N-$, $(C_2H_5)_2N-$, $CH_3-CO-$, $CH_3-O-CO-$ und $C_2H_5O-CO-$.

Im Falle, dass $R^4$ in einem stickstoffhaltigen Heterocyclyl $R^1$ als Substituent an einem oder mehreren Stickstoffatomen steht, bedeutet $R^4$ vorzugsweise Methyl, Ethyl, Propyl, Isopropyl, insbesondere Methyl und Ethyl; $-COO-CH_3$, $-COOC_2H_5$, $-COOCH(CH_3)_2$, $-COO-C(CH_3)_3$, $-CO-NH-CH_3$, $-CO-NH-C_2H_5$, $-CO-NH-CH(CH_3)_2$, $-CO-CH_3$, $-CO-C_2H_5$ und $-CO-CH(CH_3)_2$.

In den Formeln bedeuten gegebenenfalls substituiertes Cycloalkyl, Z solches mit 3 bis 7, vorzugsweise 3 bis 6 Ringkohlenstoffatomen. Beispielsweise seien aufgeführt: Cyclopropyl, Cyclobutyl, Cyclopentyl, Cyclohexyl.

Als gegebenenfalls substituierte heterocyclische 5- oder 6-gliedrige Ringe Z seien beispielhaft aufgeführt: Furyl (vorzugsweise Furyl-2), Pyrryl (vorzugsweise 2-Pyrryl); 1-Methylpyrryl-2 oder -3; Thienyl (vorzugsweise 2-Thienyl); Oxazolyl (gebunden in 2-, 4- oder 5-Stellung); Thiazolyl (gebunden in 2-, 4- oder 5-Stellung); Isoxazolyl (gebunden in 3-, 4- oder 5-Stellung); Isothiazolyl (gebunden in 3-, 4- oder 5-Stellung) 1,2,5-Oxadiazolyl-3-; 1,2,5-Thiadiazolyl-3-; 1,3,4-Oxadiazolyl-2-; 1,3,4-Thiadiazolyl-2-; 1,2,4-Oxadiazolyl (gebunden in 3- oder 5-Stellung); 1,2,4-Thiadiazolyl (gebunden in 3- oder 5-Stellung); Pyrazolyl (gebunden in 3-, 4- oder 5-Stellung); 1,2,4-Triazolyl (gebunden in 3- oder 5-Stellung); Tetrazolyl; Pyridyl (gebunden in 2-, 3- oder 4-Stellung); Pyridazinyl (gebunden in 3- oder 4-Stellung); Pyrimidinyl (gebunden in 2-, 4- oder 5-Stellung); α-Pyronyl (gebunden in 3-, 4-, 5- oder 6-Stellung); γ-Pyronyl (gebunden in 2- oder 3-Stellung) und Benzthiazolyl-2-, Thiazolyl (gebunden in 2-, 4- oder 5-Stellung).

Cycloalkyl, Z sowie die heterocyclischen 5- oder 6-gliedrigen Ringe Z können mono-, di- oder tri-, vorzugsweise mono- oder di-substituiert, insbesondere mono-substituiert sein. Als Substituenten seien beispielhaft genannt: Methyl, Ethyl, Propyl, Isopropyl, t-Butyl, vorzugsweise Methyl, Methyliden, Ethyliden und Isopropyliden, Vinyl, Propenyl, Allyl, Isopropenyl, Methoxymethyl, Methylthiomethyl, Acetyl, Acetoxymethyl und Phenyl, Methoxycarbonyl, Ethoxycarbonyl, Acetoxy, Niederalkoxy, insbesondere Methoxy und Ethoxy, Fluor, Chlor, Brom, vorzugsweise Chlor, Methylthio, Ethylthio, Methylsulfinyl, Ethylsulfinyl, Methylsulfonyl und Ethylsulfonyl.

Bevorzugt sind die Cycloalkylreste Z unsubstituiert, wobei insbesondere der unsubstituierte Cyclopropylrest hervorgehoben sei.

Die heterocyclischen 5- oder 6-gliedrigen Ringe Z sind ganz besonders bevorzugt unsubstituiert oder mono-substituiert, wobei als bevorzugte Substituenten Niederalkyl, Trifluormethyl, Niederalkylsulfonyl, Niederalkylthio und Trifluormethyl erwähnt seien. Als besonders bevorzugte heterocyclische Ringe Z seien genannt: Pyridyl-2, -3 oder -4; Furyl, 5-Methylthio-(1,3,4-Thiadiazol)-2-yl, 5-i-Propylthio-(1,3,4-Thiadiazol)-2-yl, 5-Methylsulfonyl(1,3,4-Thiadiazol)-2-yl, 5-Trifluormethyl-(1,3,4-Thiadiazol)2-yl, 5-Trifluormethyl-(1,3,4-Thiadiazol)-2-yl, 5-sec.-Butyl (1,3,4-Thiadiazol)-2-yl und Benzthiazolyl-2-.

B steht für einen gesättigten oder ungesättigten, vorzugsweise jedoch ungesättigten, gegebenenfalls substituierten heterocyclischen Rest, der 1 bis 4, vorzugsweise jedoch 1 bis 3 gleiche oder verschiedene Heteroatome aus der Reihe Sauerstoff, Schwefel und/oder Stickstoff enthalten kann.

Als geeignete Reste dieser Art seien beispielhaft genannt: Pyrazolyl, Imidazolyl, Oxazolyl, Oxadizolyl, 2-Amino- und 2-Oxo-$\Delta^4$-thiazolinyl, Tetrazolyl, Sydnonyl sowie Furyl-, Thienyl-, Pyrrolyl, Thiazolyl-, Isothiazolyl-, Isoxazolyl- und Thiadiazolyl-Reste. Der heterocyclische Rest B kann einen oder mehrere, vorzugsweise 1 bis 2, insbesondere gleiche oder verschiedene Substituenten tragen. Als Substituenten seien beispielhaft Halogen, wie Fluor, Chlor oder Brom, vorzugsweise

Fluor und Chlor, Alkyl mit 1 bis 6, vorzugsweise 1 bis 4, insbesondere 1 oder 2 Kohlenstoffatomen; Cyano, Sulfo und Methylsulfonyl aufgeführt.

Phenyl B kann einen oder mehrere, vorzugsweise 1 bis 3, insbesondere 1 oder 2 gleiche oder verschiedene Substituenten tragen. Die Substituenten können in o-, m- und/oder p-Stellung stehen. Vorzugsweise befindet sich ein Substituent in p- oder m-Stellung. Als Substituenten seien beispielhaft Halogen, wie Fluor, Chlor, Brom und Jod, vorzugsweise Fluor, Chlor und Brom; Alkyl mit 1 bis 6, vorzugsweise 1 bis 4, insbesondere 1 oder 2 Kohlenstoffatomen; Cyano und Methylsulfonyl aufgeführt. Insbesondere seien als substituierte Phenylreste B der Hydroxyphenylrest (vorzugsweise p-Hydroxyphenyl), der Methylphenylrest (vorzugsweise p-Methylphenyl), der Cyanophenylrest (vorzugsweise m- und p-Cyanophenyl), der Methylsulfonylrest (vorzugsweise p-Methylsulfonylphenyl) und der Fluorphenylrest (vorzugsweise o-Fluorphenyl und m-Fluorphenyl) genannt.

In der Definition von T bedeutet Alkyl in Alkyl-CO-O- vorzugsweise Alkyl mit 1 bis 4, insbesondere 1 oder 2 Kohlenstoffatome. Beispielhaft seien Methyl und Ethyl genannt, wobei Methyl besonders bevorzugt ist.

Der heterocyclische Ring Het in -S-Het (Definition von T) besteht aus 5 oder 6 Ringgliedern und enthält 1 bis 4, vorzugsweise 3 bis 4 gleiche oder verschiedene Heteroatome, wobei als Heteroatome Sauerstoff, Schwefel und Stickstoff stehen. Bevorzugt ist der heterocyclische Ring ungesättigt und enthält besonders bevorzugt 2 Doppelbindungen. Der heterocyclische Ring kann einen oder mehrere, vorzugsweise 1 oder 2, insbesondere einen Substituenten enthalten. Als Substituenten seien beispielhaft aufgeführt: Halogen, wie Fluor, Chlor und Brom, vorzugsweise Chlor und Brom, Amino, Niederalkylamino, Diniederalkylamino, Niederalkyl, Cycloalkyl (mit 3 bis 7, vorzugsweise 5 oder 6 Kohlenstoffatomen im Cycloalkylteil), Niederalkyloxy (Bedeutung von «Niederalkyl» siehe oben), Trifluormethyl, Phenyl, Benzyl und Acylamino mit vorzugsweise 2 bis 5, insbesondere 2 oder 3 Kohlenstoffatomen. Als -S-Het seien als besonders bevorzugt aufgeführt:

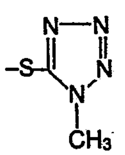 , 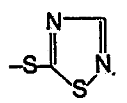 , 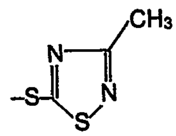 , 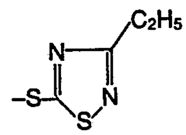 ,

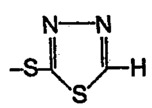 , 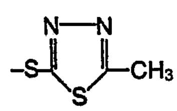 , 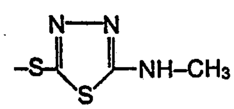 , 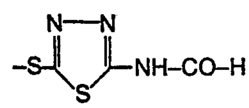 ,

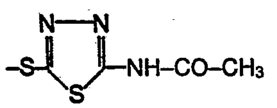 , 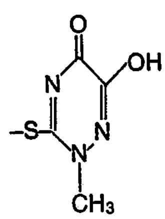 , 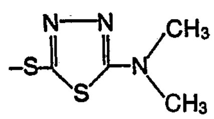 , 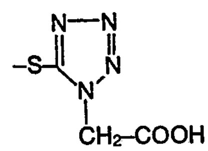

Der -S-Phenylrest in der Definition von T kann einen oder mehrere, vorzugsweise 1 bis 3, insbesondere 1 oder 2 gleiche oder verschiedene Substituenten tragen, wobei als Substituenten diejenigen bevorzugt werden, welche oben als mögliche Substituenten des Restes -S-Het aufgeführt werden.

Ganz besonders bevorzugt sind erfindungsgemässe Verbindungen, in welchen C in der D- = R-Konfiguration vorliegt.

Alle Kristallformen und Hydratformen der erfindungsgemässen Verbindungen der allgemeinen Formel (I) und ihre Salze sind in gleicher Weise antibakteriell wirksam.

Halogen W steht für Fluor, Chlor und Brom, vorzugsweise für Brom oder Chlor, insbesondere für Chlor.

Unter nukleofugen Abgangsgruppen in der Definition von W sind alle üblicherweise in der organischen Chemie verwendeten nukleofugen Gruppen zu verstehen, z. B. solche welche in Angewandte Chemie, 81 (1969), Seite 543 beschrieben sind.

Die Verbindungen der Formel (I) fallen bei der Herstellung vielfach in Form von Salzen an oder können leicht in diese überführt werden. Besonders wichtig für den Gebrauch als Arzneimittel sind die pharmazeutisch verwendbaren Salze der Verbindungen gemäss Formel (I).

Pharmazeutisch verwendbare Salze der Verbindungen der Formel (I) sind Salze dieser Verbindungen mit anorganischen und organischen Basen an der sauren Carboxylgruppe bzw. den sauren Carboxyl- und Sulfonsäuregruppen. Als Basen können hierzu alle in der pharmazeutischen Chemie, insbesondere in der Chemie der Antibiotika, üblicherweise verwendeten Basen eingesetzt werden. Als anorganische Basen seien beispielhaft genannt: Alkali- und Erdalkalihydroxide, Alkali- und Erdalkalicarbonate und Alkalihydrogencarbonate, wie Natrium- und Kaliumhydroxid, Calcium- und Magnesiumhydroxid, Natrium- und Kaliumcarbonat, Calciumcarbonat, Natrium- und Kaliumhydrogencarbonat; Aluminiumhydroxid und Ammoniumhydroxid. Als organische Amine können primäre, sekundäre und tertiäre aliphatische Amine sowie heterocyclische Amine eingesetzt werden. Beispielhaft seien genannt: Di- und Triniedrigalkylamine, z.B. Diethylamin, Triethylamin, Tri-β-hydroxyethylamin, Procain, Dibenzylamin, N,N'-Dibenzylethylendiamin, N-Benzyl-β-phenylethylamin, N-Methyl- und N-Ethylmorpholin, 1-Ephenamin, Dehydroabietylamin, N,N'-Bis-dehydroabietylethylendiamin, N-Niedrigalkylpiperidin. Auch sogenannte basische Aminosäuren wie Lysin oder Arginin können vorteilhaft als Basen Verwendung finden. Besonders bevorzugte Salze sind die Natriumsalze.

Mit dem Ausdruck «Niedrigalkyl» werden jeweils sowohl geradkettige als auch verzweigte Alkylgruppen mit 1 bis 5 vorzugsweise 1 bis 3, insbesondere 1 oder 2 Kohlenstoffatomen verstanden. Im Zusammenhang mit anderen Gruppen, wie in «Diniedrigalkylamino», bezieht sich der Ausdruck «-niedrigalkyl-» nur auf den Alkylteil der betreffenden Gruppe.

Die erfindungsgemässen Verbindungen zeigen neben guter Verträglichkeit und Löslichkeit eine breite antibakterielle Wirkung, d.h. Wirkung gegenüber mehreren Bakterienfamilien im gramnegativen und grampositiven Bereich und gegenüber β-Lactamasebildnern. Aufgrund ihrer starken antibakteriellen Eigenschaften und aufgrund ihrer Fähigkeit, das Wachstum und die Futterverwertung bei Tieren zu verbessern, stellen die erfindungsgemässen Verbindungen somit eine Bereicherung der Technik dar.

Die erfindungsgemässen Wirkstoffe wiesen bei geringer Toxizität und guter Verträglichkeit eine starke antimikrobielle Wirksamkeit auf. Diese Eigenschaften ermöglichen ihre Verwendung als Wirkstoffe in der Medizin sowohl als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemässen Wirkstoffe sind gegen ein sehr breites Spektrum von Mikroorganismen wirksam. Mit ihrer Hilfe können z.B. Gram-negative und Gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemässen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Micrococcaceae, wie Staphylokokken, z.B. Staphylococcus aureus, Staph. epidermidis, Staph. Aerogenes und Gaffkya tetragena (Staph. = Staphylococcus); Lactobacteriaceae, wie Streptokokken, z.B. Streptococcus pyogenes, α- bzw. β-hämolysierende Streptokokken, nicht (γ-)-hämolysierende Streptokokken, Str. viridans, Str. faecalis (Enterokokken), Str. agalactiae, Str. lactis, Str. equi, Str. Anaerobis und Diplococcus pneumoniae (Pneumokokken) (Str. = Streptococcus);

Neisseriaceae, wie Neisserien, z.B. Neisseria gonorrhoeae (Gonokokken), N. meningitidis (Meningokokken), N. catarrhalis und N. flava (N. = Neisseria);

Corynebacteriaceae, wie Corynebakterien, z.B. Corynebacterium diphtheriae, C. pyogenes, C. diphtheroides, C. acnes, C. parvum, C. bovis, C. renale, C. ovis, C. murisepticum, Listeria-bakterien, z.B. Listeria monocytogenes, Erysipelothrix-Bakterien, z.B. Erysipelothrix insidiosa, Kurthia-Bakterien, z.B. Kurthia zopfii (C. = Corynebacterium);

Enterobacteriaceae, wie Escherichiae-Bakterien der Coli-Gruppe, Escherichia-Bakterien, z.B. Escherichia coli, Enterobacter-Bakterien, z.B. E. aerogenes, E. cloacae, Klebsiella-Bakterien, z.B. K. pneumoniae, K. ozaenae, Erwiniae, z.B. Erwinia spec., Serratia, z.B. Serratia marcescens (E. = Enterobacter) (K. = Klebsiella), Proteae-Bakterien der Proteus-Gruppe, Proteus, z.B. Proteus vulgaris, Pr. morganii, Pr. rettgeri, Pr. mirabilis (Pe. = Proteus), Providencia z.B. Providencia sp., Salmonelleae, Salmonella-Bakterien, z.B. salmonella paratyphi A und B, S. typhi, S. enteritidis, S. cholerae suis, S. typhimurium (S. = Salmonella), Shigella-Bakterien, z.B. Shigella dysenteriae, Sh. ambigua, Sh. flexneri, Sh. boydii, Sh. sonnei (Sh. = Shigella);

Pseudomonadaceae, wie Pseudomonas-Bakterien, z.B. Pseudomonas aeruginosa, Ps. pseudomallei (Ps. = Pseudomonas), Aeromonas-Bakterien, z.B. Aeromonas liquefaciens, A. hydrophila (A. = Aeromonas); Spirillaceae, wie Vibrio-Bakterien, z.B. Vibrio cholerae, V. proteus, V. fetus (V. = Vibrio), Spirillum-Bakterien, z.B. Sprillum minus;

Parvobacteriaseae oder Brucellaceae, wie Pasteurelle-Bakterien, z.B. Pasteurella multocida, Past. pestis (Yersinia), Brucella-Bakterien, z.B. Brucella abortus, Br. melitensis, Br. suis (Br. = Brucella), Haemophilus-Bakterien, z.B. Haemophilus influenzae, H. ducreyi, H. suis, H. canis, H. aegypticus (H. = Haemophilus), Bordetella-Bakterien, z.B. Bordetella pertussis, B. bronchiseptica (B. = Bordetella), Moraxella-Bakterien, z.B. Moraxella lacunata;

Bacterioidaceae, wie Bacteroides-Bakterien, z.B. Bacteroides fragilis, B. serpens (B. = Bacteroides), Fusiforme-Bakterien, z.B. Fusobacterium fusiforme, Sphaerophorus-Bakterien, z.B. sphaerophorus necrophorus, Sph. necroticus, Sph. pyrogenes (Sph. = Sphaerophurus);

Bacillaceae, wie Aerobe Sporenbildner, z.B. Bacillus anthracis, B. subtilis, B. cereus (B. = Bacillus), Anaerobe Sporenbildner-Chlostridien, z.B. Clostridium perfringens, Cl. septicium, Cl. oedematiens, Cl. histolyticum, Cl. tetani, Cl. botulinim (Cl. = Clostridium);

Spirochaetaceae, wie Borrelia-Bakterien, z.B. Borrelia recurrentia, B. vincentii (B. = Borrelia), Treponema-Bakterien, z.B. Treponema pallidum. Tr. pertinue, Tr. carateum (Tr. = Treponema), Leptospira-Bakterien, Leptospira interrogans, z.B. Leptospira icterohaemorrhagiae, L. canicola, L. grippotyphosa, L. pomona, L. mitis, L. bovis (L. = Leptospira).

Die obige Aufzählung von Erregern ist lediglich beispielhaft und keineswegs beschränkend aufzufassen.

Als Krankheiten, die durch die erfindungsgemässen Wirkstoffe verhindert, gebessert und/oder geheilt werden können, seien beispielhaft genannt:

Erkrankungen der Atmungswege und des Rachenraumes;

Otitis; Pharyngitis; Pneumonie; Peritonitis; Pyelonephritis; Cystitis; Endocarditis; Systeminfektionen, Bronchitis; Arthritis.

Zur vorliegenden Erfindung gehören pharmazeutische Zubereitungen, die neben nichttoxischen, inerten pharmazeutisch geeigneten Trägerstoffen einen oder mehrere erfindungsgemässen Wirkstoffe enthalten oder die aus einem oder mehreren erfindungsgemässen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzukker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben anliegenden Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) und Gemische dieser Stoffe.

Salben, Pasten, Cremes und Gelee können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische oder Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süssmittel z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe nach dem oder den Trägerstoffen.

Zur vorliegenden Erfindung gehört auch die Verwendung der erfindungsgemässen Wirkstoffe sowie von pharmazeutischen Zubereitungen, die einen oder mehrere erfindungsgemässe Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rectal, vorzugsweise parenteral, insbesondere intravenös und intramusculös appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 6 bis etwa 800, vorzugsweise 15 bis 300 mg/kg Körpergewicht je 24 Stunden, gegebenenfalls in Form mehrerer, z.B. von 3 Einzelgaben zur Erzielung der gewünschten Ergebnisse zu verabreichen. Eine Einzelgabe enthält den oder die erfindungsgemässen Wirkstoffe, vorzugsweise in Mengen von etwa 2 bis etwa 300, insbesondere 10 bis 150 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in üblicher Weise zusammen mit dem Futter bzw. mit Futterzubereitungen oder mit dem Trinkwasser gegeben werden. Dadurch kann eine Infektion durch gramnegative oder gram-positive Bakterien verhindert und ebenso eine bessere Verwertung des Futters erreicht werden.

Die neuen β-Lactam-Antibiotica zeichnen sich durch starke antibakterielle Wirkungen, die in vivo und in vitro geprüft wurden und durch orale Resorbierbarkeit aus.

Die erfindungsgemässen β-Lactam-Antibiotica können zum Zwecke der Erweiterung des Wirkungsspektrums oder zur Wirkungssteigerung z.B. auch mit Aminoglykosidantibiotika wie Gentamicin, Sisomicin, Kanamicin, Amikacin oder Tobramicin kombiniert werden.

Beispiel 1

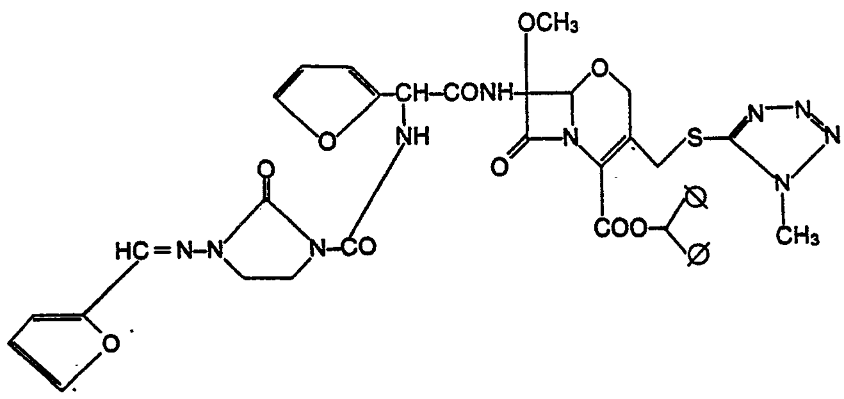

99 mg 2-(2-Oxo-3-furfurylidenamino-imidazolidin-1-yl)carbonylamino-furylessigsäure wurden bei Raumtemperatur unter Stickstoffatmosphäre in 1 ml absolutem Aceton suspendiert und mit 1 ml einer Lösung der äquimolaren Menge Triethylamin in Aceton behandelt. Man kühlte auf −25 °C, gab eine katalytische Menge an Dimethylaminopropanol dazu und danach 1 ml einer Lösung von 0,37 ml Chlorameisensäureisobutylester verdünnt mit Aceton auf 10 ml. Dieses Gemisch wurde 3 Stunden bei −20 bis −15 °C gerührt. Getrennt davon wurden 29 mg

7-Methoxy-7-amino-3-(1-methyl-1-H-tetrazol-5-ylthiomethyl)-1-oxadethia-3-cephem-4-carbonsäurediphenylmethylester

in 0,4 ml absolutem Chloroform gelöst, 0,4 ml Aceton dazugefügt und auf −15 °C gekühlt. Zu dieser Lösung wurden 0,7 ml der Lösung des gemischten

Anhydrids gegeben und 2,5 Stunden bei −15 °C bis −10 °C gerührt und noch 2,5 Stunden bei −10 bis −5 °C. Aufarbeitung erfolgte durch Eingiessen in Wasser, Extrahieren (2 ×) mit Chloroform, Waschen der organischen Phase mit gesättigter Natriumhydrogencarbonatlösung (2 ×) und Wasser, Trocknen und Einengen im Vakuum. Der Rückstand wurde durch präparative Dünnschichtchromatographie mit Benzol/Essigsäuremethylester (1 : 2) als Laufmittel getrennt:
10 mg 7-Methoxy-7} DL-α-[(2-oxo-3-furfuryliden-amino-imidazolidin-1-yl)carbonylamino-furylacetamids {-3-(1-methyl-1-H-tetrazol-5-yl-thiomethyl)-1-oxoadethia-3-cephem-4-carbonsäurediphenylmethylester.

$^1$H-NMR ppm u.a. $CDCl_3$ 250 MHz 3,48 u. 3,56 (2s, 3H); 3,83, 386 u. 3.6–4.0 (2s u. m, 7H); 4.30 (s, 2H); 4.58 u. 4.62 (2 AB-Systeme, 2H); 5.04 u. 5.10 (2s, 1H); 5.72 u. 5.76 (2d, J = 6 Hz, 1H); 6.3–6.5 (m, 3H); 6.7–6.8 (m, 1H); 6.90 (s, 1H); 7.06 u. 7.08 (2s, 1H); 7.2–7.6 (m, 12H); 7.74 u. 7.77 (2s, 1H); 9.02 u. 9.06 (2d, J = 6 Hz, 1H).

IR $cm^{-1}$ u.a. $CHCl_3$ 3600–3150 w, 3030–2850 w, 1790 s, 1740–1720 s, 1690 m, 1490 s, 1415 s, 1400 s, 1270 s, 1340–1210 s, 1080 m, 1025 m, 915 m, 705 m.

Beispiel 2

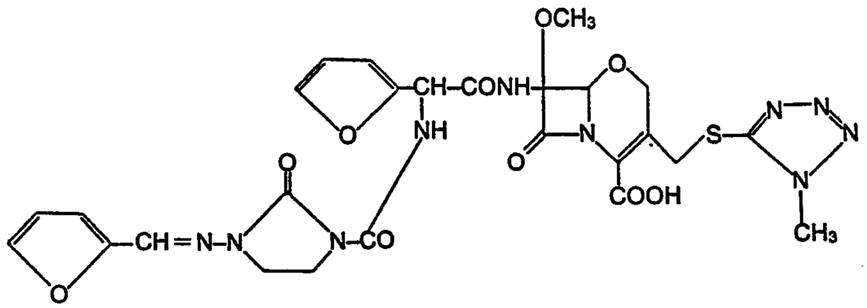

39 mg des in Beispiel 1 erhaltenen Produkts werden in 0,4 ml Dichlormethan gelöst und auf 0° gekühlt. Anschliessend gibt man 0,4 ml eines 1 : 1 Gemisches auf Trifluoressigsäure und Anisol dazu und rührt 25 Minuten bei dieser Temperatur. Nach abdestillieren des Lösungsmittels am Rotationsverdampfer wird mit Benzol verdünnt und erneut eingeengt. Nach Trocknen im Hochvakuum wird mit Ether, dem einige Tropfen Dichlormethan beigefügt sind, verrieben und abgesaugt: 28 mg weisses Pulver.

$^1$H-NMR ppm u.a. DMF–$d_7$ 200 MHz 3,36 u. 3,50 (2s; 3H); 3,95 u. 4,08 (1m u. 1s, 7H); 4,38 (AB-System, 2H); 4,68 (AB-System, 2H) 5,18 (s, 1H); 6,00 u. 6,03 (2d, 1H); 6,4–6,8 (m, 3H); 6,9–7,0 (m, 1H).

IR $cm^{-1}$ u.a. Nujol 1785 s.

Beispiel 3

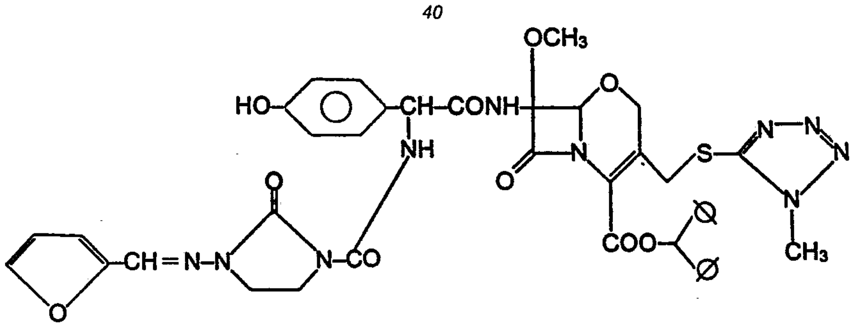

59 mg 2-(2-Oxo-3-furfurylidenamino-imidazolidin-1-yl)carbonylaminopara-hydroxyphenylessigsäure
wurden in 0,5 ml Aceton suspendiert und mit 0,5 ml einer Lösung versetzt, die aus 318 mg N-Methylmorpholin verdünnt mit Aceton auf 10 ml hergestellt worden war. Danach kühlte man auf −30 °C und fügte 0,5 ml einer Lösung von 341 mg Chlorameisensäureethylester verdünnt mit Aceton auf 10 ml hinzu und rührte 1,5 Stunden bei −25 °C. Anschliessend wurde eine Lösung von 40 mg
7-Methoxy-7-amino-3-(1-methyl-1-tetrazol-5-ylthiomethyl)-1-oxadethia-3-cephem-4-carbonsäurediphenylmethylester
gelöst in 0,5 ml DMF hinzugefügt und 1 Stunde bei −25 °C, 3 Stunden bei −20 und 3 Stunden bei −15 °C gerührt. Die Aufarbeitung erfolgte durch Eingiessen in Eiswasser, Extrahieren (3 ×) mit Chloroform, Waschen der organischen Phase mit Wasser (1 ×) und gesättigter Natriumhydrogencarbonatlösung (2 ×) Trocknen und Einengen im Vakuum. Durch anschliessende präparative Dünnschichtchromatographie konnte der gewünschte (DL-α-[(2-Oxo-3-furfurylidenamino-imidazolidin-1-yl)carbonylamino-parahydroxy-

phenylacetamid)-3-(1-methyl-1-H-tetrazol-5-yl-thiomethyl)-1-oxodethia-3-cephem-4-carbonsäurediphenylmethylester
als einen harten Schaum erhalten werden.

$^1$H-NMR ppm u.a. $CDCl_3$ 250 MHz 3.52, 3.84 u. 3.7–4.1 (2s u. 1m, 1OH); 4.2–4.7 (2m, 4H); 5.02 (s, 1H); 5.52 u. 5.54 (2d, 1H); 6.5 (m, 1H); 7.80 (s, 1H); 9.10 u. 9.14 (2d, 1H).

IR $cm^{-1}$ u.a. $CHCl_3$ 3150–3500 w, 3050–2800 w, 1785 s, 1740–1700 s, 1680 m, 1480 s, 1410 s, 1395 s, 1260 s.

Beispiel 4

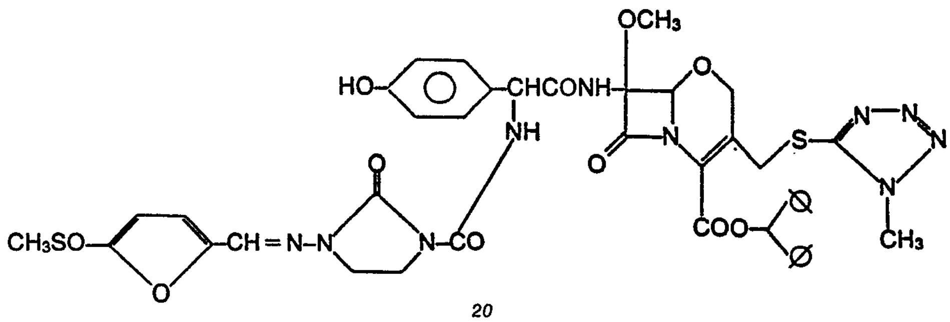

103 mg 2-[(2-Oxo-3-(4-methylsulfinylfurfuryliden)aminoimidazolidin-1-yl)carbonylamino-para-hydroxyphenylessigsäure
wurden in 0,2 ml DMF suspendiert und bei Raumtemperatur mit 0,1 ml einer Lösung von 1,3 ml N-Methylmorpholin in N,N-Dimethylformamid verdünnt auf 5 ml versetzt. Danach kühlte man die Mischung auf −30° und fügte 0,1 ml einer Lösung von 1,28 g Chlorameisensäureester in Dimethylformamid (Gesamtvolumen 5 ml) hinzu und rührte 2,5 Stunden bei −20° bis −25°. Anschliessend wurde eine Lösung von 40 mg
7-Methoxy-7-amino-3-(1-methyl-1-H-tetrazol-5-yl-thiomethyl)-1-oxadethia-3-cephem-4-carbonsäurediphenylmethylester
gelöst in 0,45 ml Dimethylformamid hinzugefügt und 1 Stunde bei −25°, 1 Stunde bei −18°, 1,5 Stunden bei −16° und über Nacht bei −11° gerührt. Die Aufarbeitung erfolgte auf die übliche Art und Weise. Durch anschliessende präparative Dünnschichtchromatographie konnte der gewünschte
7-Methoxy-7(DL-[2-oxo-3(4-methylsulfinyl-furfuryliden)-amino-imidazolidin-1-yl)carbonyl-amino-parahydroxyphenylacetamido)-3-(1-methyl-1-H-tetrazol-5-yl-thiomethyl)-1-oxadethia-3-cephem-4-carbonsäurediphenylmethylester
als ein harter Schaum erhalten werden.

$^1$H-NMR ppm u.a. $CD_3OD$, DMSO–$d_6$ 2.38 (2s, 3H); 3.22 u. 3.40 (2s, 3H); 3.6–4.9 u. 3.80 (m u. s, 7H); 4.0– (2m, 4H); 5.00 (s, 1H); 5.40 u. 5.42 (2s, 1H); 6.43 (m, 1H); 6.6–6,8 (m, 3H).

IR $cm^{-1}$ u.a. $CHCl_3$ 3400–3100 w, 3050–2800 m, 1785 s, 1720 s, 1670 s, 1510 m, 1480 s, 1390–1410 s, 1265 s.

Beispiel 5

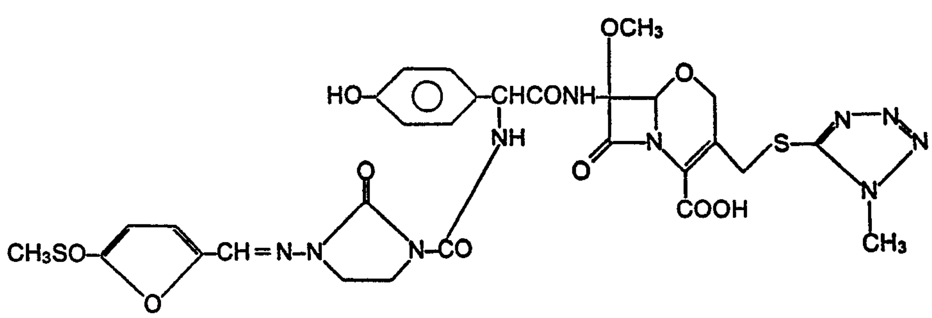

24 mg des in Beispiel 4 erhaltenen Produkts werden in 0,4 ml Dichlormethan gelöst und auf 0° gekühlt. Anschliessend gibt man 0,4 ml eines 1 : 1 Gemisches aus Trifluoressigsäure und Anisol dazu und rührt 25 Minuten bei dieser Temperatur. Nach Abdestillieren des Lösungsmittels am Rotationsverdampfer wird mit Benzol verdünnt und erneut eingeengt. Nach Trocknen im Hochvakuum wird mit Ether, dem einige Tropfen Dichlormethan beigefügt sind, verrieben und abgedampft: 13 mg weisses Pulver.

$^1$H-NMR ppm u.a. DMF–$d_7$, 250 MHz 2.58 (2s, 3H); 3.27 u. 3.50 (2s, 3H); 3.96 (m, 4H); 4.09 u. 4.10 (2s, 3H); 4.25–4.70 (2m, 4H); 5.18 (2s, 1H); 5.75 u. 5.77 (2d, 1H); 6.7–6.9 (m, 4H); 9.04 u. 9.24 (2d, 1H).

IR $cm^{-1}$ u.a. Nujol 1780 s.

Beispiel 6

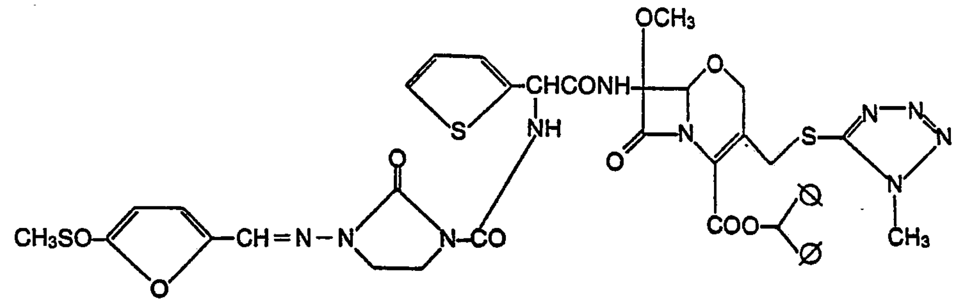

100 mg 2-(2-Oxo-3-(4-methylsulfinylfurfuryliden)aminoimidazolidin-1-yl)carbonylaminothienylessigsäure

wurden unter Stickstoffatmosphäre in 0,2 ml N,N-Dimethylformamid gelöst und bei Raumtemperatur 26 µl N-Methylmorpholin hinzugefügt. Danach kühlte man auf −30° und gab 23 µl Chlorameisensäureethylester hinzu und rührte anschliessend 3 Stunden bei −20° bis −25°. Nun wurde eine Lösung von 40 mg

7-Methoxy-7-amino-3-(1-methyl-1-H-tetrazol-5-yl-thiomethyl)-1-oxadethia-3-cephem-4-carbonsäurediphenylmethylester

gelöst in 0,45 ml Dimethylformamid hinzugefügt und 1 Stunde bei −20°, 0,5 Stunden bei −15°, 1,5 Stunden bei −13° und über Nacht bei −10° gerührt. Die Aufarbeitung erfolgt auf die übliche Art und Weise. Durch anschliessende präparative Dünnschichtchromatographie konnte der gewünschte

7-Methoxy-7-(DL-α[(2-oxo-3-(4-methylsulfinylfurfuryliden)-amino-imidazolin-1-yl)-carbonylamino-thienylacetamido)-3-(1-methyl-1-H-tetrazol-5-yl-thiomethyl)-1-oxadethia-3-cephem-4-carbonsäurediphenylmethylester

als ein harter Schaum erhalten werden.

$^1$H-NMR ppm u.a. $CDCl_3$ 250 MHz 2.46 (2s, 3H); 3.46 u. 3.54 (2s, 3H); 3.6–3.8, 3.9–4.1, 3.84 u. 3.85 (2m u. 2s, 7H); 4.26 u. 4.56 (2m, 4H); 5.04 u. 5.08 (2s, 1H); 5.82 u. 5.88 (2d, 1H); 9.00 u. 9.08 (2d, 1H).

IR $cm^{-1}$ u.a. $CHCl_3$ 3500–3100 w, 3050–2800 w, 1785 s, 1715–1740 s, 1680 s, 1480 s, 1405 s, 1265 s.

Beispiel 7

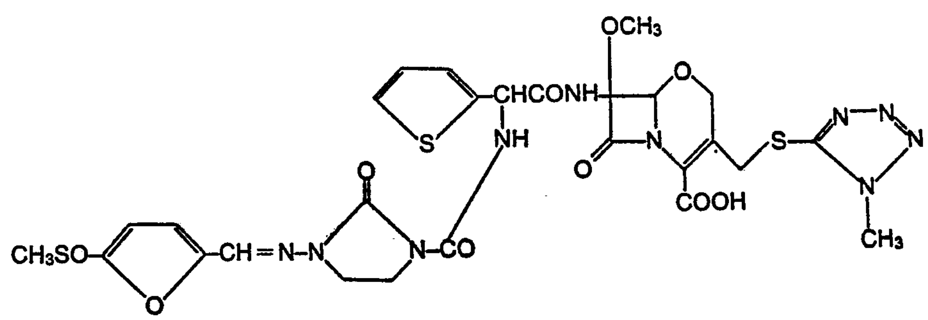

25 mg des in Beispiel 6 erhaltenen Produkts werden in 0,4 ml Dichlormethan gelöst und auf 0° gekühlt. Anschliessend gibt man 0,4 ml eines 1 : 1 Gemisches aus Trifluoressigsäure und Anisol dazu und rührt 25 Minuten bei dieser Temperatur. Nach Abdestillieren des Lösungsmittels am Rotationsverdampfer wird mit Benzol verdünnt und erneut eingeengt. Nach Trocknen im Hochvakuum wird mit Ether, dem einige Tropfen Dichlormethan beigefügt sind, verrieben und abgesaugt: 17 mg weisses Pulver.

$^1$H-NMR ppm u.a. DMF–$d_7$, 200 MHz 2.51 (2s, 3H); 3.31 u. 3.48 (2s, 3H); 3.91 (m, 4H); 4.03 (s, 3H); 4.34 u. 4.60 (2m, 4H); 5.16 (s, 1H); 6.10 u. 6.14 (2d, 1H); 7.38 (s, 1H); 9.13 u. 9.08 (2d, 1H).

IR $cm^{-1}$ u.a. Nujol 1785 s.

Beispiel 8

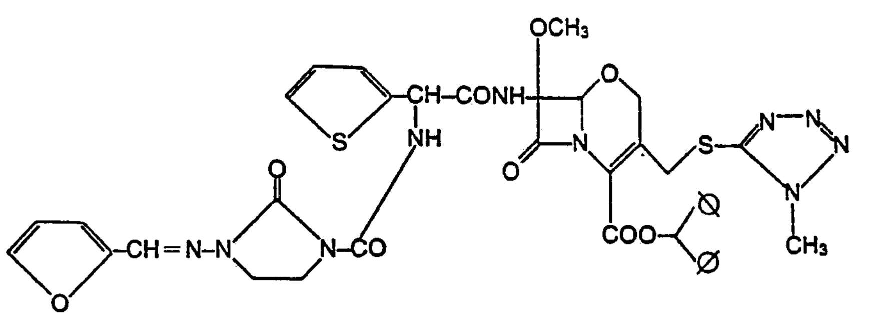

236 mg 2-(2-Oxo-3-furfurylidenamino-imidazolidin-1-yl)carbonylamino-thienyl-essigsäure wurden bei Raumtemperatur unter Stickstoffatmosphäre in 3 ml abs. Dichlormethan suspendiert und durch Zugabe von 86 µl N-Methylmorpholin gelöst. Man kühlte auf −20 °C, gab tropfenweise 56 µl Methansulfonylchlorid zu und rührte 1 h bei −20 °C. Danach wurde auf −30 °C gekühlt und man gab eine Lösung von 254 mg
7-Methoxy-7-amino-3(1-methyl-1-H-tetrazol-5-ylthiomethyl)-1-oxadethia-3-cephem-4-carbonsäurediphenylmethylester
und 60 µl N-Methylmorpholin in 4 ml absol. Dichlormethan zu. Das Gemisch durfte sich langsam auf 0 °C erwärmen und blieb bei dieser Temp. über Nacht stehen. Die Aufarbeitung erfolgte durch Eingiessen in 60 ml gesättigte $NaHCO_3$-Lösung, Extraktion mit Dichlormethan (2×), Waschen der organischen Phase mit 60 ml eiskalter 0.1 N HCl, gesättigter $NaHCO_3$-Lösung und Wasser. Nach Trocknen über $MgSO_4$ und Abdampfen des Lösungsmittels i.Vak. erhielt man ein Öl, das durch Chromatographie an 25 g Kieselgel (Essigester) gereinigt wurde. Ausb.: 73 mg
7-Methoxy-7-[D,L-(2-oxo-3-furfuryliden-amino-imidazolidin-1-yl)carbonylamino-thienyl-acetamido]-3-[1-methyl-1-H-tetrazol-5-yl-thiomethyl)-1-oxadethia-3-cephem-4-carbonsäure-diphenylmethylester.
Rf: 0.31, 0.35 (Essigester).

$^1$H-NMR (250 MHz, $CDCl_3$) δ = 3.48, 3.56 (s, 3H, $OCH_3$), 3.86 (s, 3H, N–$CH_3$), 3.75, 3.95 (m, 4H, $CH_2N$), 4.30, 4.60 (m, 4H, $CH_2S$, $CH_2O$), 5.08, 5.11 (s, 1H, Azetidinon-H), 5.86 (m, 1H, –CH–D, L), 6.51 (m, 1H, Furyl-H), 6.82 (m, 1H, Furyl-H), 6.92 (s, 1H, $CHPh_2$), 7.0, 7.08 (m, 2H, Thienyl-H), 7.2–7.6 (m, 13H, H arom, -heteroarom, –CH=N–), 7.78, 7.80 (s, 1H, CONH), 9.08, 9.15 (d, J=7 Hz, 1H, CH–NHCO).

IR (KBr) 3286 (NH Amid), 1784 (C=O, β-Lactam), 1725 (C=O, Ester), 1668, 1650 $cm^{-1}$ (C=O, Amid, Semicarbazid; C=N).

Beispiel 9

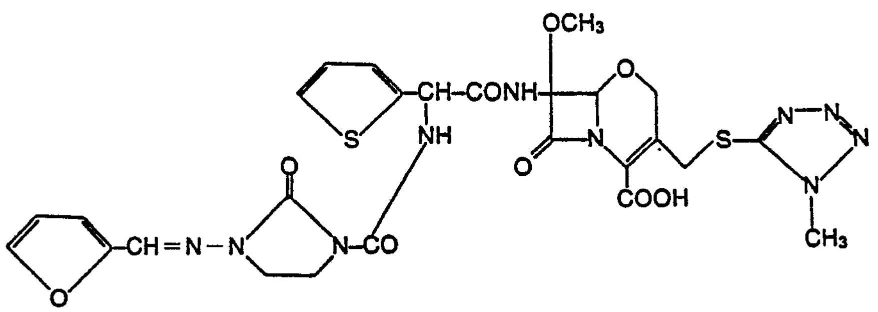

60 mg des in Beispiel 8 erhaltenen Produkts werden in 1.2 ml Dichlormethan gelöst und auf 0° gekühlt. Anschliessend gibt man 1.0 ml eines 1 : 1 Gemisches auf Trifluoressigsäure und Anisol dazu und rührt 25 Minuten bei dieser Temperatur. Nach abdestillieren des Lösunsmittels am Rotationsverdampfer wird mit Benzol verdünnt und erneut eingeengt. Nach dem Trocknen im Hochvakuum wird mit Ether, dem einige Tropfen Dichlormethan beigefügt sind, verrieben und abgesaugt: 47 mg weisses Pulver. Schmp.: 165–167 °C (Z).

$^1$H-NMR (250 MHz, DMSO) δ = 3.40, 3.42 (s, 3H, $OCH_3$), 3.95 (s, 3H, N–$CH_3$), 3.8 (m, 4H, $CH_2N$), 4.23, 4.5 (m, 4H, $CH_2S$, $CH_2O$), 5.13 (s, 1H, Azetidinon-H), 5.75, 5.92 (m, 1H, –CH–D, L), 6.65 (m, 1H, Furyl-H), 6.89 (d, J=4.5 Hz, 1H, Furyl-H), 6.9–7.6 (m, 4H), Thienyl-H, Furyl-H), 7.77 (s, 1H, CH=N), 7.97 (s, 1H, NH), 9.0 (m, 1H, NH).

IR (KBr) 3400–3300 (b, OH), 1779 (C=O, β-Lactam), 1724 (C=O, Ester), 1678 (C=O, Amid, Semicarbazid; C=N).

Beispiel 10

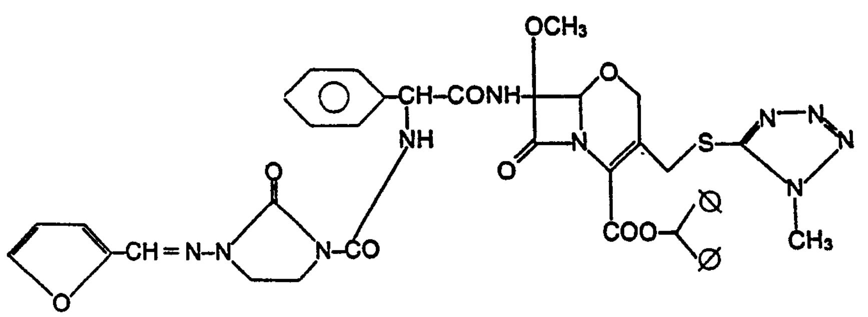

463 mg 2-(2-Oxo-3-furfurylidenamino-imidazolidin-1-yl)Carbonylamino-phenylessigsäure wurden bei Raumtemperatur unter Stickstoffatmosphäre in 3 ml absol. Dichlormethan suspendiert und durch Zugabe von 172 µl N-Methylmorpholin gelöst. Man kühlte auf −30 °C, gab tropfenweise 241 µl Trifluormethansulfonsäureanhydrid zu und rührte 1 h bei −30 °C. Dazu wurde die Lösung von 509 mg

7-Methoxy-7-amino-3(1-methyl-1-H-tetrazol-5-ylthio-methyl)-1-oxadethia-3-cephem-4-carbonsäurediphenylmethylester
und 121 µl N-Methylmorpholin in 5 ml absol. Dimethylformamid gegeben. Das Gemisch durfte sich langsam auf 0 °C erwärmen und blieb bei dieser Temp. über Nacht stehen. Die Aufarbeitung erfolgte durch Eingiessen in 100 ml gesättigte $NaHCO_3$-Lösung, Extraktion mit Dichlormethan (3 × ) Waschen der organischen Phase mit 100 ml eiskalter 0.1 N HCl, gesättigter $NaHCO_3$-Lösung und Wasser. Nach Trocknen über $MgSO_4$ und Abdampfen des Lösungsmittels i.Vak. wurde das Rohprodukt an 65 g Kieselgel (Essigester) chromatographiert.

Ausb.: 183 mg 7-Methoxy-7-[D,L-(2-oxo-3-furfuryliden-aminoimidazolidin-1-yl)carbonylamino-phenylacetamido]-3-(1-methyl-1-H-tetrazol-5-yl-thiomethyl)-1-oxadethia-3-cephem-4-carbonsäurediphenylmethylester.

RF: 0.26 (Essigester).

$^1$H-NMR (250 MHz, $CDCl_3$) δ = 3.28, 3.50 (s, 3H, $OCH_3$), 3.84 (s, 3H, N–$CH_3$), 3.5–4.0 (m, 4H, $CH_2$N), 4.25, 4.5 (m, 4H, $CH_2$S, $CH_2$O), 5.01, 5.09 (s, 1H, Azetidinon-H), 5.64, 5.75 (d, J = 7 Hz, D,L–CH–), 6.48 (m, 1H, Furyl-H), 6.80 (m, 1H, Furyl-H), 6.90 (s, 1H, $CHPh_2$), 7.2–7.6 (m, 18H), 9.16, 9.28 (d, J = 7 Hz, 1H, CHNHCO).

IR (KBr) 3394 (NH Amid), 1787 (C = O, β-Lactam), 1732 (C = O, Ester), 1681 cm$^{-1}$ (C = O, Amid; C = O, C = N, Semicarbazid).

Beispiel 11

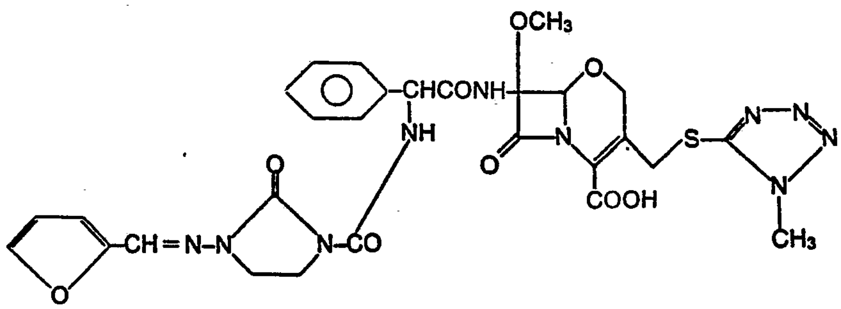

196 mg des in Beispiel 10 erhaltenen Produkts werden in 3 ml Dichlormethan gelöst und auf 0° gekühlt. Anschliessend gibt man 2.4 ml eines 1 : 1 Gemisches aus Trifluoressigsäure und Anisol dazu und rührt 25 Minuten bei dieser Temperatur. Nach abdestillieren des Lösungsmittels am Rotationsverdampfer wird mit Benzol verdünnt und erneut eingeengt. Nach Trocknen im Hochvakuum wird mit Ether, dem einige Tropfen Dichlormethan beigefügt sind, verrieben und abgesaugt: 153 mg weisses Pulver. Schmp.: 142 °C Zusammenbacken, 167 °C (Z)

RF: 0.15 (BABA)

$^1$H-NMR (250 MHz, DMSO) δ = 3.06, 3.38 (s, 3H, $OCH_3$), 3.45, 3.8 (m, 4H, $NCH_2$), 4.15–4.55 (m, 4H, $CH_2$S, $CH_2$O), 5.08, 5.11 (s, 1H, Azetidinon-H), 5.66 (d, J = 9 Hz, 1H, NHCHCO), 6.65 (m, 1H, Furyl-H), 6.87 (d, J = 4.5 Hz, Furyl-H), 7.0–7.5 (m, 6H, Ph, Furyl-H), 7.78 (s, 1H, CH = N), 7.86 (s, 1H, NH), 8.98, 9.13 (d, J = 9 Hz, 1H, NH).

IR (KBr) 3480 (b, OH), 1783 (C = O, β-Lactam), 1728 (C = O, Ester), 1680, 1640 [sh] (C = O, Amid, Semicarbazid; C = N).

Beispiel 12

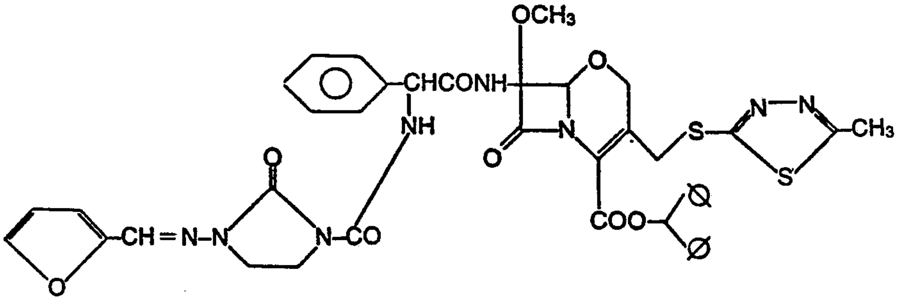

632 mg 2-(2-Oxo-3-furfurylamino-imidazolidin-1-yl)carbonylaminophenylessigsäure
wurden bei Raumtemp. unter Stickstoffatmosphäre in 4 ml absol. Dichlormethan suspendiert und durch Zugabe von 234 µl N-Methylmorpholin gelöst. Man kühlte auf −30 °C, gab tropfenweise 328 µl Trifluormethansulfonsäureanhydrid zu und rührte 45 min. bei −30 °C. Dazu wurde eine Lösung von 716 mg

7-Methoxy-7-amino-3-(2-methyl-1-thia-3,4-diazol-5-ylthiomethyl)-1-oxadethia-3-cephem-4-carbonsäure-diphenylmethylester
und 165 µl N-Methylmorpholin in 10 ml absol. Dichlormethan gegeben. Das Gemisch durfte sich langsam auf 0 °C erwärmen und blieb bei dieser Temperatur über Nacht stehen. Die Aufarbeitung erfolgte durch Eingiessen in 150 ml gesättigte $NaHCO_3$-Lösung, Extraktion mit Dichlormethan

(3 ×), waschen der organischen Phase mit 150 ml eiskalter 0.1 N HCl, gesättigter $NaHCO_3$-Lösung und Wasser. Nach Trocknen über $MgSO_4$ und Abdampfen des Lösungsmittels i.Vak. wurde das Rohprodukt an 100 g Kieselgel (Essigester) chromatographiert. Ausb.: 295 mg
7-Methoxy-7-[D,L-(2-oxo-3-furfurylidenamino-imidazolidin-1-yl)carbonylamino-phenylacetamido]-3-(2-methyl-1-thia-3,4-diazol-5-ylthiomethyl)-1-oxadethia-3-cephem-4-carbonsäure-diphenylmethylester.
Rf: 0.25 (Essigester).
Schmp.: 158 °C.

$^1$H-NMR (200 MHz, $CDCl_3$) δ = 2.69, 2.72 (s, 3H, $CH_3$), 3.30, 3.53 (s, 3H, $OCH_3$), 3.7–4.6 (m, 8H, $CH_2N$, $CH_2S$, $CH_2O$), 5.03, 5.08 (s, 1H, Azetidinon-H), 5.60, 5.66 (d, J = 7.5 Hz, 1H, NHCHCO), 6.50 (m, 1H, Furyl-H), 6.82 (m, 1H, Furyl-H), 6.90 (s, 1H, $CHPh_2$), 7.0 (s, 1H, NH), 7.25–7.6 (m, 16H, Ph, Furyl-H), 7.74–7.76 (s 1H, CH = N), 9.16, 9.24 (d, J = 7.5 Hz, 1H, NH).

IR (KBr) 3303 (NH Amid), 1784 (C = O, β-Lactam), 1726 (C = O, Ester), 1679 $cm^{-1}$ (C = O, Amid; C = O, C = N Semicarbazid).

Beispiel 13

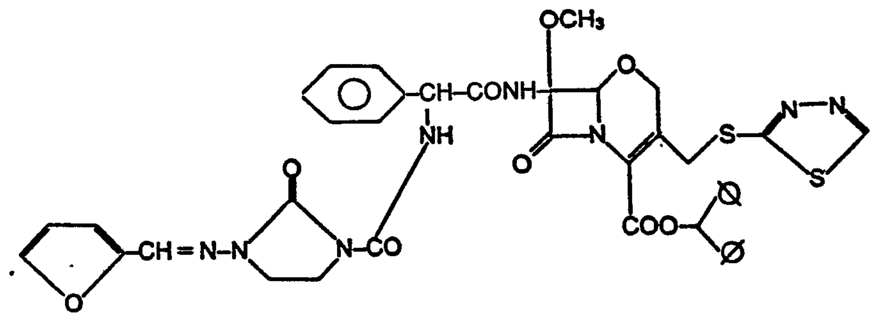

843 mg 2-(2-Oxo-3-furfurylamino-imidazolidin-1-yl)carbonylamino-phenylessigsäure wurden bei Raumtemp. unter Stickstoffatmosphäre in 5 ml absol. Dichlormethan suspendiert und durch Zugabe von 309 µl N-Methylmorpholin gelöst. Man kühlte auf −30 °C, gab tropfenweise 433 µl Trifluormethansulfonsäureanhydrid zu und rührte 45 min. bei −30 °C. Dazu wurde eine Lösung von 919 mg
7-Methoxy-7-amino-3-(1-thia-3,4-diazol-2-ylthiomethyl)-1-oxadethia-3-cephem-4-carbonsäurediphenylmethylester
und 218 µl N-Methylmorpholin in 12 ml absol. Dichlormethan gegeben. Das Gemisch durfte sich langsam auf 0 °C erwärmen und blieb bei dieser Temp. über Nacht stehen. Die Aufarbeitung erfolgte durch Eingiessen in 200 ml gesättigte $NaHCO_3$-Lösung, Extraktion mit Dichlormethan (3 ×), waschen der organischen Phase mit 200 ml eiskalter 0.1 N HCl, gesättigter $NaHCO_3$-Lösung und Wasser. Nach Trocknen über Magnesiumsulfat und Abdampfen des Lösungsmittels i.Vak. wurde das Rohprodukt an 140 g Kieselgel (Essigester) chromatographiert. Ausb.: 153 mg
7-Methoxy-7-[D,L-(2-oxo-3-furfurylidenamino-imidazolidin-1-yl)carbonylamino-phenylacetamido]-3-(1-thia-3,4-diazol-2-ylthiomethyl)-1-oxadethia-3-cephem-4-carbonsäure-diphenylmethylester.
RF: 0.25 (Essigester).
Schmp.: 164 °C.

$^1$H-NMR (250 MHz, $CDCl_3$) δ = 3.28, 3.54 (s, 3H, $OCH_3$), 3.7, 3.9 (m, 4H, $CH_2N$), 4.2–4.55 (m, 4H, $CH_2S$, $CH_2O$), 5.06, 5.10 (s, 1H, Azetidinon-H), 5.63, 5.71 (d, J = 7 Hz, 1H, NHCHCO), 6.51 (m, 1H, Furyl-H), 6.82 (d, J = 4.5 Hz, 1H, Furyl-H), 7.1–7.6 (m, 17H, Ph, Furyl-H, NH), 7.69, 773 (s, 1H, CH = N), 9.0, 9.04 (s, 1H, Thiadiazol-H), 9.19, 9.28 (d, J = 7 Hz, 1H, NH).

IR (KBr) 3299 (NH, Amid), 1787 (C = O, β-Lactam), 1724 (C = O, Ester) 1673 $cm^{-1}$ (C = O, Amid, Semicarbazid; C = N).

Beispiel 14

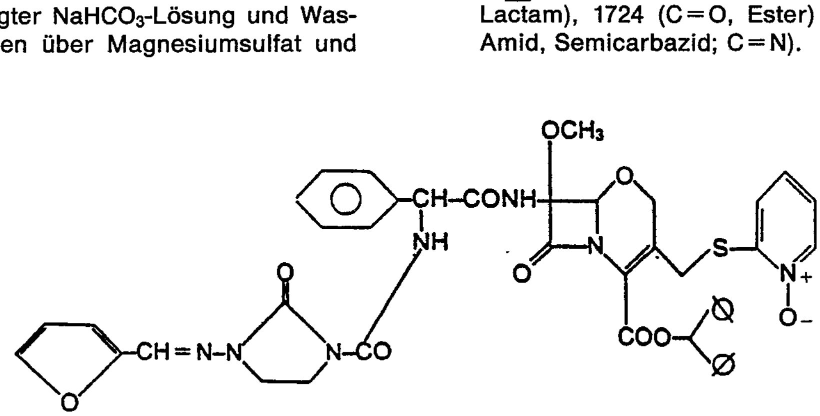

53 mg 2-(2-Oxo-3-furfurylidenamino-imidazolidin-1-yl)Carbonylamino-phenylessigsäure wurden bei Raumtemperatur unter Stickstoffatmosphäre in 0.3 ml absol. Dichlormethan suspendiert und durch Zugabe von 20 µl N-Methylmorpholin gelöst. Man kühlte auf −30 °C, gab tropfenweise 27 µl Trifluormethansulfonsäureanhydrid zu und rührte 1 h bei −30 °C. Dazu wurde die Lösung von

7-Methoxy-7-amino-3(1-oxopyrid-2-ylthio-methyl)-1-oxadethia-3-cephem-4-carbonsäure-diphenylmethylester

und 14 µl N-Methylmorpholin in 1.5 ml absol. Dichlormethan gegeben. Das Gemisch durfte sich langsam auf 0 °C erwärmen und blieb bei dieser Temp. über Nacht stehen. Die Aufarbeitung erfolgte durch Eingiessen in 60 ml eiskalte 1 N HCl, Extraktion mit Dichlormethan (2 ×), Waschen der organischen Phase mit gesättigter $NaHCO_3$-Lösung und Wasser. Nach Trocknen über $MgSO_4$ und Abdampfen des Lösungsmittels i.Vak. wurde das Rohprodukt durch Chromatographie an 10 g Kieselgel (Aceton : Essigester = 7 : 3) gereinigt.
Ausb.: 39 mg

7-Methoxy-7-[(2-oxo-3-furfuryliden-amino-imidazolidin-1-yl)carbonylamino-phenyl-acetamido]-3-(1-oxopyrid-2-yl-thiomethyl)-1-oxa-dethia-3-cephem-4-carbonsäurediphenylmethylester. Rf: 0.58 (BABA).

Schmp.: 178 °C (Z).

$^1$H-NMR (250 MHz, $CDCl_3$) δ = 3.54 (s, 3H, $OCH_3$), 3.8–4.4 (m, 8H, $CH_2N$, $CH_2S$, $CH_2O$), 5,04 (s, 1H, Azetidinon-H), 5.27 (d, J = 8 Hz, 1H, NHCHCO), 6.50 (m, 1H, Furyl-H), 6.81 (d, J = 4.5 Hz, 1H, Furyl-H), 7.05–7.55 (m, 20H, Ph, NH, Pyridyl-H, Furyl-H), 7.80 (s, 1H, CH = N), 8.25 (m, 1H, Pyridyl-H), 9.13 (d, J = 8 Hz, 1H, NHCHCO).

IR (KBr) 3298 (NH, Amid), 1786 (c = o, β-Lactam), 1726 (c = o, Ester), 1678 (c = o, Amid, Ureido; C = N).

Beispiel 15

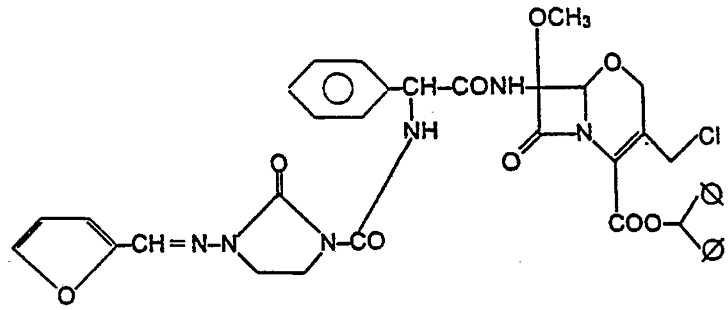

1.15 g 2-(2-oxo-3-furfurylamino-imidazolidin-1-yl)carbonylaminophenylessigsäure

wurden bei Raumtemp. unter Stickstoffatmosphäre in 6 ml absol. Dichlormethan suspendiert und durch Zugabe von 0.43 ml N-Methylmorpholin gelöst. Man kühlt auf −30 °C, gab 0.6 ml Trifluormethansulfonsäureanhydrid zu und rührte 45 min. bei −30 °C. Dazu wurde eine frisch bereitete Lösung von 1.07 g

7-Methoxy-7-amino-3-chloromethyl-1-oxa-dethia-3-cephem-4-carbonsäure-diphenylmethylester

und 0.3 ml N-Methylmorpholin in 15 ml absol. Dichlormethan gegeben. Das Gemisch durfte sich auf 0 °C erwärmen und blieb bei dieser Temp. über Nacht stehen. Die Aufarbeitung erfolgte durch Eingiessen in 200 ml gesättigte $NaHCO_3$-Lösung, Extraktion mit Dichlormethan (2 ×), waschen der organischen Phase mit 200 ml eiskalter 0.1 N HCl, gesättigter $NaHCO_3$-Lösung und Wasser. Nach Trocknen über Magnesiumsulfat wurde das Rohprodukt an 150 g, dann an 40 g Kieselgel (Toluol : Essigester 1 : 3) chromatographiert. Man erhielt

7-Methoxy-7-[D,L-(2-oxo-3-furfurylidenamino-imidazolidin-1-yl)carbonylamino-phenylacet-amido]-3-chloromethyl-1-oxa-dethia-3-cephem-4-carbonsäure-diphenylmethylester.

RF: 0.31 (Toluol : Essigester 1 : 3).

Schmp.: 184 °C.

$^1$H-NMR (250 MHz, $CDCl_3$) δ = 3.5, 3.7 (s, 3H, $OCH_3$), 3.3–4.5 (m, 8H, $CH_2N$, $CH_2S$, $CH_2O$), 5.5 (d, J = 8.5 Hz, 1H, NHCHCO), 6.5 (m, 1H, Furyl-H), 6.8 (m, 1H, Furyl-H), 7.0–7.6 (m), 7.8 (s, 1H, CH = N), 9.2 (m, 1H, NH).

IR (KBr) 3300 (NH, Amid), 1785 (C = O, β-Lactam), 1727 (C = O, Ester), 1784 (C = O, Amid, Semicarbazid; C = N).

Wie für die Beispiele 2, 5, 7, 9 und 11 explizit beschrieben, wurden aus den aus Beispiel 3, 12–15 erhaltenen Diphenylmethylestern die entsprechenden Säuren freigesetzt:

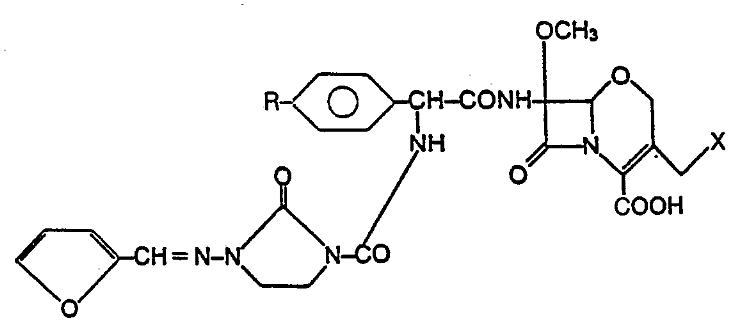

Beispiel 16

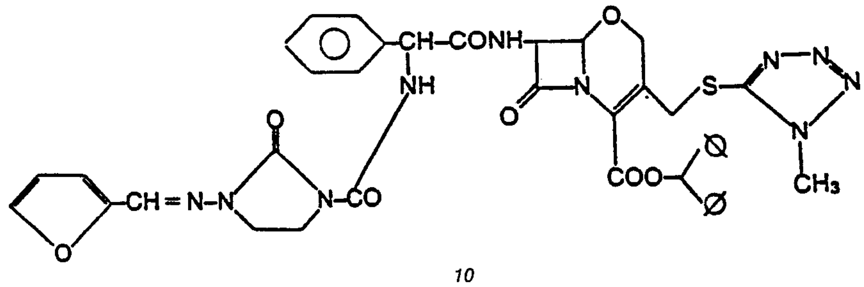

553 mg 2-(2-Oxo-3-furfurylamino-imidazolidin-1-yl)carbonylamino-phenylessigsäure

wurden bei Raumtemp. unter Stickstoffatmosphäre in 3.2 ml absol. Dichlormethan suspendiert und durch Zugabe von 205 µl N-Methylmorpholin gelöst. Man kühlte auf −30 °C, gab 287 µl Trifluormethansulfonsäureanhydrid zu und rührte 45 min. bei −30 °C. Dazu gab man eine Lösung von 571 mg 7-Amino-3-(1-methyl-1-H-tetrazol-5-ylthiomethyl)-1-oxadethia-3-cephem-4-carbonsäurediphenylmethylester

und 144 µl N-Methylmorpholin in 8 ml absol. Dichlormethan und rührte 3 h bei −20 °C. Die Aufarbeitung erfolgte durch Eingiessen in 100 ml gesättigte $NaHCO_3$-Lösung. Extraktion mit Dichlormethan (3×), waschen mit 0.1 N kalter HCl, gesättigter $NaHCO_3$-Lösung und Wasser. Nach Trocknen über Magnesiumsulfat und Abdampfen des Lösungsmittels i.Vak. wurde das Rohprodukt an 60 g Kieselgel (Essigester) chromatographiert.

Ausb.: 204 mg 7-[(2-oxo-3-furfurylidenaminoimidazolidin-1-yl)-carbonylaminophenylacetamido-3-(1-methyl-1-H-tetrazol-5-yl-thiomethyl)-1-oxadethia-3-cephem-4-carbonsäure-diphenylmethylester.
Rf: 0.30 (Essigester).
Schmp.: 172 °C.

$^1$H-NMR (200 MHz, $CDCl_3$) δ = 3.5–4.0 (m, 4H, $CH_2N$), 3.83 (s, 3H, $NCH_3$), 4.18, 4.33 (d, J = 14 Hz, 2H, $CH_2O$), 4.54, 4.65 (d, J = 18 Hz, 2H, $CH_2S$), 4.70 (dd, J = 1 Hz, J = 7.5 Hz, 1H, Azetidinon-H), 5.0 (d, J = 1 Hz, 1H, Azetidinon-H), 5.59 (d, J = 7.5 Hz, 1H, NHCHCO), 6.50 (m, 1H, Furyl-H), 6.81 (d, J = 4.5 Hz, 1H, Furyl-H), 6.94 (s, 1H, $CHPh_2$), 7.2–7.6 (m, 16H, Ph, Furyl-H), 7.70 (s, 1H, CH=N), 9.25 (d, J = 7.5 Hz, 1H, NH).

IR (KBr) 3310 (NH, Amid), 1789 (C=O, β-Lactam), 1732 (C=O, Ester), 1679 cm$^{-1}$ (C=O, Amid, Semicarbazid; C=N).

Beispiel 17

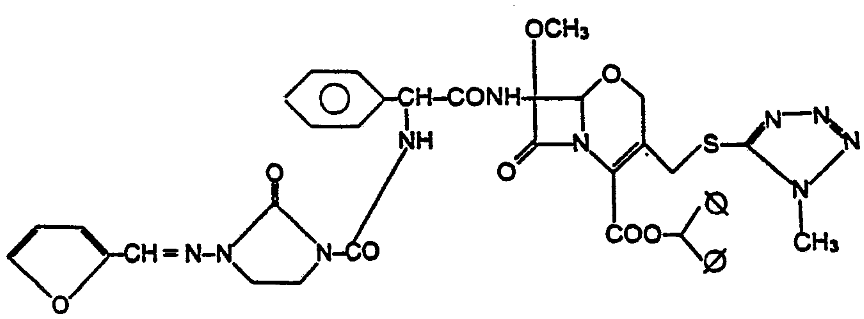

98 mg 7-[(2-Oxo-3-furfurylidenaminoimidazolin-1-yl)carbonylaminophenylacetamido-3-(1-methyl-1-H-tetrazol-5-ylthiomethyl)-1-oxadethia-3-cephem-4-carbonsäurediphenylmethylester

werden in 0.8 ml absol. Dichlormethan unter Stickstoffatmosphäre gelöst und auf −70 °C gekühlt. Dazu tropft man 15 µl (0.22 mmol, 1.8 Äquiv. -iodometr. bestimmt) frisch hergestelltes t-Butyl-hypochlorit und sofort danach während 30 sec. 90 µl (0.18 mmol, 1.5 Äquiv.) einer frisch hergestellten 2 M Lösung von Lithiummethoxid in wasserfreiem Methanol. Anschliessend wird 12.0 min. bei −70 °C gerührt, dann werden rasch 0.1 ml Eisessig zugegeben und es wird weitere 2 min. bei −70 °C gerührt. Zur Aufarbeitung wird die Reaktionsmischung unter Rühren in 100 ml eiskalte 0.1 N $Na_2S_2O_3$-Lösung gegossen und mit 3 × 50 ml Dichlormethan extrahiert. Die organischen Extrakte werden mit 2 × 80 ml kalter gesättigter $NaHCO_3$-Lösung und mit Wasser gewaschen und über Magnesiumsulfat getrocknet. Nach Abdampfen des Lösungsmittels i.Vak. werden durch Verreiben des Rückstands mit Ether 92 mg 7-Methoxy-7-[(2-oxo-3-furfuryliden-amino-imidazolidin-1-yl)carbonylamino-phenylacetamido]-3-(1-methyl-1-H-tetrazol-5-yl-thiomethyl)-1-oxa-dethia-3-cephem-4-carbonsäure-diphenylmethylester erhalten.
Schmp. ab 145 °C (Z).
RF: 0.26 (Essigester)
IR (KBr): 1788, 1730, 1682 cm$^{-1}$.

Beispiel 18

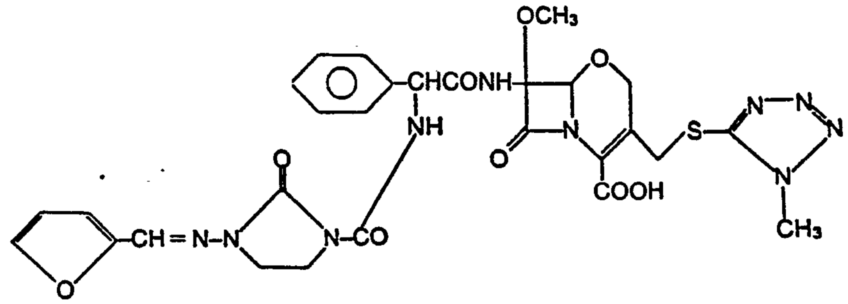

100 mg 7-Methoxy-7-phenylglycylamino-3-(1-methyl-1-H-tetrazol-5-ylthiomethyl)-1-oxadethia-3-cephem-4-carbonsäuretrifluoracetat werden in 80%igen wässrigem Tetrahydrofuran gelöst und bei konstanten pH (7.2) mit 41 mg 1-Chlorcarbonyl-2-oxo-3-furfurylidenaminoimidazol unter Kühlung umgesetzt. Danach wird mit Wasser verdünnt, zweimal mit Essigester ausgeschüttelt, mit frischem Essigester überschichtet und bis pH 1.8 angesäuert und nochmals mit Essigester extrahiert. Trocknen und Einengen der Essigesterphasen ergibt

7-Methoxy-7[D,L-(2-oxo-3-furfuryliden-amino-imidazolidin-1-yl)carbonylamino-phenylacetamido]-3-(1-methyl-1-H-tetrazol-5-yl-thiomethyl)-1-oxadethia-3-cephem-4-carbonsäure (siehe Beispiel 11).

Beispiel 19

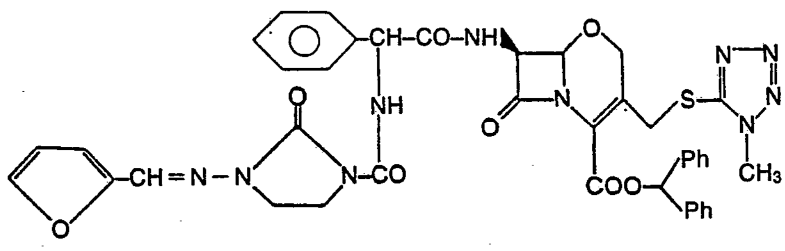

Wie für Beispiel 21 beschrieben, erhält man aus 90 mg

7-Amino-3-(1-methyl-1-H-tetrazol-5-yl-thiomethyl)-1-oxadethia-3-cephem-4-carbonsäure-diphenylmethylester

und 87 mg

2-(2-Oxo-3-furfurylamino-imidazolidin-1-yl)-carbonylamino-phenylessigsäure

nach 12 h bei 0 °C und Chromatographie (Ethylacetat : Toluol 95 : 5) 50 mg des entsprechenden Oxacephems als farblosen Hartschaum.

$^1$H-NMR (250 MHz, $CDCl_3$-DMSO) δ 3.72–3.85 (m, 4H, $CH_2N$), 4.01 (s, 3H, $NCH_3$), 4.22, 4.30 (AB-Signal, J = 14 Hz, $CH_2S$), 4.59, 4.72 (AB-Signal, J = 18 Hz), 5.01 (d, J = 4 Hz, 1H, H–6), 5.54 (dd, J = 4 Hz, J = 9 Hz, 1H, H–7), 5.66 (d, J = 7,5 Hz, NHCHCO), 6.50, 6.81 (m, 2H, Furyl-H), 6.99 (s, 1H, $CHPh_2$), 7.25–7.75 (m, 17H, $C_6H_5$, Furyl-H, CH = N), 8.40 (d, J = 9 Hz, 1H, NH), 9.16 (d, J = 7.5 Hz, 1H, CHNHCO).

Beispiel 20

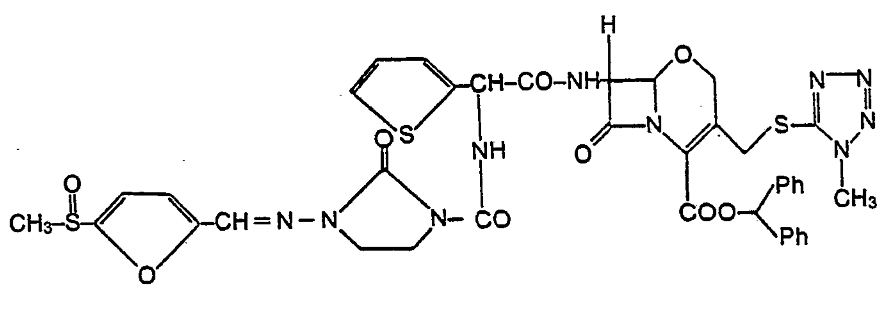

Wie in Beispiel 6 beschrieben, erhält man aus 290 mg

7-Amino-3-(1-methyl-1-H-tetrazol-5-yl-thiomethyl)-1-oxadethia-3-cephem-4-carbonsäure-diphenylmethylester

und 724 mg

2-[2-oxo-3-(4-methylsulphinylfuryliden)-amino-imidazolidin-1-yl]-carbonylamino-thienylessigsäure

nach 4 h bei −20 °C 312 mg des entsprechenden Oxacephems.

$^1$H-NMR (250 MHz, $CDCl_3$) δ 2.45 (s, 3H, $CH_3SO$–), 3.7–4.0 (m, $CH_2N$), 3.83 (s, $NCH_3$) zus. 7H, 4.25 (m, 2H, $CH_2S$), 4.57 (m, 2H, $CH_2O$), 5.03 (d, J = 4 Hz, 1H,

H–6), 5.55 (dd, J=4 Hz, J=8.5 Hz, 1H, H–7), 5.82 (d, J=7.5 Hz, 1H, NHCHCO), 6.40 (d, J=4 Hz, 1H), 6.8–7.7 (m, 17H, Furyl-H, Thienyl-H, CH=N, $C_6H_5$, NH), 9.03 (d, J=7.5 Hz, 1H, CHNHCO).

Beispiel 21

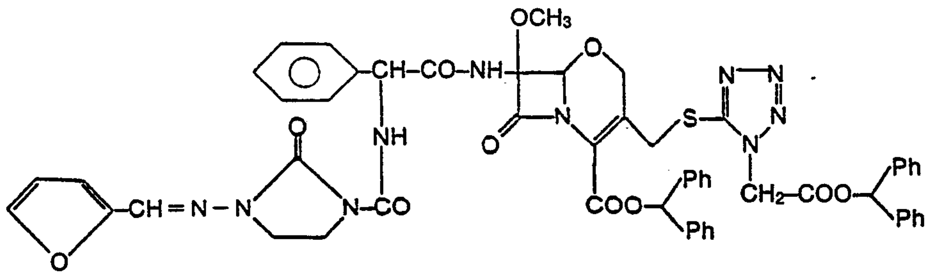

Wie für Beispiel 10 beschrieben, erhält man aus 1,34 g

7-Amino-7-methoxy-3-(1-carboxymethyl-1-H-tetrazol-5-yl-thiomethyl)-1-oxadethia-3-cephem-4-carbonsäure-bis-diphenylmethylester

und 0,87 g

2-(2-Oxo-3-furfurylidenamino-imidazolidin-1-yl)-carbonylamino-phenylessigsäure

0,53 g des entsprechenden Oxacephems als Hartschaum. Rf = 0.35 (Toluol : Ethylacetat 6 : 4).

$^1$H-NMR (250 MHz, $CDCl_3$) δ 3.38, 3.53 (s, 3H, $OCH_3$), 3.84, 3.86 (s, 3H, $NCH_3$), 3.5–4.0 (m, $CH_2N$), 4.2 (m, 2H, $CH_2S$), 4.53, 4.70 (AB-Signal, J=19 Hz, 2H, $CH_2O$), 4.85, 4.80 (s, 1H, Azetidinon-H), 5.07 (s, 2H, N–$CH_2$–COO), 5.63, 5.71 (d, J=7.5 Hz, 1H, NHCHCO), 6.48, 6.81 (m, 2H, Furyl-H), 6.92 (s, 1H, $CHPh_2$), 7.2–7.6 (m, 28H, $C_6H_5$, NH, CH=N, Furyl-H)m 9.11, 9.18 (d, J=7.5 Hz, 1H, CHNHCO).

IR (KBr) 1778 (C=O, β-Lactam), 1750 (C=O, Ester), 1717 (C=O, Ester), 1676 $cm^{-1}$ (C=O, Amid; C=N).

Beispiel 22

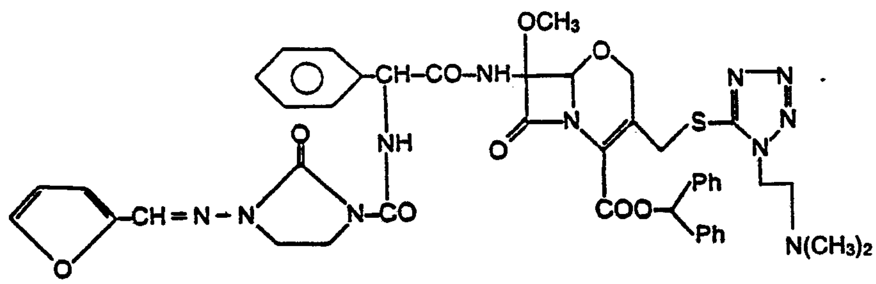

Wie für Beispiel 10 beschrieben, erhält man aus 737 mg

7-Amino-7-methoxy-3-(1-dimethylaminoethyl-1-H-tetrazol-5-yl-thiomethyl)-1-oxadethia-3-cephem-4-carbonsäurediphenylmethylester und 604 mg

2-(2-Oxo-3-furfurylidenamino-imidazolidin-1-yl)-carbonylamino-phenylessigsäure

nach Chromatographie (Ethylacetat : Aceton) und Behandeln mit Chloroform/Ethylacetat 235 mg des entsprechenden Oxacephems in Form eines Pulvers.

$^1$H-NMR (250 MHz, $CDCl_3$) δ 2.23 (s, 6H, $N(CH_3)_2$), 2.70 (t, J=6 Hz, 2H, $CH_2N$), 3.32, 3.55 (s, 3H, $OCH_3$), 3.7–4.1 (m, 4H, $CH_2N$), 4.24 (t, J=6 Hz, $CH_2N$), 4.27 (m, $CH_2S$) zus. 4H, 4.60, 4.81 (AB-Signal, J=18 Hz, 2H, $CH_2O$), 4.89, 5.05 (s, 1H, Azetidinon-H), 5.66, 5.72 (d, 3=7 Hz, 1H, NHCHCO), 6.49, 6.82 (m, 2H, Furyl-H), 6.95 (s, 1H, $CHPh_2$), 7.2–7.6 (m, 18H, $C_6H_6$, NH, CH=N, Furyl-H), 9.17, 9.29 (d, J=7 Hz, CHNHCO).

IR (KBr) 1789 (C=O, β-Lactam), 1714 (C=O, Ester), 1679 $cm^{-1}$ (C=O, Amid; C=N).

Beispiel 23

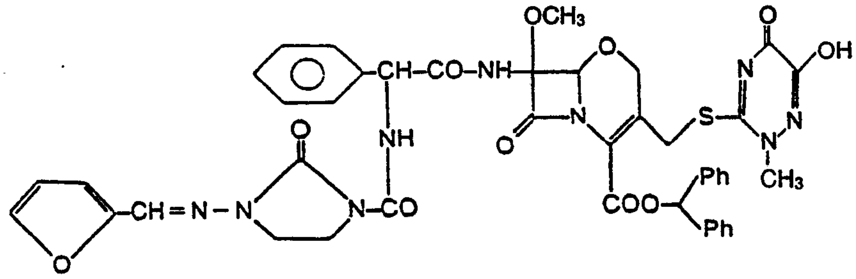

Wie für Beispiel 10 beschrieben, erhält man aus 387 mg
7-Amino-7-methoxy-3-(1-methyl-5-hydroxy-4-oxo-1,4-dihydro-1,3,6-triazin-2-yl)-thiomethyl-1-oxadethia-3-cephem-4-carbonsäure-diphenylmethylester
und 325 mg
2-(2-Oxo-3-furfurylidenamino-imidazolidin-1-yl)-carbonylamino-phenylessigsäure
213 mg des entsprechenden Oxacephems in Form eines Pulvers.

RF = 0,3 (BABA).

$^1$H-NMR (250 MHz, DMSO) δ 3.34, 3.48, 3.53 (s, 6H, $OCH_3$, $NCH_3$), 3.6–4.3 (m, 6H, $CH_2N$, $CH_2S$), 4.55 (m, 2H, $CH_2O$), 5.16, 5.24 (s, 1H, H–6), 5.67, 5.69 (d, J=7.5 Hz, 1H, NHCHCO), 6.5, 6.8 (m, 2H, Furyl-H), 7.00 (s, 1H, $CHPh_2$), 7.2–7.7 (m, 18H, $C_6H_5$, NH, CH=N, Furyl-H), 9.14, 9.20 (d, J=7.5 Hz, CHNHCO), 9.41 (6s, 1H, OH).

IR (KBr) 3400 (OH), 1785 (C=O, β-Lactam), 1727 (C=O, Ester), 1680 (C=O, Amid; C=N).

Beispiel 24

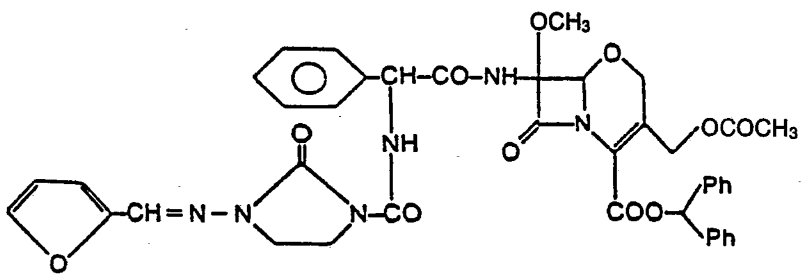

Wie für Beispiel 10 beschrieben, erhält man aus 2,13 g
3-Acetoxymethyl-7-amino-7-methoxy-1-oxa-dethia-3-cephem-4-carbonsäurediphenylmethylester
und 2,18 g
2-(2-Oxo-3-furfurylamino-imidazolidin-1-yl)-carbonylamino-phenylessigsäure
und Chromatographie des Rohprodukts (Toluol : Ethylacetat, 1 : 4) 1,67 g des entsprechenden Oxacephems als hellen Hartschaum.

$^1$H-NMR (250 MHz, $CDCl_3$) δ 1.98, 2.05 (s, 3H, $OCOCH_3$), 3.29, 3.48 (s, 3H, $OCH_3$), 3.5–4.0 (m, 4H, $CH_2N$), 4.4–4.8 (m, 4H, $CH_2O$), 5.01, 5.06 (s, 1H, H–6), 5.65, 5.76 (d, J=7 Hz, 1H, NHCHCO), 6.47, 6.79 (m, 2H, Furyl-H), 6.92 (s, 1H, $CHPh_2$), 7.2–7.7 (m, 18H, $C_6H_5$, NH, CH=N, Furyl-H), 9.15, 9.27 (d, 1H, CHNHCO).

IR (KBr) 3299 (NH), 1784 (C=O, β-Lactam), 1720, 1730 (C=O, Ester), 1677 (C=O, Amid; C=N).

Beispiel 25

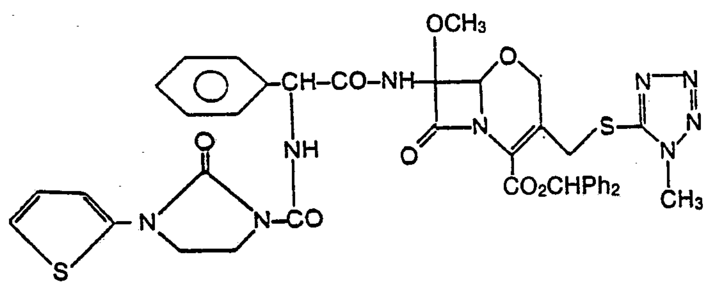

Wie für Beispiel 10 beschrieben, erhält man aus 1,53 g
7-Amino-7-methoxy-3-(1-methyl-1-H-tetrazol-5-yl-thiomethyl)-1-oxadethia-3-cephem-4-carbonsäurediphenylmethylester
und 1,35 g
2-[2-oxo-3-(2-thienyl)-imidazolidin-1-yl]-carbonylamino-phenylessigsäure
nach Chromatographie (Ethylacetat : Toluol 57 : 43) 1,57 g
7-Methoxy-7-{D,L-[2-oxo-3-(2-thienyl)-imidazolidin-1-yl]-carbonylamino-phenylacet-amido}-3-(1-methyl-1-H-tetrazol-5-yl-thiomethyl)-1-oxadethia-3-cephem-4-carbonsäurediphenylmethylester.

Schmp. 162 °C (Zers.), Rf = 0.22 (Ethylacetat : Toluol 1 : 1).

$^1$H-NMR (250 MHz, $CDCl_3$) δ 3.31, 3.52 (s, 3H, $OCH_3$), 3.82, 3.84 (s, 3H, $NCH_3$), 3.7–4.1 (m, 4H, $CH_2N$), 4.2–4.6 (m, 4H, $CH_2S$, $CH_2O$), 5.03, 5.09 (s, 1H, Azitidinon-H), 5.64, 5.83 (d, J=7 Hz, 1H, NHCHCO), 6.45 (m, 1H, Thienyl-H), 6.8–7.6 (m, 19H, Ph, Thienyl-H, $CHPh_2$, NH), 9.07, 9.17 (d, J=7 Hz, 1H, CHNHCO).

IR (KBr) 1787 (C=O, β-Lactam), 1711 (C=O, Ester), 1680 $cm^{-1}$ (C=O, Amid).

Beispiel 26

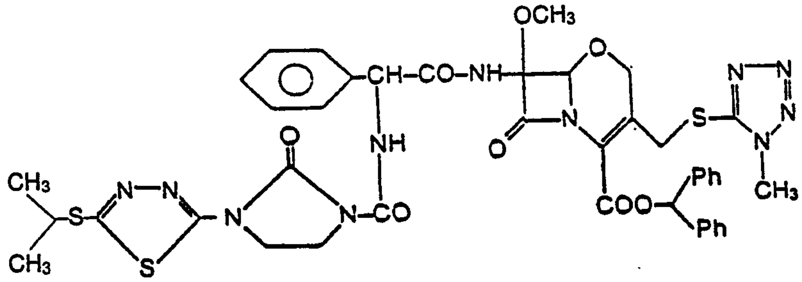

Wie für Beispiel 10 beschrieben, erhält man aus 241 mg 7-Amino-7-methoxy-3-(1-methyl-1-H-tetrazol-5-yl-thiomethyl)-1-oxadethia-3-cephem-4-carbonsäurediphenylmethylester und 353 mg 2-[2-Oxo-3-(5-isopropylthio-1-thia-3,4-diazol-2-yl)-imidazolidin-1-yl]-carbonylamino-phenylessigsäure nach Chromatographie 50 mg 17-Methoxy-7{D,L-[2-oxo-3-(5-isopropylthio-1-thia-3,4-diazol-2-yl)-imidazolidin-1-yl]-carbonylamino-phenylacetamido}-1-oxa-dethia-3-cephem-4-carbonsäurediphenylmethylester.

Schmp.: 177 °C (Zers.), Rf: 0.47 (Ethylacetat).

$^1$H-NMR (250 MHz, $CDCl_3$) δ 1.44, 1.47 (s, 6H, $CH_3$), 3.33, 3.52 (s, 3H, $OCH_3$), 3.85, 3.87 (s, $NCH_3$), 3.8 (m, CH), 4.0–4.3 (m, $CH_2N$, $CH_2S$), zus. 10H, 4.54 (m, 2H, $CH_2O$), 5.04, 5.08 (s, 1H, Azetidinon-H), 5.52, 5.55 (d, J=7.5 Hz, 1H, NHCHCO), 6.52, 6.54 (s, 1H, NH), 6.89, 6.91 (s, 1H, $CHPh_2$), 7.2–7.6 (m, 15H, Ph), 8.95, 9.02 (d, J=7.5 Hz, 1H, CHNHCO).

Beispiel 27

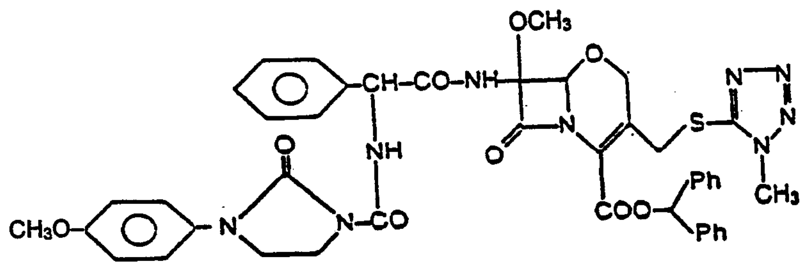

Wie für Beispiel 10 beschrieben, erhält man aus 4,07 g 7-Amino-7-methoxy-3-(1-methyl-1-H-tetrazol-5-yl-thiomethyl)-1-oxadethia-3-cephem-4-carbonsäurediphenylmethylester und 3,84 g 2-[2-oxo-3-(4-anisyl)-imidazolidin-1-yl]-carbonylaminophenylessigsäure nach Chromatographie an 500 g Kieselgel (Ethylacetat : Toluol 65 : 45) 1,52 g 7-Methoxy-7-{D,L-[2-oxo-3-(4-anisyl)-imidazolidin-1-yl]-carbonylamino-phenylacetamido}-3-(1-methyl-1-H-tetrazol-5-yl-thiomethyl)-1-oxadethia-3-cephem-4-carbonsäurediphenylmethylester.

Schmp.: 156 °C (Zers.),

Rf = 0,29 (Ethylacetat : Toluol 65 : 45).

$^1$H-NMR (250 MHz, $CDCl_3$) δ 3.36, 3.53 (s, 3H, $OCH_3$), 3.81, 3.83 (s, 6H, $OCH_3$, N–$CH_3$), 3.7–4.0 (m, 4H, $CH_2N$), 4.25, 4.5 (m, 4H, $CH_2S$, $CH_2O$), 5.03, 5.08 (s, 1H, Azetidinon-H), 5.61, 5.67 (d, J=7 Hz, 1H, NHCHCO), 6.91 (d, J=8 Hz, Anisyl-H), 6.91 (s, $CHPh_2$) zus. 3H, 7.2–7.6 (m, 17H, Ph, Anisyl-H, NH), 9.24, 9.30 (d, J=7 Hz, CHNHCO).

IR (KBr) 3295 (NH, Amid), 1787 (C=O, β-Lactam), 1711 (C=O, Ester), 1675 $cm^{-1}$ (C=O, Amid).

Beispiel 28

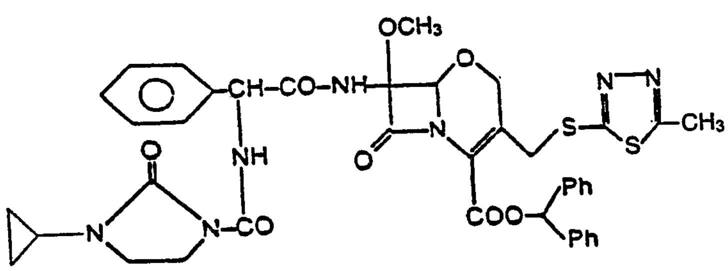

Wie für Beispiel 10 beschrieben, erhält man aus 370 mg 7-Amino-7-methoxy-3-(2-methyl-1-thia-3,4-diazol-5-yl-thiomethyl)-1-oxadethia-3-cephem-4-carbonsäurediphenylmethylester und 278 mg

2-(3-Cyclopropyl-2-oxo-imidazolidin-1-yl)-carbonylamino-phenylessigsäure
nach Chromatographie (Ethylacetat) 123 mg
7-Methoxy-7-[D,L-(3-cyclopropyl-2-oxo-imidazolidin-1-yl)-carbonylamino-phenylacetamido]-3-(2-methyl-1-thia-3,4-diazol-5-yl-thiomethyl)-1-oxadethia-3-cephem-4-carbonsäure-diphenylmethylester.
Schmp. ~ 190 °C (Zers.), Rf = 0,27 (Ethylacetat).
$^1$H-NMR (250 MHz, $CDCl_3$) δ 0.75 (m, 4H, Cyclopropyl-H), 2.50 (m, 1H, Cyclopropyl-H), 2.71, 2.72 (s, 3H, $CH_3$), 3.4, 3.8 (m, $CH_2N$), 3.40, 3.53 (s, $OCH_3$) zusammen 7H, 4.2–4.55 (m, 4H, $CH_2S$, $CH_2O$), 5.04, 5.08 (s, 1H, Azetidinon-H), 5.53 (d, J=8 Hz, 1H, NHCHCO), 6.83, 6.87 (s, 1H, NH), 6.92, 6.94 (s, 1H, $CHPh_2$), 7.2–7.6 (m, 15H, Ph), 9.14 (d, J=8 Hz, 1H, CHNHCO).
IR (KBr) 1783 (C=O, β-Lactam), 1714 (C=O, Ester), 1681 $cm^{-1}$ (C=O, Amid).

Beispiel 29

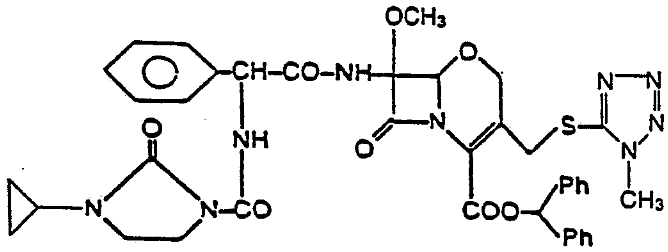

Wie für Beispiel 10 beschrieben, erhält man aus 2,54 g
7-Amino-7-methoxy-3-(1-methyl-1-H-tetrazol-5-yl-thiomethyl)-1-oxadethia-3-cephem-4-carbonsäurediphenylmethylester
und 1,97 g
2-(3-Cyclopropyl-2-oxo-imidazolidin-1-yl)-carbonylaminophenylessigsäure
nach Chromatographie (Ethylacetat : Toluol 95 : 5) 1,62 g
7-Methoxy-2-[D,L-(3-cyclopropyl-2-oxo-imidazolidin-1-yl)-carbonylamino-phenylacetamido]-3-(1-methyl-1-H-tetrazol-5-yl-thiomethyl)-1-oxadethia-3-cephem-4-carbonsäurediphenylmethylester.
Schmp.: 151 °C (Zers.), Rf = 0.36 (Ethylacetat).
$^1$H-NMR (250 MHz, $CDCl_3$) δ 0.75 (m, 4H, Cyclopropyl-H), 2.50 (m, 1H, Cyclopropyl-H), 3.4, 3.8 (m, $CH_2N$), 3.43, 3.54 (s, $OCH_3$), 3.87, 3.89 (s, $NCH_3$) zusammen 10H, 4.3 (m, 2H, $CH_2S$), 4.55 (m, 2H, $CH_2O$), 5.04, 5.09 (s, 1H, Azetidinon-H), 5.48 (d, J=7 Hz, 1H, NHCHCO), 6.69, 6.78 (s, 1H, NH), 6.91, 6.95 (s, 1H, $CHPh_2$), 7.2–7.6 (m, 15H, Ph), 9.13 (d, J=7 Hz, 1H, CHNHCO).
IR (KBr)1785 (C=O, β-Lactam), 1715 (C=O, Ester), 1660 $cm^{-1}$ (C=O, Amid).

Beispiel 30

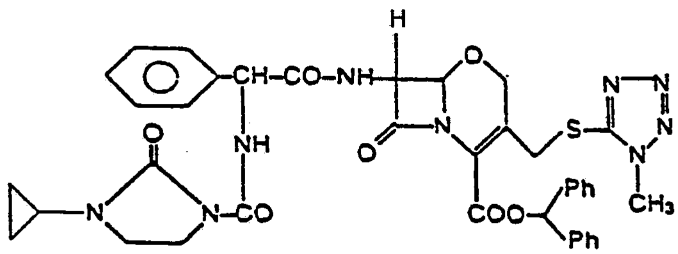

Wie für Beispiel 21 beschrieben, erhält man aus 441 mg
7-Amino-3-(1-methyl-1-H-tetrazol-5-yl-thiomethyl)-1-oxadethia-3-cephem-4-carbonsäure-diphenylmethylester
und 210 mg
2-(3-Cyclopropyl)-2-oxo-imidazolidin-1-yl)-carbonylamino-phenylessigsäure
nach 3 h bei −20 °C und 4 h bei 0 °C 322 mg
7-[D-(3-cyclopropyl)-2-oxo-imidazolidin-1-yl)-carbonylamino-phenylacetamido]-3-(1-methyl-1-H-tetrazol-5-yl-thiomethyl)-1-oxadethia-3-cephem-4-carbonsäurediphenylmethylester
als Pulver.
Schmp.: 148–153 °C (Zers.).
$^1$H-NMR (250 MHz, $CDCl_3$) δ 0.75 (m, 4H, Cyclopropyl-H), 2.50 (m, 1H, Cyclopropyl-H), 3.5–3.8 (m, 4H, $CH_2N$), 4.98 (s, 1H, N-$CH_3$), 4.25 (m, 2H, $CH_2S$), 4.6 (m, 2H, $CH_2O$), 5.00 (d, J=4 Hz, 1H, Azetidinon-H), 5.51 (dd, J=4 Hz, J=9 Hz, 1H, Azetidinon-H), 5.66 (d, J=7.5 Hz, 1H, NHCHCO), 6.96 (s, 1H, $CHPh_2$), 7.2–7.7 (m, 16H, Ph, NH), 9.14 (d, J=7.5 Hz, 1H, CHNHCO).
IR (KBr) 1782 (C=O, β-Lactam), 1713 (C=O, Ester), 1664 $cm^{-1}$ (C=O, Amid).

Abspaltung der Diphenylmethylester-Schutzgruppe

Wie für die Beispiele 2, 5, 7, 9 und 11 explizit beschrieben, wurden aus den oben erhaltenen Diphenylmethylestern die entsprechenden Säuren freigesetzt:

Beispiel 31

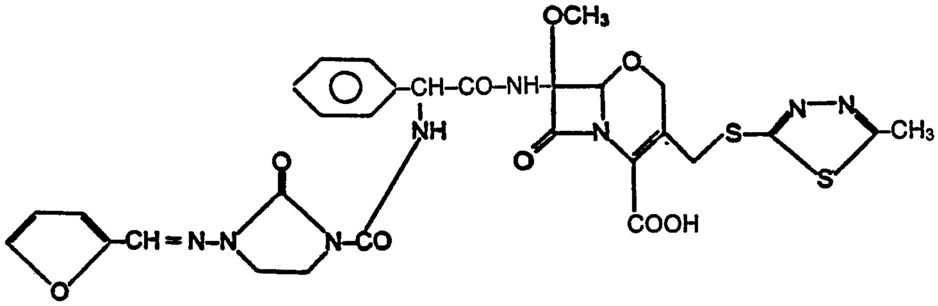

Pulver, Schmp.: ab 146 °C (Zers.), Rf = 0.28 (BABA)
$^1$H-NMR (250 MHz, DMSO) δ 2.67, 2.69 (s, 3H, $CH_3$), 3.06, 3.32 (s, $OCH_3$), 3.4, 3.75 (m, $CH_2N$) zusammen 7H, 5.05, 5.09 (s, 1H, H–6), 5.64 (d, J = 7.5 Hz, 1H, NHCHCO), 6.62, 6.84 (m, 2H, Furyl-H), 6.9–7.5 (m, 6H, $C_6H_5$, NH), 7.74, 7.84 (s, 2H, CH = N, Furyl-H), 8.95, 9.11 (d, J = 7.5 Hz, 1H, CHNHCO), 9.44, 9.48 (s, 1H, COOH).
IR (KBr) ~ 3300 (b, OH), 1781 (C = O, β-Lactam, 1680–1720 $cm^{-1}$ (C = O, Säure, Amid; C = N).

Beispiel 32

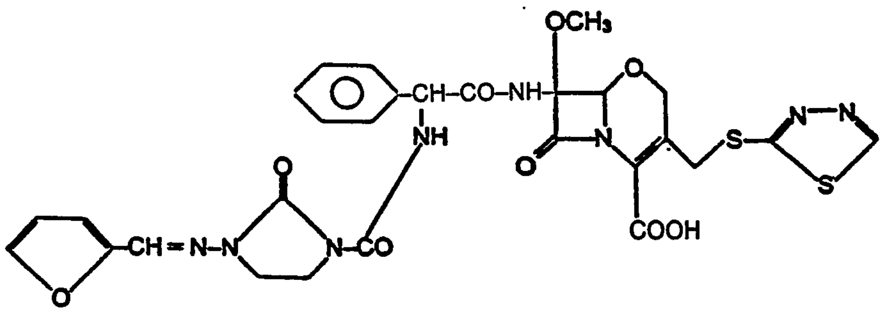

Pulver, Schmp.: ab 143 °C (Zers.), Rf = 0.27 (BABA).
$^1$H-NMR (250 MHz, DMSO) δ 3.06, 3.35 (s, $OCH_3$), 3.4, 3.75 (m, $CH_2N$), zusammen 7H, 4.1–4.6 (m, 4H, $CH_2S$, $CH_2O$), 5.06, 5.09 (s, 1H, H–6), 5.63 (d, J = 7.5 Hz, 1H, NHCHCO), 6.63, 6.84 (m, 2H, Furyl-H), 6.9–7.5 (m, 6H, $C_6H_5$, NH), 7.74, 7.84 (s, 2H, CH = N, Furyl-H), 8.95, 9.10 (d, J = 7.5 Hz, 1H, CHNHCO), 9.44, 9.48 (s, 1H, COOH), 9.58, 9.60 (s, 1H, Thiadiazol-H).
IR (KBr) ~ 3300 (b, OH), 1780 (C = O, β-Lactam), 1660–1721 $cm^{-1}$ (C = O, Säure, Amid; C = N).

Beispiel 33

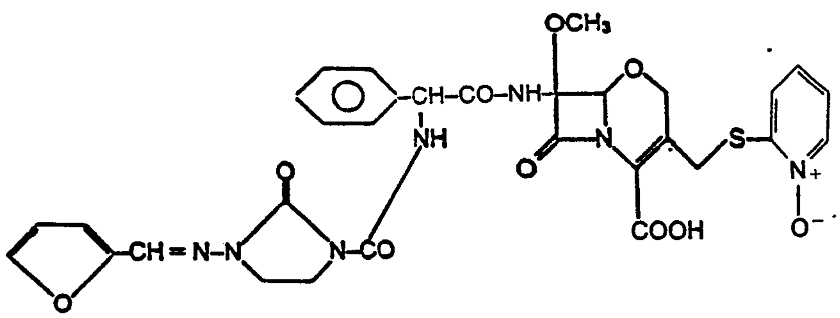

Pulver, Schmp.: ab 148 °C (Zers.), Rf = 0.15 (BABA).
$^1$H-NMR (250 MHz, DMSO) δ 3.37 (s, $OCH_3$), 3.4, 3.7 (m, $CH_2N$) zus. 7H, 3.9–4.5 (m, 4H, $CH_2S$, $CH_2O$), 5.13 (s, 1H, H–6), 5.65 (d, J = 7.5 Hz, 1H, NHCHCO), 6.65, 6.86 (m, 2H, Furyl-H), 7.2–7.5 (m, 9H, $C_6H_5$, Pyridyl-H, NH), 7.75, 7.86 (s, 2H, CH = N, Furyl-H), 8.35 (d, J = 8 Hz, 1H, Pyridyl-H), 8.98 (d, J = 7.5 Hz, 1H, CHNHCO), 9.50 (s, 1H, COOH).
IR (KBr) 3500–3100 (b, OH), 3300 (NH), 1785 (C = O, β-Lactam), 1725 (C = O, Säure), 1680 $cm^{-1}$ (C = O, Amid; C = N).

Beispiel 34

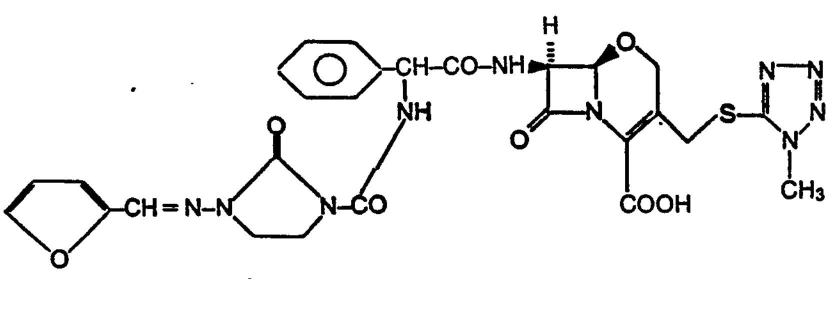

Farbloses Pulver
$^1$H-NMR (250 MHz, DMSO) δ 3.75–3.85 (m, $CH_2N$), 3.95 (s, 3H, N–$CH_3$), 4.17, 4.39 (AB-Signal, J = 12.5 Hz, 2H, $CH_2S$), 4.43, 4.68 (AB-Signal, J = 18,5 Hz, 2H, $CH_2O$), 5.01 (d, J = 4Hz, 1H, H–6), 5.69 (dd, J = 4 Hz, J = 9 Hz, 1H, H–7), 5.67 (d, J = 7.5 Hz, 1H, NHCHCO), 6.51, 6.81 (m, 2H, Furyl-H), 7.3–7.6 (m, 6H, $C_6H_5$, Furyl-H), 7.72 (s, 1H, CH = N), 8.42 (d, J = 9 Hz, 1H, NH), 9.17 (d, J = 7.5 Hz, 1H, CHNHCO).

Beispiel 35

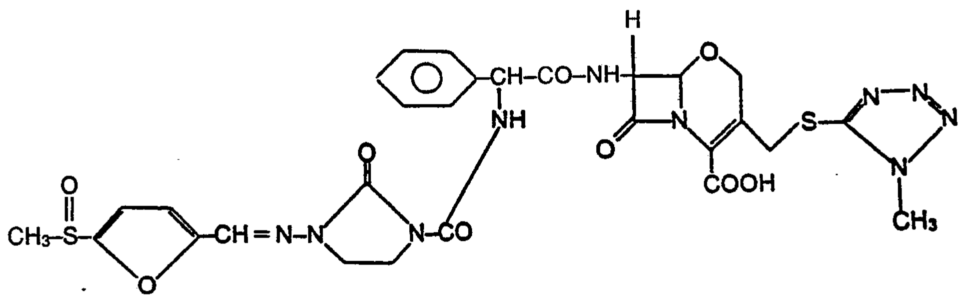

Gelbes Pulver, Schmp.: ab 154 °C (Zers.), Rf = 0.3 (BABA).
$^1$H-NMR (250 MHz, DMSO) δ 2.50 (s, $CH_3SO$–), 3.4, 4.3 (m, 4H, $CH_2N$), 3.85 (s, 3H, $NCH_3$), 4.2–4.6 (m, 4H, $CH_2S$, $CH_2O$), 5.03 (d, J = 4 Hz, 1H, H–6), 5.67 (dd, J = 4 Hz, J = 9 Hz, 1H, H–7), 5.85 (d, J = 7.5 Hz, 1H, NHCHCO), 6.63, 6.87 (m, 2H, Furyl-H), 6.9–7.5 (m, 6H, $C_6H_5$, NH), 7.7 (s, 1H, CH = N), 8.97 (d, J = 7.5 Hz, 1H, CHNHCO), 9.59 (s, 1H, COOH).
IR (KBr) 3500–3100 (b, OH), 3306 (NH), 1783 (C = O, β-Lactam), 1728 (C = O, Säure), 1690 (C = O, Amid; C = N).

Beispiel 36

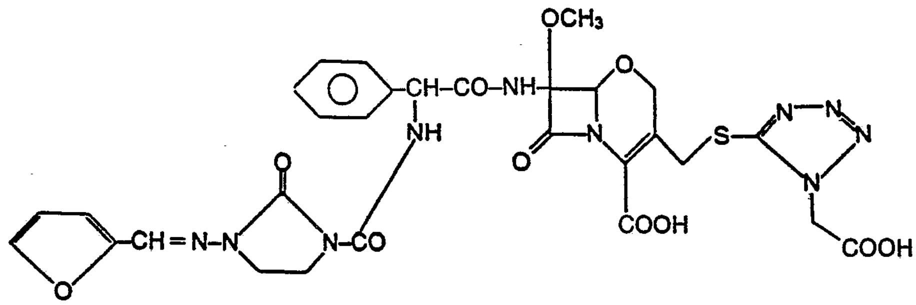

Hellbraunes Pulver, Schmp.:
ab 139 °C (Zusammenbacken)
ab ~ 159 °C (Zers.).
$^1$H-NMR (250 MHz, DMSO) δ 3.06, 3.37 (s, $OCH_3$), 3.4, 3.8 (m, $CH_2N$), zus. 7H, 4.2–4.6 (m, 4H, $CH_2S$, $CH_2O$), 5.0 (s, 2H, N–$CH_2$–COO), 5.06, 5.10 (s, 1H, H–6), 5.65 (d, J = 7 Hz, 1H, NHCHCO), 6.63, 6.88 (m, 2H, Furyl-H), 7.0–7.5 (m, 6H, $C_6H_5$, Furyl-H), 7.80 (s, 1H, CH = N), 7.87 (s, 1H, NH), 9.0, 9.1 (d, J = 7 Hz, 1H, CHNHCO), 9.3 (s, 1H, COOH), 9.5, 9.503 (s, 1H, COOH).
IR (KBr) 3550–3050 (b, OH), 1876 (C = O), β-Lactam, 1725, 1710 (C = O, Säure), 1680 $cm^{-1}$ (sh, C = O, Amid; C = N).

Beispiel 37

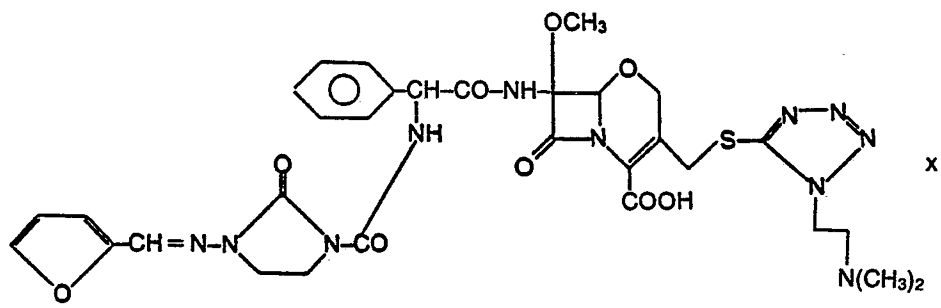

x $HOCOCF_3$

Helles Pulver
$^1$H-NMR (250 MHz, DMSO–$D_2O$) δ 3.1 (s, 6H, $NCH_3$), 3.3, 3.5 (s, $OCH_3$), 3.6 (t, $CH_2N$), zus. 5H, 3.7–4.8 (m, 10H, $CH_2S$, $CH_2O$, $CH_2N$), 5.01, 5.08 (s, 1H, H–6), 5.7 (m, 1H, NHCHCO), 6.5, 6.8 (m, 2H, Furyl-H), 7.0–7.6 (m, 9H, $C_6H_5$, Furyl-H, CH = N).
IR (KBr) 3600–3000 (b, OH), 1780 (C = O, β-Lactam).

Beispiel 38

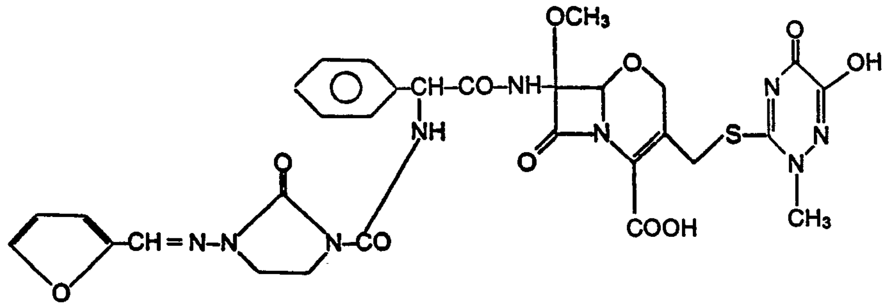

Hellgelbes Pulver

$^1$H-NMR (250 MHz, DMSO–$D_2O$) δ 3.3, 3.5, 3.55 (s, 6H, $OCH_3$, $NCH_3$), 3.5–4.6 (m, $CH_2S$, $CH_2N$, $CH_2O$), 5.7 (bs, 1H, NHCHCO), 6.5, 6.8 (m, 2H, Furyl-H), 7.2–7.7 (m, 7H, $C_6H_5$, Furyl-H, CH=N).

IR (KBr) 1783 (C=O, β-Lactam), 1775 (sh), 1590 $cm^{-1}$.

Beispiel 39

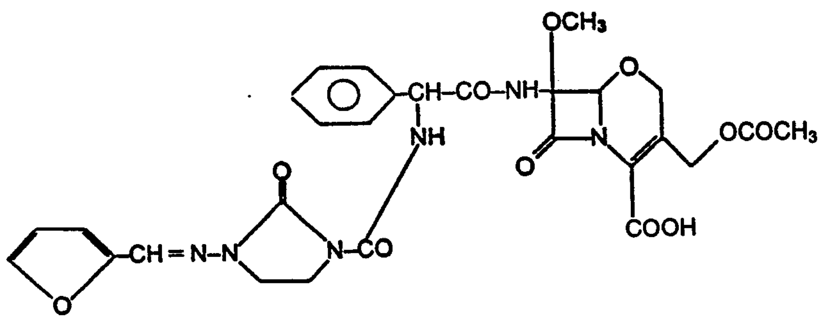

Farbloses Pulver, Schmp.: ab 150 °C (Zers.), Rf = 0.35 (BABA).

$^1$H-NMR (250 MHz, $CDCl_3$–DMSO) δ 2.01, 2.04 (s, 3H, $OCOCH_3$), 3.07, 3.38 (s, $OCH_3$), 3.3–3.9 (m, $CH_2N$), zus. 7H, 4.3–4.8 (m, 4H, $CH_2O$), 5.05, 5.10 (s, 1H, H–6), 5.65 (m, 1H, NHCHCO), 6.51, 6.82 (m, 2H, Furyl-H), 7.2–7.5 (m, 5H, $C_6H_5$), 7.77 (s, 1H, CH=N), 7.87 (bs, 1H, Furyl-H), 9.0, 9.1 (d, J=7 Hz, 1H, CHNHCO), 9.48, 9.50 (s, 1H).

Beispiel 40

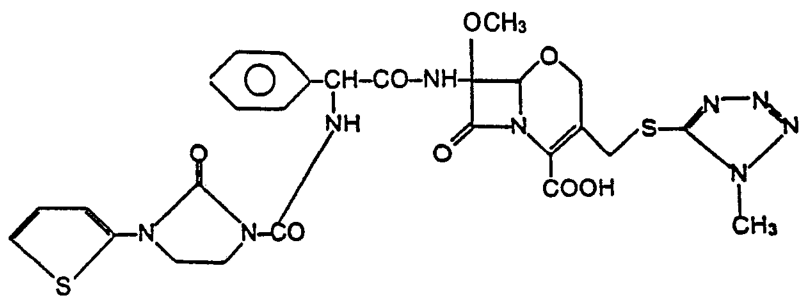

Helles Pulver, Schmp.: ab 171 °C (Zers.), Rf = 0.4 (BABA).

$^1$H-NMR (250 MHz, DMSO) δ 3.06, 3.34 (s, 3H, $OCH_3$), 3.91, 3.94 (s, $NCH_3$), 3.7–4.5 (m, $CH_2N$, $CH_2S$, $CH_2O$), zus. 11H, 5.03, 5.05 (s, 1H, H–6), 5.6 (m, 1H, NHCHCO), 6.4–7.5 (m, 9H, $C_6H_5$, NH, Thienyl-H), 8.98, 9.03 (d, J=7.5 Hz, 1H, CHNHCO), 9.46 (bs, 1H, COOH).

IR (KBr) 3600–3100 (b, OH), 3300 (NH), 1786 (C=O, β-Lactam), 1713 (C=O, Säure), 1680 $cm^{-1}$ (sh, C=O, Amid, C=N).

Beispiel 41

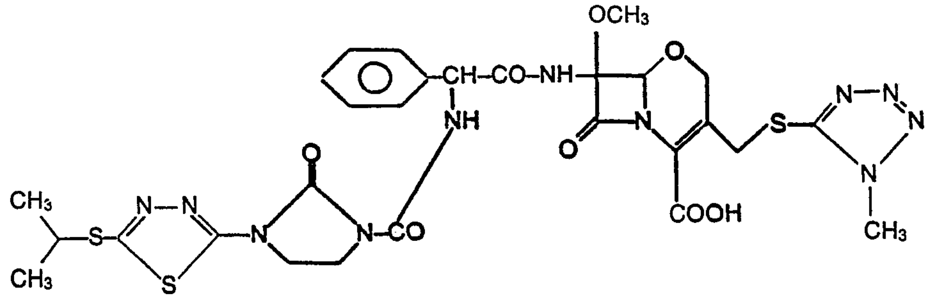

Pulver, Schm.: ab 174 °C (Zers.), Rf = 0.3 (BABA).

$^1$H-NMR (250 MHz, $CDCl_2$–DMSO) δ 1.44, 1.47 (s, 6H, $CH_3$), 3.1, 3.4 (s, $OCH_3$), 3.89 (s, $NCH_3$), 3.0–4.5 (m, $CH_2N$, $CH_2S$, $CH_2O$, CH), zus. 13H, 4.98 (bs, 1H, H–6), 5.52, 5.64 (d, J=7 Hz, 1H, NHCHCO), 6.49 (s, 1H, NH), 7.2–7.6 (m, 5H, $C_6H_5$), 8.9, 9.1 (m).

IR (KBr) 3600–3100 (b, OH), 3330 (NH), 1774 (C=O, β-Lactam), 1720 (C=O, Säure), 1677 $cm^{-1}$ (C=O, Amid; C=N).

Beispiel 42

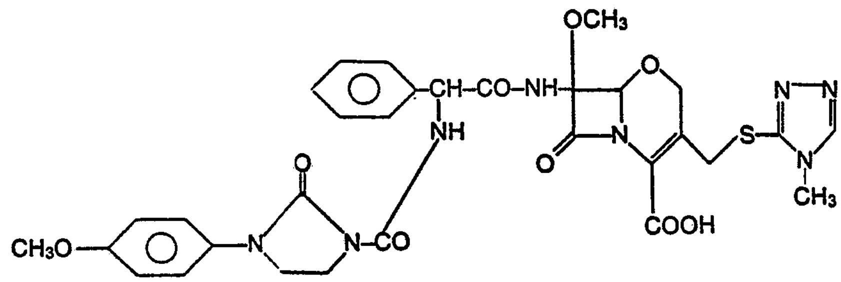

Farbloses Pulver, Schmp.: ab 160 °C (Zers.), Rf = 0.38 (BABA).

$^1$H-NMR (250 MHz, DMSO) δ 3.08, 3.37 (s, $OCH_3$), 3.4 (m, $CH_2N$), zus. 5H, 3.76 (s, $OCH_3$), 3.93, 3.95 (s, $NCH_3$), 3.7–4.0 (m, $CH_2N$), zus. 8H, 4.2–4.55 (m, 4H, $CH_2S$, $CH_2O$), 5.05–5.08 (s, 1H, H–6), 5.67 (d, J=9 Hz, 1H, NHCHCO), 6.85–7.55 (m, 10H, NH, H aromat.), 9.03, 9,19 (d, J=9 Hz, 1H, CHNHCO), 9.46, 9.48 (s, 1H, COOH).

IR (KBr) 3500–3150 (b, OH), 1784 (C=O, β-Lactam), 1708 (C=O, Säure), 1675 $cm^{-1}$ (sh, C=O, Amid; C=N).

Beispiel 43

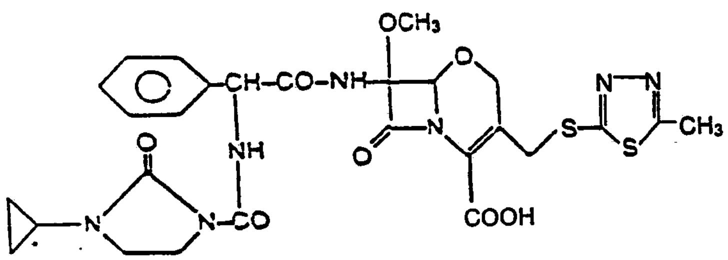

Pulver, Schm.: ab 155 °C (Zers.), Rf = 0.2 (BABA).

$^1$H-NMR (250 MHz, DMSO) δ 0.70 (m, 4H, Cyclopropyl-H), 2.5 (m, Cyclopropyl-H unter DMSO), 3.11, 3.39 (s, $OCH_3$), 3.3–3.7 (m, $CH_2N$), zus. 7H 4.2–4.6 (m, 4H, $CH_2S$, $CH_2O$), 5.09, 5.11 (s, 1H, H–6), 5.65 (d, J=8 Hz, 1H, NHCHCO), 7.1–7.5 (m, 5H, $C_6H_5$), 9.01 (d, J=8 Hz, 1H, NH), 9.16, 9.20 (s, 1H, NH), 9.44, 9.47 (s, 1H, COOH).

IR (KBr) 3600–3100 (b, OH), 1783 (C=O, β-Lactam), 1711 (C=O, Säure), 1675 $cm^{-1}$ (C=O, Amid; C=N).

Beispiel 44

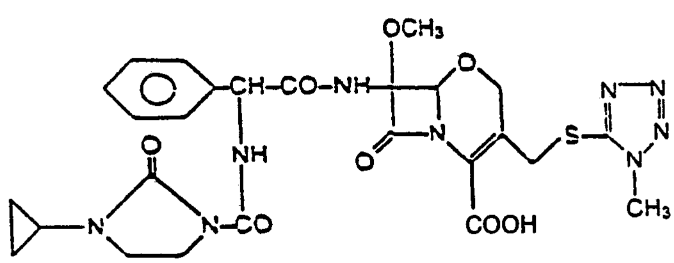

Pulver, Schmp.: ab 146 °C (Zers.), Rf = 0.2 (BABA).

$^1$H-NMR (250 MHz, DMSO) δ 0.65 (m, 4H, Cyclopropyl-H), 2.5 (m, Cyclopropyl-H unter DMSO), 3.07, 3.35 (s, $OCH_3$), 3.3–3.8 (m, $CH_2N$), zus. 7H, 3.94, 3.96 (s, 3H, $NCH_3$), 4.2–4.5 (m, 4H, $CH_2S$, $CH_2O$), 5.05, 5.08 (s, 1H, H–6), 5.61 (d, J=9 Hz, 1H, NHCHCO), 6.9–7.5 (m, 6H, $C_6H_5$, NH), 8.98, 9.15 (d, J=9 Hz, 1H, CHNHCO), 9.45 (bs, 1H, COOH).

IR (KBr) 3600–3100 (b, OH), 3300 (NH), 1787 (C=O, β-Lactam), 1712 (C=O, Säure), 1675 $cm^{-1}$ (sh, C=O, Amid; C=N).

Beispiel 45

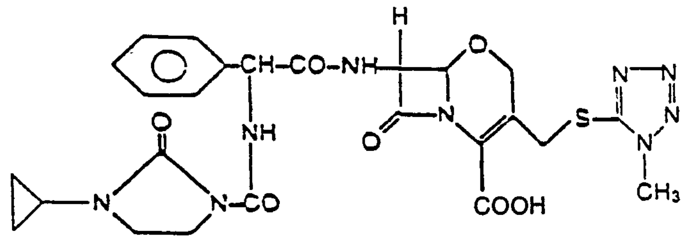

Helles Pulver, Schmp.: ab 143 °C (Zers.), Rf = 0.23 (BABA).

$^1$H-NMR (250 MHz, DMSO) δ 0.69 (m, 4H, Cyclopropyl-H), 2.5 (m, Cyclopropyl-H unter DMSO), 3.25–3.8 (m, $CH_2N$), zus. 7H, 3.95 (s, 3H, $NCH_3$), 4.2–4.6 (m, 4H, $CH_2S$, $CH_2O$), 5.1 (d, J = 4 Hz, 1H, H–6), 5.5 (dd, J = 4 Hz, J = 9 Hz, 1H, H–7), 5.64 (d, J = 8 Hz, 1H, NHCHCO), 7.1–7.5 (m, 6H, $C_6H_5$, NH), 9.0, 9.1 (d, J = 8 Hz, 1H, CHNHCO), 9.48 (s, 1H, COOH).

IR (KBr) 3500–3100 (b, OH), 1784 (C = O, β-Lactam), 1710 (C = O, Säure), 1780 $cm^{-1}$ (sh, C = O, Amid; C = N).

Beispiel 46

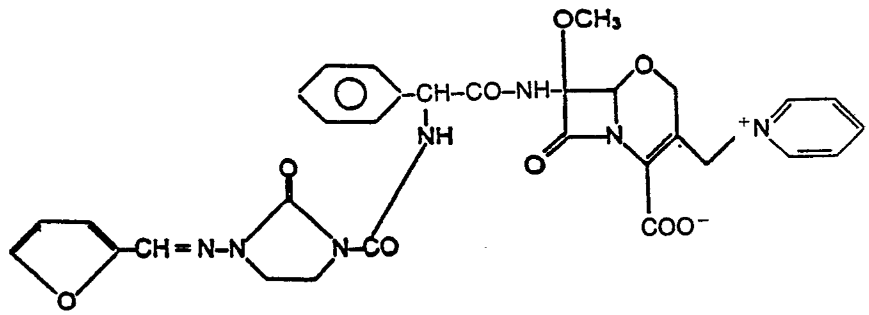

Zu einer Suspension von 687 mg
7-Methoxy-7-[(2-Oxo-3-furfurylidenamino-imidazolidin-1-yl)-carbonylamino-phenylacetamido]-3-acetoxymethyl-1-oxadethia-3-cephem-4-carbonsäure
in 5,8 ml Wasser wurden 42 mg $NaHCO_3$, dann 285 µl Pyridin und 2,3 g Kaliumthiocyanat gegeben. Mit 6 N HCl wurde der pH auf pH 6,5 gestellt, dann wurde 7 h auf 60 °C erwärmt. Nach Abkühlen wurden 12 ml Wasser zugegeben, unlösliches Material wurde abfiltriert und das Filtrat wurde an Diaion (RTM) HP-20 chromatographiert. (a.) $H_2O$ b.) $H_2O$ : MeOH 1 : 1). Das Lösungsmittel wurde i.Vak. abgedampft, und der Rückstand wurde mit wenig Methanol gewaschen. Man erhielt 129 mg
7-Methoxy-7-[(2-Oxo-3-furfuryliden-amino-imidazolidin-1-yl)-carbonylamino-phenylacetamido]-3-(1-pyridinomethyl)-1-oxadethia-3-cephem-4-carboxylat
in Form eines Pulvers. Rf = 0.3 ($CH_3CN$ : $H_2O$).

$^1$H-NMR (250 MHz, DMSO–$D_2O$) δ 3.1, 3.3 (s, $OCH_3$), 3.3–4.0 (m, $CH_2N$), zus. 7H, 4.3–4.5 (m, 2H, $CH_2O$), 4.98, 5.03 (s, 1H, H–6), 5.15–5.45 (m, 2H, $CH_2N^{\oplus}$), 5.66 (m, 1H, NHCHCO), 7.0–7.6 (m, 1H, 4-Pyridyl-H), 8,78 (d, J = 7 Hz, 2H, 2-Pyridyl-H).

Beispiel 47

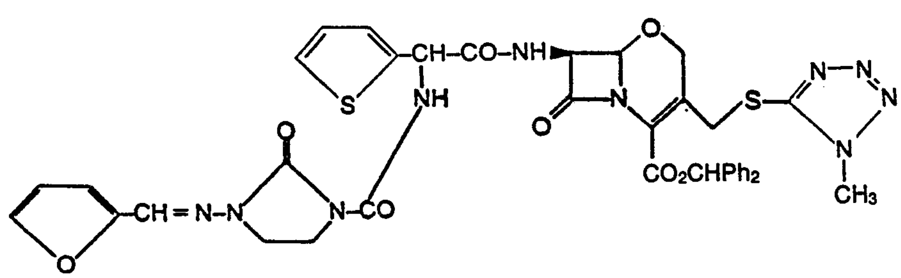

Analog Beispiel 10 wurden aus 924 mg
7-Amino-3-(1-methyl-1-H-tetrazol-5-yl-thiomethyl)-1-oxadethia-3-cephem-4-carbonsäurediphenylmethylester
und 940 mg
2-(2-Oxo-3-furfurylidenamino-imidazolidin-1-yl)-carbonyl-amino-thienylessigsäure
nach 6 h bei 0 °C und Chromatographie (Ethylacetat) 412 mg des entsprechenden Oxacephems als ein weisser harter Schaum erhalten.

$^1$H-NMR (250 MHz, $CDCl_3$) δ 3.91 (s, 3H, N–$CH_3$), 3.9, 4.1 (m, 4H, $CH_2N$), 4.29, 4.38 (AB-Signal, J = 12 Hz, 2H, $CH_2S$), 4.59, 4.85 (AB-Signal, J = 16 Hz, 2H, $CH_2O$), 5.14 (d, J = 4.5 Hz, 1H, H–6), 5.82 (dd, J = 4.5 Hz, J = 8 Hz, H–7), 5.86 (d, J = 9 Hz, NHCHCO),

zusammen 2H, 6.58, 6.88 (m, Furyl-H), 6.97 (s, 1H, $C\underline{H}Ph_2$), 7.0–7.6 (m, 15–H, $C_6H_5$, Furyl-H, Thienyl-H, NH), 7.83 (s, 1H, CH=N), 9.13 (d, J=8 Hz, 1H, $CHN\underline{H}CO$).

Beispiel 48

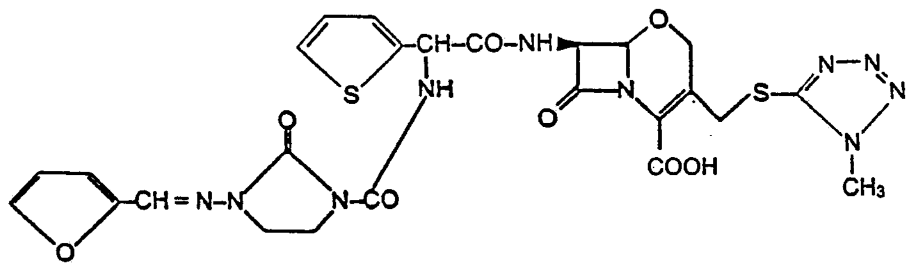

Analog Beispiel 11 wurde die Titelverbindung aus dem entsprechenden Diphenylmethylester (Beispiel 47) als ein Pulver erhalten.

$^1$H-NMR (250 MHz, DMSO) δ 3.7–4.0 (m, 4H, $CH_2N$), 4.0 (s, 3H, $N-CH_3$), 4,22, 4.36 (AB-Signal, J=14 Hz, 2H, $CH_2S$), 4.59, 4.72 (AB-Signal, J=17.5 Hz, 2H, $CH_2O$), 5.20 (d, J=4.5 Hz, 1H, H–6), 5.65 (dd, J=4.5 Hz, J=10 Hz, 1H H–7), 5.98 (d, J=9 Hz, 1H, $NHC\underline{H}CO$), 6.64, 6.86 (m, 2H, Furyl-H), 7.01, 7.13, 7.46 (m, 3H, Thienyl-H), 7.78 (s, CH=N), 7.82 (m, Furyl-H), zusammen 2H, 9.08 (d, J=9 Hz, 1H, $N\underline{H}CHCO$), 9,39 (d, J=10 Hz, 1H, NH).

IR (KBr): ~3400 (b, OH), 1777 (C=O, β-Lactam), 1722 (C=O, Säure), 1680 $cm^{-1}$ (C=O, Amide; C=N).

**Patentansprüche**

1. Verbindungen der Formel

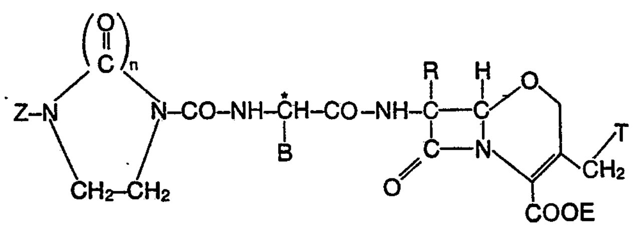

(I)

in welcher
R für Wasserstoff oder Methoxy steht,
n 1 und 2 ist
Z für die Gruppe

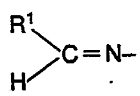

steht,
worin $R^1$ für gegebenenfalls durch einen oder mehrere, gleiche oder verschiedene Reste aus der Gruppe bestehend aus Halogen, Amino, Mono-$C_1$–$C_6$-alkylamino, Di-$C_1$–$C_6$-alkylamino, Pyrrolidyl, Piperidyl, HCO–NH–, $C_1$–$C_6$-Alkyl-CO–NH–, H–CO–N($C_1$–$C_6$-alkyl)–, $C_1$–$C_6$-Alkyl-CO–N($C_1$–$C_6$-alkyl)–, $(C_1–C_6\text{-Alkyl})_2C=N–$, $C_1$–$C_6$-Alkyl-$SO_2$–NH–, HO–$SO_2$–NH–, HO–$SO_2$–N($C_1$–$C_6$-alkyl)–, Amidino, $(C_1–C_6\text{-Alkyl})_2$–N–CH=N–,

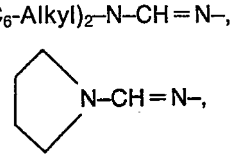

Guanido, Nitro, Azido, Hydroxy, $C_1$–$C_6$-Alkyloxy–, H–CO–O–, $C_1$–$C_6$-Alkyl–CO–O–, $C_1$–$C_6$-Alkyl–O–CO–O–, $H_2N$–CO–O–, $C_1$–$C_6$-Alkyl–NH–CO–O–, $(C_1–C_6\text{-Alkyl})_2N$–CO–O–,

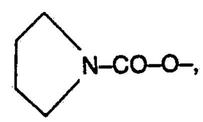

$H_2N$–$SO_2$–O–, $C_1$–$C_6$-Alkyl–NH–$SO_2$–O–, $(C_1–C_6\text{-Alkyl})_2N$–$SO_2$–O–, HOOC–, $H_2N$–CO–, $(C_1–C_6\text{-Alkyl})_2N$–CO–, OHC–, HO–$SO_2$–O–, HS–, $C_1$–$C_6$-Alkyl–S–, $C_1$–$C_6$-Alkyl–S(=O)–, $HO_3S$–, $C_1$–$C_6$-Alkyl–$SO_2$–, $H_2N$–SO–, $C_1$–$C_6$-Alkyl–NH–$SO_2$–, $(C_1–C_6\text{-Alkyl})_2$–N–$SO_2$–, HO–$SO_2$–S–, geradkettiges oder verzweigtes Alkyl mit 1 bis 6 Kohlenstoffatomen, Phenyl und Phenoxy, substituiertes Aryl mit 6 bis 10 Kohlenstoffatomen im Arylteil oder gegebenenfalls durch einen oder mehrere, gleiche oder verschiedene Reste aus der Gruppe bestehend aus $C_1$–$C_6$-Alkyl, Trifluormethyl, Halogen, Amino, $C_1$–$C_6$-Alkylamino, Di–$C_1$–$C_6$-alkylamino, Formylamino, Acetylamino, $CH_3$–O–CO–NH–, $C_2H_5$O–CO–NH–, $CH_3$–$SO_2$–NH–, Hydroxy, Methoxy, Ethoxy, Methylthio, Ethylthio, $CH_3$–$SO_2$–, $CH_3$–SO–, HOOC–, $HO_3S$–, HCO–, $C_1$–$C_6$-Alkyl–CO–, $C_1$–$C_6$-Alkyl–O–CO– und CN am Ringkohlenstoff oder gegebenenfalls durch Reste aus der Gruppe bestehend aus $C_1$–$C_6$-Alkyl, –C≡N, –CHO, –COO– $C_1$–$C_6$-Alkyl, –CO–$NH_2$,

$-CO-NH-C_1-C_6$-Alkyl und $-CO$-alkyl, am Ringstickstoff substituierte heteroparaffinische, heteroaromatische oder heteroolefinische 5- bis 7-gliedrige Ringe mit 1 bis 3 gleichen oder verschiedenen Heteroatomen aus der Gruppe bestehend aus Sauerstoff, Schwefel oder Stickstoff steht, oder wenn n = 1 ist,

Z für Cycloalkyl mit 3 bis 7 Ringkohlenstoffatomen oder für Furyl, Pyryl, 1-Methylpyrryl-2 oder -3, Thienyl, Oxazolyl (gebunden in 2-, 4- oder 5-Stellung), Thiazolyl (gebunden in 2-, 4- oder 5-Stellung), Isoxazolyl (gebunden in 3-, 4- oder 5-Stellung), Isothiazolyl (gebunden in 3-, 4- oder 5-Stellung), 1,2,5-Oxadiazolyl-3-, 1,2,5-Thiadiazolyl-3-, 1,3,4-Oxadiazolyl-2-, 1,3,4-Thiadiazolyl-2-, 1,2,4-Oxadiazolyl (gebunden in 3- oder 5-Stellung), 1,2,4-Thiadiazolyl (gebunden in 3- oder 5-Stellung), Pyrazolyl (gebunden in 3-, 4- oder 5-Stellung), 1,2,4-Triazolyl (gebunden in 3- oder 5-Stellung), Tetrazolyl, Pyridyl (gebunden in 2-, 3- oder 4-Stellung), Pyridazinyl (gebunden in 3- oder 4-Stellung), Pyrimidinyl (gebunden in 2-, 4- oder 5-Stellung), α-Pyronyl (gebunden in 3-, 4-, 5- oder 6-Stellung), γ-Pyronyl (gebunden in 2- oder 3-Stellung) und Benzthiazolyl-2- steht, wobei Z gegebenenfalls durch $C_1-C_6$-Alkyl, $C_1-C_6$-Alkyliden, Vinyl, Propenyl, Allyl, Isopropenyl, $C_1-C_6$-Alkoxymethyl, $C_1-C_6$-Alkylthiomethyl, Trifluormethyl, Hydroxymethyl, Formyl, $C_1-C_6$-Alkanoyl, $C_1-C_6$-Alkanoyloxymethyl, Benzyl, Phenyl, Cyanomethyl, $CH_3-NH-CO-CH_2-$, $(CH_3)_2N-CO-CH_2-$, $C_1-C_6$-Alkoxycarbonyl, Carboxy, Cyano, Hydroxy, $C_1-C_6$-Alkanoyloxy, $C_1-C_6$-Alkoxy, Benzyloxy, Halogen, Mercapto, $C_1-C_6$-Alkylthio, $C_1-C_6$-Alkylsulfinyl, $C_1-C_6$-Alkylsulfonyl, $CH_3-CO-NH-$, $CH_3-CO-N(CH_3)-$, $CH_3-SO_2-NH-$ und $CH_3-SO_2-\underset{CH_3}{\underset{|}{N}}-$ mono-di oder trisubstituiert sein kann,

B für gegebenenfalls durch Halogen, Hydroxy, Alkyl mit 1 bis 6 Kohlenstoffatomen, Cyano und Methylsulfonyl substituiertes Phenyl oder Cyclohexadienyl steht, oder für einen heterocyclischen Rest aus der Gruppe bestehend aus Pyrazolyl, Imidazolyl, Oxazolyl, Oxadiazolyl, 2-Amino- und 2-Oxo-$\Delta^4$-thiazolinyl, Tetrazolyl, Sydnonyl, Furyl, Thienyl-, Pyrrolyl, Thiazolyl-, Isothiazolyl-, Isoxazolyl- und Thiadiazolyl steht, wobei der heterocyclische Rest ein- oder mehrfach durch Halogen, Alkyl mit 1 bis 6 Kohlenstoffatomen, Cyano, Sulfo und Methylsulfonyl substituiert sein kann,

T $C_1-C_4$-Alkyl-CO-O-, Pyridinium, Aminopyridinium, Carbamoylpyridinium, Carbamoyloxy, die Gruppe -S-Phenyl, welche durch Halogen, Amino, $C_1-C_6$-Alkylamino, Di-$C_1-C_6$-alkylamino, $C_1-C_6$-Alkyl, Cycloalkyl mit 3 bis 7 Kohlenstoffatomen, $C_1-C_6$-Alkyloxy, Trifluormethyl, Phenyl, Benzyl und Acylamino mit 2 bis 5 Kohlenstoffatomen substituiert sein kann, oder die Gruppe -S-Het bedeutet, in welcher Het für einen in der β-Lactamchemie üblichen heterocyclischen 5- oder 6-gliedrigen Ring steht,

E für Wasserstoff, eine pharmazeutisch verwendbare Estergruppierung, ein salzbildendes Kation oder eine geeignete Schutzgruppe steht, oder eine negative Ladung bedeutet, wenn in T ein quartärer Stickstoff enthalten ist, wobei die Verbindungen der Formel I bezüglich des Chiralitätszentrums Ċ in den beiden möglichen R- und S-Konfigurationen sowie als Gemische der daraus resultierenden Diastereomeren vorliegen können, und wobei die Verbindungen der Formel I, falls Z für die Gruppe

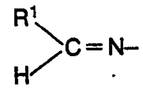

steht, bezüglich der Iminogruppe sowohl in der syn-Form als auch in der anti-Form vorliegen können und wobei die Verbindungen der Formel I auch in den verschiedenen Hydratformen vorliegen können, und die nichttoxischen, pharmazeutisch verträglichen Salze dieser Verbindungen.

2. Verbindungen der Formel I gemäss Anspruch 1, in der

R für Methoxy steht,

Z für die Gruppe

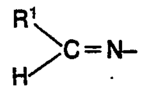

steht, worin

$R^1$ gegebenenfalls durch Halogen, Alkyl mit 1 bis 4 Kohlenstoffatomen, Alkoxy mit 1 bis 4 Kohlenstoffatomen, Nitro, 1 oder 2 Hydroxygruppen, S-Alkyl mit 1 bis 4 Kohlenstoffatomen oder Alkylsulfonyl mit 1 bis 4 Kohlenstoffatomen substituiertes Phenyl bedeutet oder gegebenenfalls durch Halogen, Alkyl oder Alkoxycarbonyl mit 1 bis 4 Kohlenstoffatomen, vorzugsweise in der 4- oder 5-Stellung substituiertes Furyl oder Thienyl bedeutet, wobei der Furyl- und Thienylring bevorzugt in 2- und 3-Stellung gebunden ist, oder Pyridyl bedeutet, oder

Z für Cyclopropyl, Furyl, Pyridyl, Thienyl, Benzthiazolyl-2- oder für 1,3,4-Thiadiazolyl-2-, welches in 5-Stellung durch sec.-Butyl, Trifluormethyl, Methylthio, i-Propylthio oder Methylsulfonyl substituiert sein kann, steht,

B für Phenyl, Hydroxyphenyl oder Cyclohexadienyl oder für Furyl, Thienyl, Pyrrolyl, Imidazolyl, Thiazolyl, Isothiazolyl, Isoxazolyl, Thiadiazolyl steht, und

n 1 oder 2 ist,

T für $-OCOCH_3$, für gegebenenfalls durch Alkyl mit 1 bis 4 Kohlenstoffatomen oder $CF_3$ oder $CH_2COOH$ substituiertes Thiadiazolylthio oder Tetrazolylthio, für Pyridinium, Aminopyridinium, Carbamoylpyridinium oder Carbamoyloxy oder 1,2,5,6-Tetrahydro-2-methyl-5,6-dioxo-as-triazin-3-yl-thio, steht und

Ċ in der D- = R-Konfiguration vorliegt.

3. Verfahren zur Herstellung von β-Lactamantibiotika der Formel I

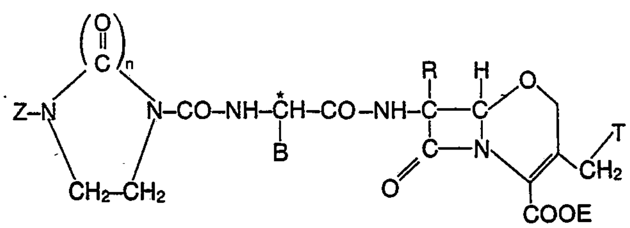

(I)

in welcher

R für Wasserstoff oder Methoxy steht,

n 1 und 2 ist

Z für die Gruppe

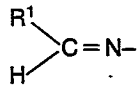

steht,

worin $R^1$ für gegebenenfalls durch einen oder mehrere, gleiche oder verschiedene Reste aus der Gruppe bestehend aus Halogen, Amino, Mono-$C_1$–$C_6$-alkylamino, Di-$C_1$–$C_6$-alkylamino, Pyrrolidyl, Piperidyl, HCO–NH–, $C_1$–$C_6$-Alkyl–CO–NH–, H–CO–N($C_1$–$C_6$-alkyl)–, $C_1$–$C_6$-Alkyl–CO–N($C_1$–$C_6$-alkyl)–, ($C_1$–$C_6$-Alkyl)$_2$C=N–, $C_1$–$C_6$-Alkyl–$SO_2$–NH–, HO–$SO_2$–NH–, HO–$SO_2$–N($C_1$–$C_6$-alkyl)–, Amidino, ($C_1$–$C_6$-Alkyl)$_2$–N–CH=N–,

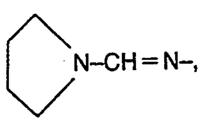

Guanido, Nitro, Azido, Hydroxy, $C_1$–$C_6$-Alkyloxy–, H–CO–O–, $C_1$–$C_6$-Alkyl–CO–O–, $C_1$–$C_6$-Alkyl–O–CO–O–, $H_2N$–CO–O–, $C_1$–$C_6$-Alkyl–NH–CO–O–, ($C_1$–$C_6$-Alkyl)$_2$N–CO–O–,

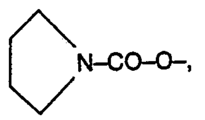

$H_2N$–$SO_2$–O–, $C_1$–$C_6$-Alkyl–NH–$SO_2$–O–, ($C_1$–$C_6$-Alkyl)$_2$N–$SO_2$–O–, HOOC–, $H_2N$–CO–, ($C_1$–$C_6$-Alkyl)$_2$N–CO–, OHC–, HO–$SO_2$–O–, HS–, $C_1$–$C_6$-Alkyl–S–, $C_1$–$C_6$-Alkyl–S–, $C_1$–$C_6$-Alkyl–S(=O)–, $HO_3S$–, $C_1$–$C_6$-Alkyl–$SO_2$–, $H_2N$–$SO_2$–, $C_1$–$C_6$-Alkyl–NH–$SO_2$–, ($C_1$-$C_6$-Alkyl)$_2$–N–$SO_2$–, HO–$SO_2$–S–, geradkettiges oder verzweigtes Alkyl mit 1 bis 6 Kohlenstoffatomen, Phenyl und Phenoxy, substituiertes Aryl mit 6 bis 10 Kohlenstoffatomen im Arylteil oder gegebenenfalls durch einen oder mehrere, gleiche oder verschiedene Reste aus der Gruppe bestehend aus $C_1$–$C_6$-Alkyl, Trifluormethyl, Halogen, Amino, $C_1$–$C_6$-Alkylamino, Di–$C_1$–$C_6$-alkylamino, Formylamino, Acetylamino, $CH_3$–O–CO–NH–, $C_2H_5O$–CO–NH–, $CH_3$–$SO_2$–NH–, Hydroxy, Methoxy, Ethoxy, Methylthio, Ethylthio, $CH_3$–$SO_2$–, $CH_3$–SO–, HOOC–, $HO_3S$–, HCO–, $C_1$–$C_6$-Alkyl–CO–, $C_1$–$C_6$-Alkyl–O–CO– und CN am Ringkohlenstoff oder gegebenenfalls durch Reste aus der Gruppe bestehend aus $C_1$–$C_6$-Alkyl, –C≡N, –CHO, –COO–$C_1$–$C_6$-alkyl, –CO–$NH_2$, –CO–NH–$C_1$–$C_6$-alkyl und –CO-alkyl, am Ringstickstoff substituierte heteroparaffinische, heteroaromatische oder heteroolefinische 5- bis 7-gliedrige Ringe mit 1 bis 3 gleichen oder verschiedenen Heteroatomen aus der Gruppe bestehend aus Sauerstoff, Schwefel oder Stickstoff steht,

oder wenn n = 1 ist,

Z für Cycloalkyl mit 3 bis 7 Ringkohlenstoffatomen oder für Furyl- Pyryl, 1-Methylpyrryl-2 oder -3, Thienyl, Oxazolyl (gebunden in 2-, 4- oder 5-Stellung), Thiazolyl (gebunden in 2-, 4- oder 5-Stellung), Isoxazolyl (gebunden in 3-, 4- oder 5-Stellung), Isothiazolyl (gebunden in 3-, 4- oder 5-Stellung), 1,2,5-Oxadiazolyl-3-, 1,2,5-Thiadiazolyl-3-, 1,3,4-Oxadiazolyl-2-, 1,3,4-Thiadiazolyl-2-, 1,2,4-Oxadiazolyl (gebunden in 3- oder 5-Stellung), 1,2,4-Thiadiazolyl (gebunden in 3- oder 5-Stellung), Pyrazolyl (gebunden in 3-, 4- oder 5-Stellung), 1,2,4-Triazolyl (gebunden in 3- oder 5-Stellung), Tetrazolyl, Pyridyl (gebunden in 2-, 3- oder 4-Stellung), Pyridazinyl (gebunden in 3- oder 4-Stellung), Pyrimidinyl (gebunden in 2-, 4- oder 5-Stellung), α-Pyronyl (gebunden in 3-, 4-, 5- oder 6-Stellung), γ-Pyronyl (gebunden in 2- oder 3-Stellung) und Benzthiazolyl-2-, steht, wobei Z gegebenenfalls durch $C_1$–$C_6$-Alkyl, $C_1$–$C_6$-Alkyliden, Vinyl, Propenyl, Allyl, Isopropenyl, $C_1$–$C_6$-Alkoxymethyl, $C_1$–$C_6$-Alkylthiomethyl, Trifluormethyl, Hydroxymethyl, Formyl, $C_1$–$C_6$-Alkanoyl, $C_1$–$C_6$-Alkanoyloxymethyl, Benzyl, Phenyl, Cyanomethyl, $CH_3$–NH–CO–$CH_2$–, $(CH_3)_2N$–CO–$CH_2$–, $C_1$–$C_6$-Alkoxycarbonyl, Carboxy, Cyano, Hydroxy, $C_1$–$C_6$-Alkanoyloxy, $C_1$–$C_6$-Alkoxy, Benzyloxy, Halogen, Mercapto, $C_1$–$C_6$-Alkylthio, $C_1$–$C_6$-Alkylsulfinyl, $C_1$–$C_6$-Alkylsulfonyl, $CH_3$–CO–NH–, $CH_3$–CO–N($CH_3$)–, $CH_3$–$SO_2$–NH– und $CH_3$–$SO_2$–N($CH_3$)– mono-di- oder trisubstituiert sein kann,

B für gegebenenfalls durch Halogen, Hydroxy, Alkyl mit 1 bis 6 Kohlenstoffatomen, Cyano und Methylsulfonyl substituiertes Phenyl oder Cyclohexadienyl steht, oder für einen heterocyclischen Rest aus der Gruppe bestehend aus Pyrazolyl, Imidazolyl, Oxazolyl, Oxadiazolyl, 2-Amino- und 2-Oxo-$\Delta^4$-thiazolinyl, Tetrazolyl, Sydnonyl sowie Furyl, Thienyl-, Pyrrolyl, Thiazolyl-, Isothiazolyl-, Isoxazolyl- und Thiadiazolyl steht, wobei der heterocyclische Rest ein- oder mehrfach durch Halogen, Alkyl mit 1 bis 6 Kohlenstoffatomen, Cyano, Sulfo und Methylsulfonyl substituiert sein kann,

T $C_1$–$C_4$-Alkyl–CO–O–, Pyridinium, Aminopyridinium, Carbamoylpyridinium, Carbamoyloxy, die Gruppe –S-Phenyl, welche durch Halogen, Amino, $C_1$–$C_6$-Alkylamino, Di–$C_1$–$C_6$-alkylamino, $C_1$–$C_6$-Alkyl, Cycloalkyl mit 3 bis 7 Kohlenstoffatomen, $C_1$–$C_6$-Alkyloxy, Trifluormethyl, Phenyl, Benzyl und Acylamino mit 2 bis 5 Kohlenstoffatomen substituiert sein kann, oder die Gruppe –S-Het bedeutet, in welcher Het für einen in der β-Lactamchemie üblichen heterocyclischen 5- oder 6-gliedrigen Ring steht,

E für Wasserstoff, eine pharmazeutisch verwendbare Estergruppierung, ein salzbildendes Kation oder eine geeignete Schutzgruppe steht, oder eine negative Ladung bedeutet, wenn in T ein quartärer Stickstoff enthalten ist, wobei die Verbindungen der Formel I bezüglich des Chiralitätszentrums Ċ in den beiden möglichen R- und S-Konfigurationen sowie als Gemische der daraus resultierenden Diastereomeren vorliegen können, und wobei die Verbindungen der Formel I, falls Z für die Gruppe

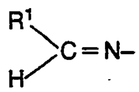

steht, bezüglich der Iminogruppe sowohl in der syn-Form als auch in der anti-Form vorliegen können und wobei die Verbindungen der Formel I auch in den verschiedenen Hydratformen vorliegen können, und die nichttoxischen, pharmazeutisch verträglichen Salze dieser Verbindungen, dadurch gekennzeichnet, dass man

a) Verbindungen der Formel II

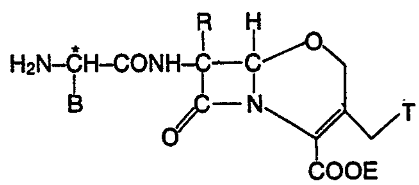

(II)

in welcher R, B, Ċ, T und E die oben angegebene Bedeutung haben,

mit Verbindungen der Formel III

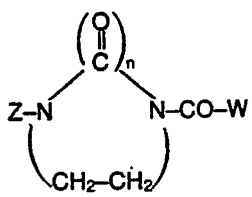

(III)

in welcher

Z und n die oben angegebene Bedeutung haben und

W für Halogen, Azid oder eine andere nukleofuge Abgangsgruppe steht,

in Gegenwart eines Lösungsmittels und gegebenenfalls eines Säurebindemittels bei Temperaturen von etwa −70 °C bis etwa +50 °C umsetzt und die erhaltenen β-Lactamantibiotica gegebenenfalls in ihre nichttoxischen, pharmazeutisch verträglichen Salze überführt oder aus den erhaltenen Salzen die freien Säuren herstellt,

oder dass man

b) Verbindungen der Formel IV

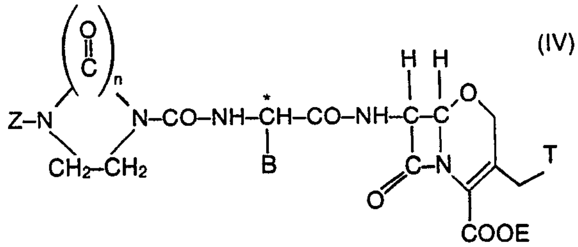

(IV)

in welcher Z, n, Ċ, B und T die oben angegebene Bedeutung haben, R für Wasserstoff steht und E eine leicht entfernbare esterbildende Gruppe, eine Acetoxymethylgruppe, den kationischen Rest einer Base oder Wasserstoff bedeutet, mit 1–10 Äquivalenten pro Äquivalent β-Lactam-Antibiotikum der Formel (IV) einer Base in Anwesenheit eines Überschusses von Methanol in einem inerten organischen Lösungsmittel unter Zusatz eines N-Halogenierungsmittels umsetzt und die Verbindung der Formel I gegebenenfalls nach vorheriger Abspaltung der Schutzgruppe, Überführung in ein Salz oder einen pharmazeutisch verwendbaren Ester isoliert, oder dass man

c) eine Verbindung der Formel V

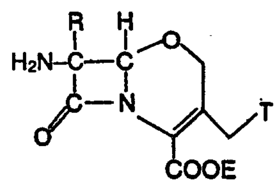

(V)

in der R, T und E die oben angegebene Bedeutung haben, mit einem Acylierungsmittel der Formel VI

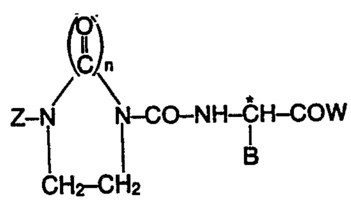

(VI)

in der Z, n, Ċ und B die oben angegebene Bedeutung haben und W eine reaktive funktionelle Gruppe ist, acyliert, oder dass man

d) eine Verbindung der Formel VIII

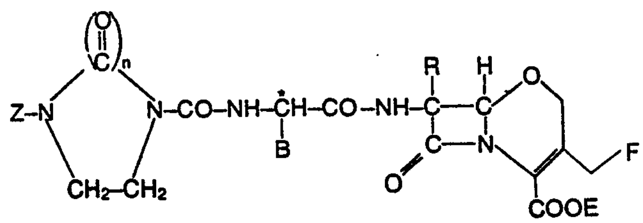

(VIII)

in der Z, n, Ċ, B, R und E die oben angegebene Bedeutung haben und F eine abspaltbare Gruppe darstellt, mit einer geeigneten Substanz der allgemeinen Formel

$R^2H$ (IX)

oder mit einem tertiären Amin so umsetzt, dass die eingangs genannten Verbindungen der Formel (I) entstehen.

4. Arzneimittel, gekennzeichnet durch einen Gehalt an mindestens einem β-Lactam-Antibiotikum gemäss den Ansprüchen 1 oder 2.

5. Verwendung von Verbindungen der Formel I gemäss Anspruch 1 zur Herstellung von Arzneimitteln.

6. Verbindungen der Formeln

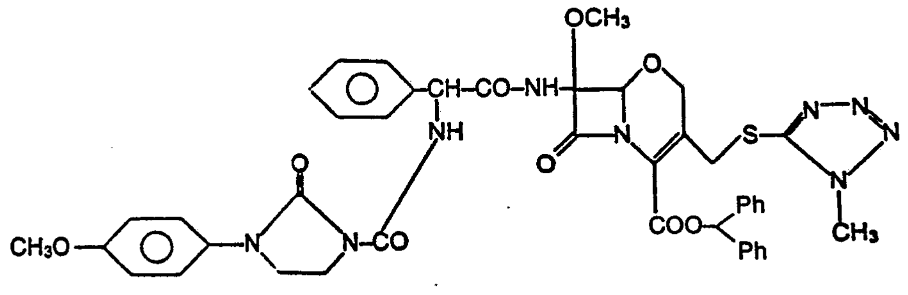

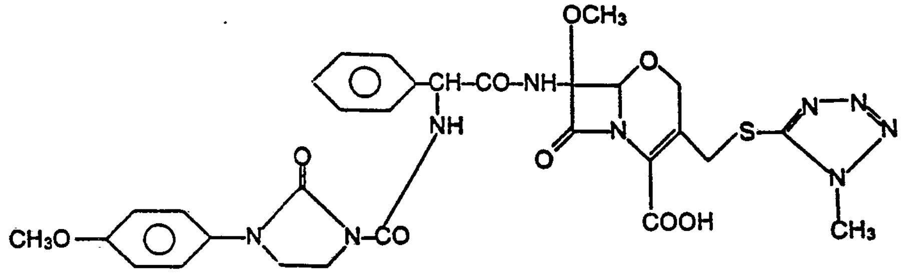

## Claims

1. Compounds of the formula

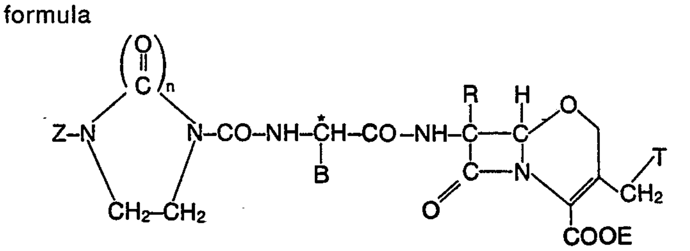

(I)

in which

R represents hydrogen or methoxy,

n is 1 or 2

Z is the group

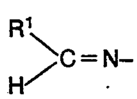

wherein

$R^1$ represents aryl which has 6 to 10 carbon atoms in the aryl part and is optionally substituted by one or more identical or different radicals from the group consisting of halogen, amino, mono-$C_1$–$C_6$-alkylamino, di-$C_1$–$C_6$-alkylamino, pyrrolidyl, piperidyl, HCO–NH–, $C_1$–$C_6$-alkyl–CO–NH–, H–CO–N($C_1$–$C_6$-alkyl)–, $C_1$–$C_6$-alkyl–CO–N($C_1$–$C_6$-alkyl)–, ($C_1$–$C_6$-alkyl)$_2$C=N–, $C_1$–$C_6$-alkyl-$SO_2$–NH–, HO–$SO_2$–NH–, HO–$SO_2$–N($C_1$–$C_6$-alkyl)–, amidino, ($C_1$–$C_6$-alkyl)$_2$–N–CH=N–,

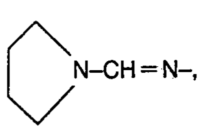

guanido, nitro, azido, hydroxy, $C_1$–$C_6$-alkyloxy–, H–CO–O–, $C_1$–$C_6$-alkyl–CO–O–, $C_1$–$C_6$-alkyl–O–CO–O–, $H_2N$–CO–O–, $C_1$–$C_6$-alkyl–NH–CO–O–, ($C_1$–$C_6$-alkyl)$_2$N–CO–O–,

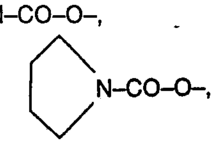

$H_2N$–$SO_2$–O–, $C_1$–$C_6$-alkyl–NH–$SO_2$–O–, ($C_1$–$C_6$-alkyl)$_2$N–$SO_2$–O–, HOOC–, $H_2N$–CO–, ($C_1$–$C_6$-alkyl)$_2$N–CO–, OHC–, HO–$SO_2$–O–, HS–, $C_1$–$C_6$ alkyl–S–, $C_1$–$C_6$-alkyl–$\underset{O}{\underset{\|}{S}}$–, $HO_3S$–, $C_1$–$C_6$-alkyl–$SO_2$–, $H_2N$–$SO_2$–, $C_1$–$C_6$-alkyl–NH–$SO_2$–, ($C_1$–$C_6$-alkyl)$_2$–N–$SO_2$–, HO–$SO_2$–S–, straight-chain or branched alkyl having 1 to 6 carbon atoms, phenyl, and phenoxy, or heteroparaffinic, heteroaromatic or heteroolefinic 5-membered to 7-membered rings which have 1 to 3 identical or different hereto atoms from the group consisting of oxygen, sulphur, and nitrogen and are optionally substituted at the ring carbon atoms by one or more identical or different radicals from the group consisting of $C_1$–$C_6$-alkyl, trifluoromethyl, halogen, amino,

$C_1$–$C_6$-alkylamino, di–$C_1$–$C_6$-alkylamino, formylamino, acetylamino, $CH_3$–O–CO–NH–, $C_2H_5O$–CO–NH–, $CH_3$–$SO_2$–NH–, hydroxy, methoxy, ethoxy, methylthio, ethylthio, $CH_3$–$SO_2$–, $CH_3$–SO–, HOOC–, $HO_3S$–, HCO–, $C_1$–$C_6$-alkyl–CO–, $C_1$–$C_6$-alkyl–O–CO– and CN, or optionally substituted at the ring nitrogen atoms by radicals from the group consisting of $C_1$–$C_6$-alkyl, $-C{\equiv}N$, –CHO, –COO–$C_1$–$C_6$-alkyl, –CO–$NH_2$, –CO–NH–$C_1$–$C_6$-alkyl and –CO–alkyl,
or, when n is 1

Z represents cycloalkyl having 3 to 7 ring carbon atoms or furyl, pyryl, 1-methylpyrr-2-yl or -3-yl, thienyl, oxazolyl (bonded in the 2-, 4- or 5-position), thiazolyl (bonded in the 2-, 4- or 5-position), isoxazolyl (bonded in the 3-, 4- or 5-position), isothiazolyl (bonded in the 3-, 4- or 5-position), 1,2,5-oxadiazol-3-yl, 1,2,5-thiadiazol-3-yl, 1,3,4-oxadiazol-2-yl, 1,3,4-thiadiazol-2-yl, 1,2,4-oxadiazolyl (bonded in the 3- or 5-position), 1,2,4-thiadiazolyl (bonded in the 3- or 5-position), pyrazolyl (bonded in the 3-, 4- or 5-position), 1,2,4-triazolyl (bonded in the 3- or 5-position), tetrazolyl, pyridyl (bonded in the 2-, 3- or 4-position), pyridazinyl (bonded in the 3- or 4 position), pyrimidinyl (bonded in the 2-, 4- or 5-position), α-pyronyl (bonded in the 3-, 4-, 5- or 6-position), γ-pyronyl (bonded in the 2- or 3-position) and benzothiazol-2-yl, and Z may be optionally mono- di- or trisubstituted by $C_1$–$C_6$-alkyl, $C_1$–$C_6$-alkylidene, vinyl, propenyl, allyl, isopropenyl, $C_1$–$C_6$-alkoxymethyl, $C_1$–$C_6$-alkylthiomethyl, trifluoromethyl, hydroxymethyl, formyl, $C_1$–$C_6$-alkanoyl, $C_1$–$C_6$-alkanoyloxymethyl, benzyl, phenyl, cyanomethyl, $CH_3$–NH–CO–$CH_2$–$(CH_3)_2$N–CO–$CH_2$–, $C_1$–$C_6$-alkoxycarbonyl, carboxyl cyano, hydroxyl, $C_1$–$C_6$-alkanoyloxy, $C_1$–$C_6$-alkoxy, benzyloxy, halogen, mercapto, $C_1$–$C_6$-alkylthio, $C_1$–$C_6$-alkylsulphinyl, $C_1$–$C_6$-alkylsulphonyl, $CH_3$–CO–NH–, $CH_3$–CO–N($CH_3$)–, $CH_3$–$SO_2$–NH– and $CH_3$–$SO_2$–N–
|
$CH_3$,

B represents cyclohexadienyl or phenyl which is optionally substituted by halogen, hydroxyl, alkyl having 1 to 6 carbon atoms, cyano or methylsulphonyl, or represents a heterocyclic radical from the group consisting of pyrazolyl, imidazolyl, oxazolyl, oxadiazolyl, 2-amino- and 2-oxo-$\Delta^4$-thiazolinyl, tetrazolyl, sydnonyl, furyl, thienyl, pyrrolyl, thiazolyl, isothiazolyl, isoxazolyl and thiadiazolyl, and the heterocyclic radical may be monosubstituted or polysubstituted by halogen, alkyl having 1 to 6 carbon atoms, cyano, sulfo or methylsulphonyl,

T denotes $C_1$–$C_4$-alkyl–CO–O–, pyridinium, aminopyridinium, carbamoylpyridinium, carbamoyloxy, the group –S-phenyl, which may be substituted by halogen, amino, $C_1$–$C_6$-alkylamino, di–$C_1$–$C_6$-alkylamino, $C_1$–$C_6$-alkyl, cycloalkyl having 3 to 7 carbon atoms, $C_1$–$C_6$-alkyloxy, trifluoromethyl, phenyl, benzyl or acylamino having 2 to 5 carbon atoms, or the group –S-Het, in which Het represents a heterocyclic 5- or 6-membered ring customary in β-lactam chemistry,

E represents hydrogen, a pharmaceutically usable ester group, a salt-forming cation or a suitable protective group, or denotes a negative charge when a quaternary nitrogen is present in T,

and, with regard to the chirality centre Ċ, the compounds of the formula I may be present in the two possible R- and S-configurations and as mixtures of the resulting diastereomers, and, if Z represents the group

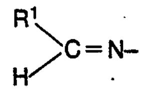

the compounds of the formula I, in respect of the imino group, may be present both in the syn form and in the anti form, and the compounds of the formula I may also be present in the various hydrated forms,
and the nontoxic, pharmaceutically tolerated salts of these compounds.

2. Compounds of the formula I according to Claim 1, in which
R represents methoxy,
Z represents the group

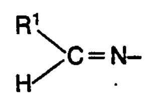

wherein
$R^1$ denotes phenyl which is optionally substituted by halogen, alkyl having 1 to 4 carbon atoms, alkoxy having 1 to 4 carbon atoms, nitro, 1 or 2 hydroxyl groups, S-alkyl having 1 to 4 carbon atoms or alkylsulphonyl having 1 to 4 carbon atoms, or denotes thienyl or furyl which is optionally substituted, preferably in the 4- or 5-position, by halogen, alkyl or alkoxycarbonyl having 1 to 4 carbon atoms, the furyl and thienyl ring preferably being bonded in the 2- and 3-position, or denotes pyridyl, or

Z represents cyclopropyl, furyl, pyridyl, thienyl, benzothiazol-2-yl or 1,3,4-thiadiazol-2-yl which may be substituted in the 5-position by sec-butyl, trifluoromethyl, methylthio, isopropylthio or methylsulphonyl,

B represents phenyl, hydroxyphenyl or cyclohexadienyl or represents furyl, thienyl, pyrrolyl, imidazolyl, thiazolyl, isothiazolyl, isoxazolyl, or thiadiazolyl, and

n is 1 or 2,

T represents $-OCOCH_3$, tetrazolylthio or thiadiazolylthio which is optionally substituted by alkyl having 1 to 4 carbon atoms or $CF_3$ or $CH_2COOH$, or represents pyridinium, aminopyridinium, carbamoylpyridinium or carbamoyloxy or
1,2,5,6-tetrahydro-2-methyl-5,6-dioxo-as-triazin-3-yl-thio,
and

Ċ is present in the D- = R-configuration.

3. Process for the preparation of β-lactam antibiotics of the formula I

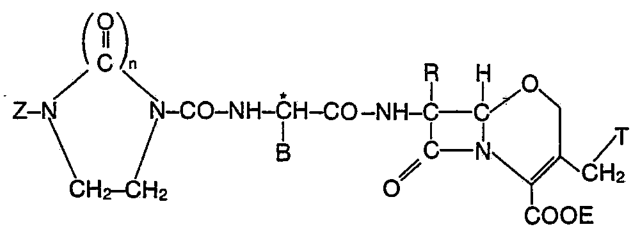

(I)

in which

R represents hydrogen or methoxy

n is 1 or 2

Z is the group

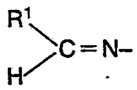

wherein

$R^1$ represents aryl which has 6 to 10 carbon atoms in the aryl part and is optionally substituted by one or more identical or different radicals from the group consisting of halogen, amino, mono-$C_1$–$C_6$-alkylamino, di–$C_1$–$C_6$-alkylamino, pyrrolidyl, piperidyl, HCO–NH–, $C_1$–$C_6$-alkyl–CO–NH–, H–CO–N($C_1$–$C_6$-alkyl)–, $C_1$–$C_6$-alkyl–CO–N($C_1$–$C_6$-alkyl)–, ($C_1$–$C_6$-alkyl)$_2$C=N–, $C_1$–$C_6$-alkyl–$SO_2$–NH–, HO–$SO_2$–NH–, HO–$SO_2$–N($C_1$–$C_6$-alkyl)–, amidino, ($C_1$–$C_6$-alkyl)$_2$–N–CH=N–,

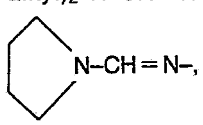

guanido, nitro, azido, hydroxy, $C_1$–$C_6$-alkyloxy–, H–CO–O–, $C_1$–$C_6$-alkyl–CO–O–, $C_1$–$C_6$-alkyl–O–CO–O–, $H_2N$–CO–O–, $C_1$–$C_6$-alkyl–NH–CO–O–, ($C_1$–$C_6$-alkyl)$_2$N–CO–O–,

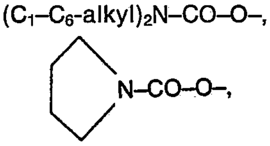

$H_2N$–$SO_2$–O–, $C_1$–$C_6$-alkyl–NH–$SO_2$–O–, ($C_1$–$C_6$-alkyl)$_2$N–$SO_2$–O–, HOOC–, $H_2N$–CO–, ($C_1$–$C_6$-alkyl)$_2$N–CO–, OHC–, HO–$SO_2$–O–, HS–, $C_1$–$C_6$ alkyl–S–, $C_1$–$C_6$-alkyl–S(=O)–, $HO_3S$–, $C_1$–$C_6$-alkyl–$SO_2$–, $H_2N$–$SO_2$–, $C_1$–$C_6$-alkyl–NH–$SO_2$–, ($C_1$–$C_6$alkyl)$_2$–N–$SO_2$–, HO–$SO_2$–S–, straight-chain or branched alkyl having 1 to 6 carbon atoms, phenyl, and phenoxy, or heteroparaffinic, heteroaromatic or heteroolefinic 5-membered to 7-membered rings which have 1 to 3 identical or different hetero atoms from the group consisting of oxygen, sulphur, and nitrogen and are optionally substituted at the ring carbon atoms by one or more identical or different radicals from the group consisting of $C_1$–$C_6$-alkyl, trifluoromethyl, halogen, amino, $C_1$–$C_6$-alkylamino, di–$C_1$–$C_6$-alkylamino, formylamino, acetylamino, $CH_3$–O–CO–NH–, $C_2H_5O$–CO–NH–, $CH_3$–$SO_2$–NH–, hydroxy, methoxy, ethoxy, methylthio, ethylthio, $CH_3$–$SO_2$–, $CH_3$–SO–, HOOC–, $HO_3S$–, HCO–, $C_1$–$C_6$-alkyl–CO–, $C_1$–$C_6$-alkyl–O–CO– and CN, or optionally substituted at the ring nitrogen atoms by radicals from the group consisting of $C_1$–$C_6$-alkyl, –C≡N, –CHO, –COO–$C_1$–$C_6$-alkyl, –CO–$NH_2$, –CO–NH–$C_1$–$C_6$-alkyl and –CO-alkyl,

or, when n is 1

Z represents cycloalkyl having 3 to 7 ring carbon atoms or furyl, pyryl, 1-methylpyrr-2-yl or -3-yl, thienyl, oxazolyl (bonded in the 2-, 4- or 5-position), thiazolyl (bonded in the 2-, 4- or 5-position), isoxazolyl (bonded in the 3-, 4- or 5-position), isothiazolyl (bonded in the 3-, 4- or 5-position), 1,2,5-oxadiazol-3-yl, 1,2,5-thiadiazol-3-yl, 1,3,4-oxadiazol-2-yl, 1,3,4-thiadiazol-2-yl, 1,2,4-oxadiazolyl (bonded in the 3- or 5-position), 1,2,4-thiadiazolyl (bonded in the 3- or 5-position), pyrazolyl (bonded in the 3-, 4- or 5-position), 1,2,4-triazolyl (bonded in the 3- or 5-position), tetrazolyl, pyridyl (bonded in the 2-, 3- or 4-position), pyridazinyl (bonded in the 3- or 4-position), pyrimidinyl (bonded in the 2-, 4- or 5-position), α-pyronyl (bonded in the 3-, 4-, 5- or 6-position), γ-pyronyl (bonded in the 2- or 3-position) and benzothiazol-2-yl, and Z may be optionally mono- di- or trisubstituted by $C_1$–$C_6$-alkyl, $C_1$–$C_6$-alkylidene, vinyl, propenyl, allyl, isopropenyl, $C_1$–$C_6$-alkoxymethyl, $C_1$–$C_6$-alkylthiomethyl, trifluoromethyl, hydroxymethyl, formyl, $C_1$–$C_6$-alkanoyl, $C_1$–$C_6$-alkanoyloxymethyl, benzyl, phenyl, cyanomethyl, $CH_3$–NH–CO–$CH_2$–($CH_3$)$_2$N–CO–$CH_2$–, $C_1$–$C_6$-alkoxycarbonyl, carboxyl cyano, hydroxyl, $C_1$–$C_6$-alkanoyloxy, $C_1$–$C_6$-alkoxy, benzyloxy, halogen, mercapto, $C_1$–$C_6$-alkylthio, $C_1$–$C_6$-alkylsulphinyl, $C_1$–$C_6$-alkylsulphonyl, $CH_3$–CO–NH–, $CH_3$–CO–N($CH_3$)–, $CH_3$–$SO_2$–NH– and $CH_3$–$SO_2$–N($CH_3$)–,

B represents cyclohexadienyl or phenyl which is optionally substituted by halogen, hydroxyl, alkyl having 1 to 6 carbon atoms, cyano or methylsulphonyl, or represents a heterocyclic radical from the group consisting of pyrazolyl, imidazolyl, oxazolyl, oxadiazolyl, 2-amino- and 2-oxo-$\Delta^4$-thiazolinyl, tetrazolyl, sydnonyl, furyl, thienyl, pyrrolyl, thiazolyl, isothiazolyl, isoxazolyl and thiadiazolyl, and the heterocyclic radical may be monosubstituted or polysubstituted by halogen, alkyl having 1 to 6 carbon atoms, cyano, sulfo or methylsulphonyl,

T denotes $C_1$–$C_4$-alkyl–CO–O–, pyridinium, aminopyridinium, carbamoylpyridinium, carbamoyloxy, the group –S-phenyl, which may be substituted by halogen, amino, $C_1$–$C_6$-alkylamino, di–$C_1$–$C_6$-alkylamino, $C_1$–$C_6$-alkyl, cycloalkyl having 3 to 7 carbon atoms, $C_1$–$C_6$-alkyloxy, trifluoromethyl, phenyl, benzyl or acylamino having 2 to 5 carbon atoms, or the group –S-Het, in which Het represents a heterocyclic 5- or 6-membered ring customary in β-lactam chemistry,

E represents hydrogen, a pharmaceutically usable ester group, a salt-forming cation or a suitable protective group, or denotes a negative charge when a quaternary nitrogen is present in T,

and, with regard to the chirality centre Ċ, the compounds of the formula I may be present in the two possible R- and S-configurations and as mixtures of the resulting diastereomers, and, if Z represents the group

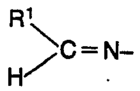

the compounds of the formula I, in respect of the imino group, may be present both in the syn form and in the anti form, and the compounds of the formula I may also be present in the various hydrated forms, and the nontoxic, pharmaceutically tolerated salts of these compounds, characterized in that

a) compounds of the formula II

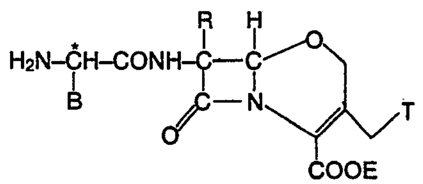

(II)

in which

R, B, Ċ, T and E have the meaning given above, are reacted with compounds of the formula III

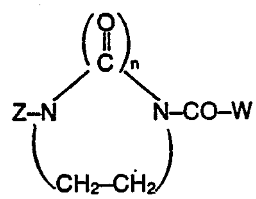

(III)

in which

Z and n have the meaning given above and
W represents halogen, azide or another nucleofugic leaving group,

in the presence of a solvent and, if appropriate, of an acid-binding agent at temperatures of about −70 °C to about +50 °C, and the β-lactam antibiotics obtained are, if appropriate, converted to their nontoxic, pharmaceutically tolerated salts, or the free acids are prepared from the salts obtained, or

b) compounds of the formula IV

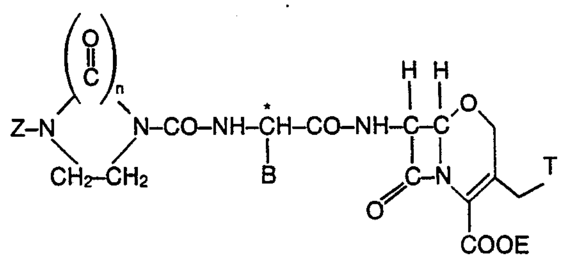

(IV)

in which

Z, n, Ċ, B and T have the meaning given above,
R represents hydrogen and
E denotes a readily removable ester-forming group, an acetoxymethyl group, the cationic radical of a base or hydrogen,
are reacted with 1–10 equivalents, per equivalent of β-lactam antibiotic of the formula (IV), of a base in the presence of an excess of methanol in an inert organic solvent, with the addition of an N-halogenating agent, and, if necessary after elimination of the protective group beforehand or conversion to a salt or a pharmaceutically usable ester, the compound of the formula I is isolated or

c) a compound of the formula V

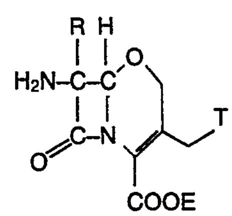

(V)

in which
R, T and E have the meaning given above, is acylated with an acylating agent of the formula VI

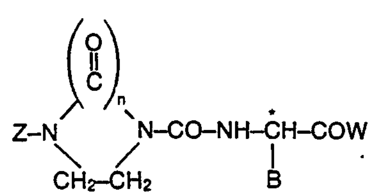

(VI)

in which

Z, n, Ċ and B have the meaning given above and

W is a reactive functional group, or

d) a compound of the formula VIII

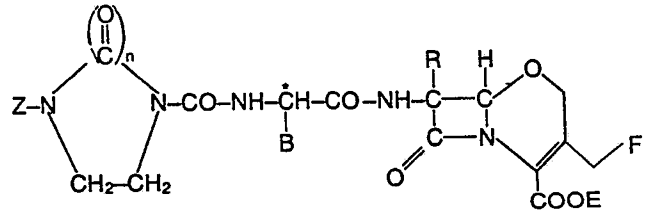

(VIII)

in which

Z, n, Ċ, B, R and E have the meaning given above and

F represents a group which can be eliminated, is reacted with a suitable substance of the general formula

$R^2H$ (IX)

or with a tertiary amine in such a way that the compounds stated at the outset, of the formula (I), are formed.

4. Medicaments characterized in that they contain at least one β-lactam antibiotic according to Claims 1 or 2.

5. Use of compounds of the formula I according to Claim 1 for the preparation of medicaments.

6. Compounds of the formulae

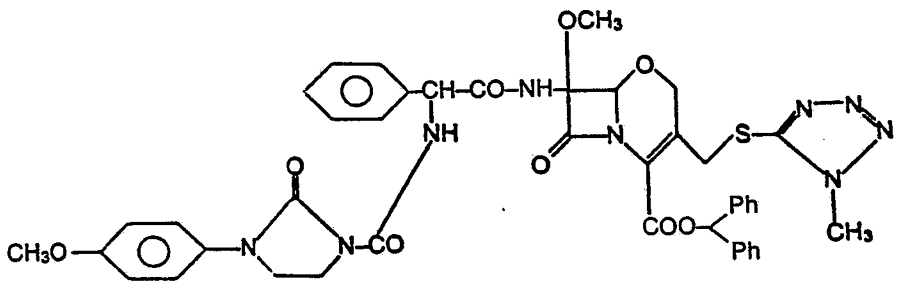

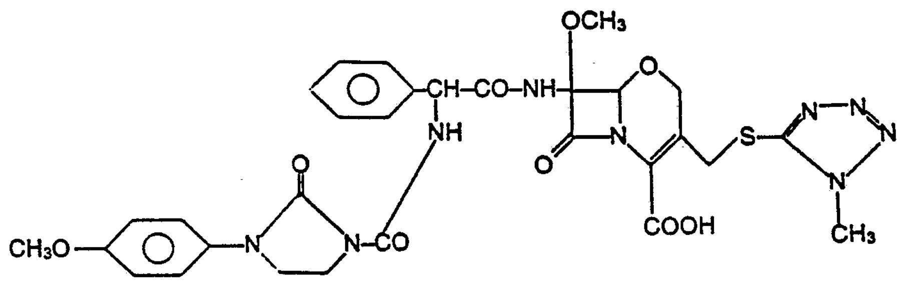

**Revendications**

1. Composés de formule

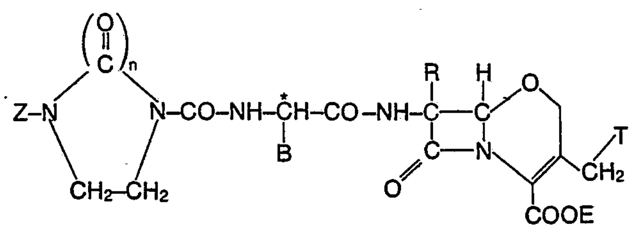

(I)

dans laquelle

R est l'hydrogène ou le groupe méthoxy,

n a les valeurs 1 et 2,

Z représente le groupe 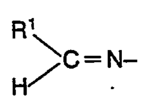

dans lequel $R^1$ représente un groupe aryle portant éventuellement un ou plusieurs substituants identiques ou différents du groupe comprenant les restes halogéno, amino, mono-alkylamino en $C_1$ à $C_6$, di(alkyle en $C_1$ à $C_6$)amino, pyrrolidyle, pipéridyle, HCO–NH–, (alkyle en $C_1$ à $C_6$)–CO–NH–, H–CO–N(alkyle en $C_1$ à $C_6$)–, (alkyle en $C_1$ à $C_6$)–CO–N(alkyle en $C_1$ à $C_6$)–, (alkyle en $C_1$ à $C_6$)$_2$C=N–, (alkyle en $C_1$ à $C_6$)–$SO_2$–NH–, HO–$SO_2$–NH–, HO–$SO_2$–N(alkyle en $C_1$ à $C_6$)–, amidino, (alkyle en $C_1$ à $C_6$)$_2$–N–CH=N–,

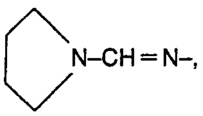

guanido, nitro, azido, hydroxy, alkyloxy en $C_1$ à $C_6$, H–CO–O–, (alkyle en $C_1$ à $C_6$)–CO–O–, (alkyle en $C_1$ à $C_6$)–O–CO–O–, $H_2N$–CO–O–, (alkyle en $C_1$ à $C_6$)–NH–CO–O–, (alkyle en $C_1$ à $C_6$)$_2$N–CO–O–,

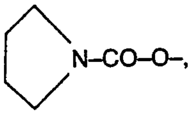

$H_2N$–$SO_2$–O–, (alkyle en $C_1$ à $C_6$)–NH–$SO_2$O–, (alkyle en $C_1$ à $C_6$)$_2$N–$SO_2$–O–, HOOC–, $H_2N$–CO–, (alkyle en $C_1$ à $C_6$)$_2$N–CO–, OHC–, HO–$SO_2$–O–, HS–, (alkyle en $C_1$ à $C_6$)–S–, (alkyle en $C_1$ à $C_6$)–S(O)–, $HO_3S$–, (alkyle en $C_1$ à $C_6$)–$SO_2$–, $H_2N$–$SO_2$–, (alkyle en $C_1$ à $C_6$)–NH–$SO_2$–, (alkyle en $C_1$ à $C_6$)$_2$–N–$SO_2$–, HO–$SO_2$–S–, alkyle à chaîne droite ou à chaîne ramifiée ayant 1 à 6 atomes de carbone, phényle ou phénoxy, avec 6 à 10 atomes de carbone dans la partie aryle, ou des noyaux pentagonaux à heptagonaux hétéro-paraffiniques, hétéro-aromatiques ou hétéro-oléfiniques éventuellement substitués par un ou plusieurs restes identiques ou différents dans le groupe comprenant les restes alkyle en $C_1$ à $C_6$, trifluorométhyle, halogéno, amino, alkylamino en $C_1$ à $C_6$, di(alkyle en $C_1$ à $C_6$)amino, formylamino, acétylamino, $CH_3$–O–CO–NH–, –$C_2H_5$O–CO–NH–, $CH_3$–$SO_2$–NH–, hydroxy, méthoxy, éthoxy, méthylthio, éthylthio, $CH_3$–$SO_2$–, $CH_3$–SO–, HOOC–, $HO_3$–S–, HCO–, (alkyle en $C_1$ à $C_6$)–CO–, (alkyle en $C_1$ à $C_6$)–O–CO– et CN sur le carbone du noyau ou le cas échéant substitués par des restes du groupe comprenant les restes alkyle en $C_1$ à $C_6$, –C≡N, –CHO, –COO–(alkyle en $C_1$ à $C_6$), –CO–$NH_2$, –CO–NH-(alkyle en $C_1$ à $C_6$) et –CO-alkyle sur l'atome d'azote du noyau, avec 1 à 3 hétéroatomes identiques ou différents dans le groupe comprenant l'oxygène, le soufre ou l'azote,

ou bien lorsque n est égal à 1,

Z est un groupe cycloalkyle de 3 à 7 atomes de carbone dans le noyau ou un groupe furyle, pyryle, 1-méthylpyrryl-2 ou -3, thiényle, oxazolyle (lié en position 2, 4 ou 5), thiazolyle (lié en position 2, 4 ou 5), isoxazolyle (lié en position 3, 4 ou 5), isothiazolyle (lié en position 3, 4 ou 5), 1,2,5-oxadiazolyle-3, 1,2,5-thiadiazolyle-3, 1,3,4-oxadiazolyle-2, 1,3,4-thiadiazolyle-2, 1,2,4-oxadiazolyle (lié en position 3 ou 5), 1,2,4-thiadiazolyle (lié en position 3 ou 5), pyrazolyle (lié en position 3, 4 ou 5), 1,2,4-triazolyle (lié en position 3 ou 5), tétrazolyle, pyridyle (lié en position 2, 3 ou 4), pyridazinyle (lié en position 3 ou 4), pyrimidinyle (lié en position 2, 4 ou 5), α-pyronyle (lié en position 3, 4, 5 ou 6), γ-pyronyle (lié en position 2 ou 3) et benzothiazolyle-2, Z pouvant le cas échéant porter un, deux ou trois substituants alkyle en $C_1$ à $C_6$, alkylidène en $C_1$ à $C_6$, vinyle, propényle, allyle, isopropényle, (alkoxy en $C_1$ à $C_6$)méthyle, (alkylthio en $C_1$ à $C_6$)méthyle, trifluorométhyle, hydroxyméthyle, formyle, alcanoyle en $C_1$ à $C_6$, (alcanoyloxy en $C_1$ à $C_6$)méthyle, benzyle, phényle, cyanométhyle, $CH_3$–NH–CO–$CH_2$–, $(CH_3)_2N$–CO–$CH_2$–, (alkoxy en $C_1$ à $C_6$)carbonyle, carboxy, cyano, hydroxy, alcanoyloxy en $C_1$ à $C_6$, alkoxy en $C_1$ à $C_6$, benzyloxy, halogéno, mercapto, alkylthio en $C_1$ à $C_6$, alkylsulfinyle en $C_1$ à $C_6$, alkylsulfonyle en $C_1$ à $C_6$, $CH_3$–CO–NH–, $CH_3$–CO–N($CH_3$)–, $CH_3$–$SO_2$–NH– et $CH_3$–$SO_2$–N($CH_3$)–,

B est un groupe phényle ou cyclohexadiényle éventuellement substitué par un radical halogéno, hydroxy, alkyle ayant 1 à 6 atomes de carbone, cyano et méthylsulfonyle, ou un reste hétérocyclique du groupe comprenant les restes pyrazolyle, imidazolyle, oxazolyle, oxadiazolyle, 2-amino- et 2-oxo-$\Delta^4$-thiazolinyle, tétrazolyle, sydnonyle, furyle, thiényle, pyrrolyle, thiazolyle, isothiazolyle, isoxazolyle et thiadiazolyle, le reste hétérocyclique pouvant porter un ou plusieurs substituants halogéno, alkyle ayant 1 à 6 atomes de carbone, cyano, sulfo et méthylsulfonyle,

T est un groupe (alkyle en $C_1$ à $C_4$)–CO–O–, pyridinium, aminopyridinium, carbamoylpyridinium, carbamoyloxy, le groupe –S-phényle, qui peut être substitué par un radical halogéno, amino, alkylamino en $C_1$ à $C_6$, di(alkyle en $C_1$ à $C_6$)amino, alkyle en $C_1$ à $C_6$, cycloalkyle de 3 à 7 atomes de carbone, alkyloxy en $C_1$ à $C_6$, trifluorométhyle, phényle, benzyle et acylamino ayant 2 à 5 atomes de carbone, ou bien le groupe –S-Het dans lequel Het représente un noyau hétérocyclique pentagonal ou hexagonal classique dans la chimie du β-lactame,

E est l'hydrogène, un groupe ester pharmaceutiquement utilisable, un cation formant un sel ou un groupe protecteur approprié, ou une charge négative, lorsqu'un atome d'azote quaternaire est contenu dans T, les composés de formule I pouvant exister par rapport au centre de chiralité Ċ avec les deux configurations R et S possibles ainsi que comme mélanges des diastéréoisomères résultants, et les composés de formule I, au cas où Z représente le groupe

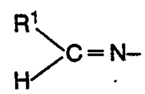

pouvant exister relativement au groupe imino tant sous la forme syn que sous la forme anti et les composés de formule I pouvant exister également sous les différentes formes hydratées, et les sels non toxiques pharmaceutiquement acceptables de ces composés.

2. Composés de formule I suivant la revendication 1, dans laquelle:

R est un groupe méthoxy,

Z est le groupe

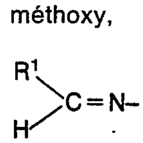

où

$R^1$ représente un groupe phényle éventuellement substitué par un halogène, un groupe alkyle ayant 1 à 4 atomes de carbone, un groupe alkoxy ayant 1 à 4 atomes de carbone, un groupe nitro, 1 ou 2 groupes hydroxy, un groupe S-alkyle ayant 1 à 4 atomes de carbone ou un groupe alkylsulfonyle ayant 1 à 4 atomes de carbone, ou un groupe $CH_3OOC$–, ou un groupe furyle ou thiényle éventuellement substitué par un halogène, un groupe alkyle ou alkoxycarbonyle, ayant 1 à 4 atomes de carbone, de préférence en position 4 ou 5, le noyau furyle et thiényle étant de préférence lié en position 2 et 3; ou un groupe pyridyle; ou bien

Z est un groupe cyclopropyle, furyle, pyridyle, thiényle, benzothiazolyle-2 ou 1,3,4-thiadiazolyle-2, qui peut être substitué en position 5 par un reste sec.-butyle, trifluorométhyle, méthylthio, isopropylthio ou méthylsulfonyle,

B est un groupe phényle, hydroxyphényle ou cyclohexadiényle ou un groupe furyle, thiényle, pyrrolyle, imidazolyle, thiazolyle, isothiazolyle, isoxazolyle, thiadiazolyle, et

n a la valeur 1 ou 2,

T est un groupe $-OCOCH_3$, un groupe thiadiazolylthio ou tétrazolylthio éventuellement substitué par un radical alkyle ayant 1 à 4 atomes de carbone ou $CF_3$ ou $CH_2COOH$, un groupe pyridinium, aminopyridinium, carbamoylpyridinium ou carbamoyloxy ou un groupe 1,2,5,6-tétrahydro-2-méthyl-5,6-dioxo-as-triazine-3-yl-thio, et

Ċ présente la configuration D = R.

3. Procédé de production d'antibiotiques du type β-lactames de formule I

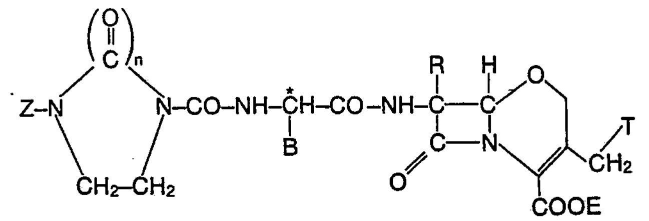

(I)

dans laquelle

R est l'hydrogène ou le groupe méthoxy,

n a les valeurs 1 et 2,

Z représente le groupe

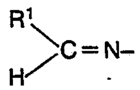

dans lequel $R^1$ représente un groupe aryle portant éventuellement un ou plusieurs substituants identiques ou différents du groupe comprenant les restes halogéno, amino, mono-alkylamino en $C_1$ à $C_6$, di(alkyle en $C_1$ à $C_6$)amino, pyrrolidyle, pipéridyle, HCO–NH–, (alkyle en $C_1$ à $C_6$)–CO–NH–, H–CO–N(alkyle en $C_1$ à $C_6$)–, (alkyle en $C_1$ à $C_6$)–CO–N(alkyle en $C_1$ à $C_6$)–, (alkyle en $C_1$ à $C_6$)$_2$C=N–, (alkyle en $C_1$ à $C_6$)–$SO_2$–NH–, HO–$SO_2$–NH–, HO–$SO_2$–N(alkyle en $C_1$ à $C_6$)–, amidino, (alkyle en $C_1$ à $C_6$)$_2$–N–CH=N–,

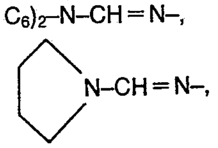

guanido, nitro, azido, hydroxy, alkyloxy en $C_1$ à $C_6$, H–CO–O–, (alkyle en $C_1$ à $C_6$)–CO–O–, (alkyle en $C_1$ à $C_6$)–O–CO–O–, $H_2N$–CO–O–, (alkyle en $C_1$ à $C_6$)–NH–CO–O–, (alkyle en $C_1$ à $C_6$)$_2$N–CO–O–,

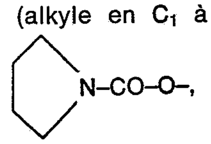

$H_2N$–$SO_2$–O–, (alkyle en $C_1$ à $C_6$)–NH–$SO_2$–O–, (alkyle en $C_1$ à $C_6$)$_2$N–$SO_2$–O–, HOOC–, $H_2N$–CO–, (alkyle en $C_1$ à $C_6$)$_2$N–CO–, OHC–, HO–$SO_2$–O–, HS–, (alkyle en $C_1$ à $C_6$)–S–, (alkyle en $C_1$ à $C_6$)–$\underset{\underset{O}{\|}}{S}$–, $HO_3S$–, (alkyle en $C_1$ à $C_6$)–$SO_2$–, $H_2N$–$SO_2$–, (alkyle en $C_1$ à $C_6$)–NH–$SO_2$–, (alkyle en $C_1$ à $C_6$)$_2$–N–$SO_2$–, HO–$SO_2$–S–, alkyle à chaîne droite ou à chaîne ramifiée ayant 1 à 6 atomes de carbone, phényle ou phénoxy, avec 6 à 10 atomes de carbone dans la partie aryle, ou des noyaux pentagonaux à heptagonaux hétéro-paraffiniques, hétéro-aromatiques ou hétéro-oléfiniques éventuellement substitués par un ou plusieurs restes identiques ou différents dans le groupe comprenant les restes alkyle en $C_1$ à $C_6$, trifluorométhyle, halogéno, amino, alkylamino en $C_1$ à $C_6$, di(alkyle en $C_1$ à $C_6$)amino, formylamino, acétylamino, $CH_3$–O–CO–NH–, –$C_2H_5$O–CO–NH–, $CH_3$–$SO_2$–NH–, hydroxy, méthoxy, éthoxy, méthylthio, éthylthio, $CH_3$–$SO_2$–, $CH_3$–SO–, HOOC–, $HO_3S$–, HCO–, (alkyle en $C_1$ à $C_6$)–CO–, (alkyle en $C_1$ à $C_6$)–O–CO– et CN sur le carbone du noyau ou le cas échéant substitués par des restes du groupe comprenant les restes alkyle en $C_1$ à $C_6$, –C≡N, –CHO, –COO– (alkyle en $C_1$ à $C_6$), –CO–$NH_2$, –CO–NH–(alkyle en $C_1$ à $C_6$) et –CO-alkyle sur l'atome d'azote du noyau, avec 1 à 3 hétéroatomes identiques ou différents dans le groupe comprenant l'oxygène, le soufre ou l'azote,

ou bien lorsque n est égal à 1,

Z est un groupe cycloalkyle de 3 à 7 atomes de carbone dans le noyau ou un groupe furyle, pyryle, 1-méthylpyryl-2 ou -3, thiényle, oxazolyle (lié en position 2, 4 ou 5), thiazolyle (lié en position 2, 4 ou 5) isoxazolyle (lié en position 3, 4 ou 5), isothiazolyle (lié en position 3, 4 ou 5), 1,2,5-oxadiazolyle-3, 1,2,5-thiadiazolyle-3, 1,3,4-oxadiazolyle-2, 1,3,4-thiadiazolyle-2, 1,2,4-oxadiazolyle (lié en position 3 ou 5), 1,2,4-thiadiazolyle (lié en position 3 ou 5), pyrazolyle (lié en position 3, 4 ou 5), 1,2,4-triazolyle (lié en position 3 ou 5), tétrazolyle, pyridyle (lié en position 2, 3 ou 4), pyridazinyle (lié en position 3 ou 4), pyrimidinyle (lié en position 2, 4 ou 5), α-pyronyle (lié en position 3, 4, 5 ou 6), γ-pyronyle (lié en position 2 ou 3) et benzothiazolyle-2, Z pouvant le cas échéant porter un, deux ou trois substituants alkyle en $C_1$ à $C_6$, alkylidène en $C_1$ à $C_6$, vinyle, propényle, allyle, isopropényle, (alkoxy en $C_1$ à $C_6$)méthyle, (alkylthio en $C_1$ à $C_6$)méthyle, trifluorométhyle, hydroxyméthyle, for-

myle, alcanoyle en $C_1$ à $C_6$, (alcanoyloxy en $C_1$ à $C_6$)méthyle, benzyle, phényle, cyanométhyle, $CH_3$–NH–CO–$CH_2$–, $(CH_3)_2$N–CO–$CH_2$–, (alkoxy en $C_1$ à $C_6$)carbonyle, carboxy, cyano, hydroxy, alcanoyloxy en $C_1$ à $C_6$, alkoxy en $C_1$ à $C_6$, benzyloxy, halogéno, mercapto, alkylthio en $C_1$ à $C_6$, alkylsulfinyle en $C_1$ à $C_6$, alkylsulfonyle en $C_1$ à $C_6$, $CH_3$–CO–NH–, $CH_3$–CO–N($CH_3$)–, $CH_3$–$SO_2$–NH– et $CH_3$–$SO_2$–N–,
$CH_3$

B est un groupe phényle ou cyclohexadiényle éventuellement substitué par un radical halogéno, hydroxy, alkyle ayant 1 à 6 atomes de carbone, cyano et méthylsulfonyle, ou un reste hétérocyclique du groupe comprenant les restes pyrazolyle, imidazolyle, oxazolyle, oxadiazolyle, 2-amino- et 2-oxo-$\Delta^4$-thiazolinyle, tétrazolyle, sydnonyle, ainsi que furyle, thiényle, pyrrolyle, thiazolyle, isothiazolyle, isoxazolyle et thiadiazolyle, le reste hétérocyclique pouvant porter un ou plusieurs substituants halogéno, alkyle ayant 1 à 6 atomes de carbone, cyano, sulfo et méthylsulfonyle,

T est un groupe (alkyle en $C_1$ à $C_4$)–CO–O, pyridinium, aminopyridinium, carbamoylpyridinium, carbamoyloxy, le groupe –S-phényle, qui peut être substitué par un radical halogéno, amino, alkylamino en $C_1$ à $C_6$, di(alkyle en $C_1$ à $C_6$)amino, alkyle en $C_1$ à $C_6$, cycloalkyle ayant 3 à 7 atomes de carbone, alkyloxy en $C_1$ à $C_6$, trifluorométhyle, phényle, benzyle et acylamino ayant 2 à 5 atomes de carbone, ou bien le groupe –S-Het dans lequel Het représente un noyau hétérocyclique pentagonal ou hexagonal classique dans la chimie du β-lactame,

E est l'hydrogène, un groupe ester pharmaceutiquement utilisable, un cation formant un sel ou un groupe protecteur approprié, ou une charge négative, lorsqu'un atome d'azote quaternaire est contenu dans T, les composés de formule I pouvant exister par rapport au centre de chiralité Ċ avec les deux configurations R et S possibles ainsi que comme mélanges des diastéréoisomères résultants, et les composés de formule I, au cas où Z représente le groupe

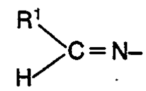

pouvant exister relativement au groupe imino tant sous la forme syn que sous la forme anti et les composés de formule I pouvant exister également sous les différentes formes hydratées, et les sels non toxiques pharmaceutiquement acceptables de ces composés,
caractérisé en ce que:

a) on fait réagir des composés de formule II:

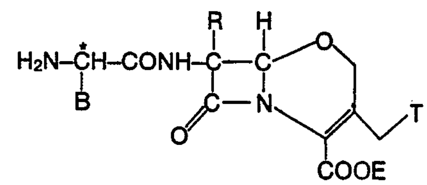
(II)

dans laquelle R, B, Ċ, T et E ont la définition indiquée ci-dessus
avec des composés de formule III

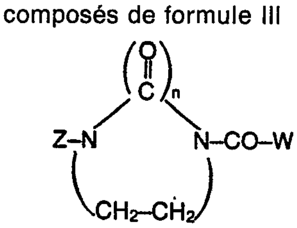
(III)

dans laquelle
Z et n ont la définition indiquée ci-dessus et
W est un halogène, un azide ou un autre groupe partant nucléofuge,
en présence d'un solvant et, le cas échéant, en présence d'un accepteur d'acide, à des températures d'environ −70 °C à environ +50 °C, et on transforme les antibiotiques du type β-lactame obtenus éventuellement en leurs sels non toxiques pharmaceutiquement acceptables, ou on prépare les acides libres à partir des sels obtenus, ou bien

b) on fait réagir des composés de formule IV:

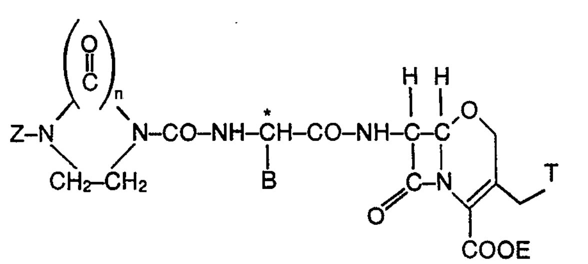
(IV)

dans laquelle Z, n, Ċ, B et T ont la définition indiquée ci-dessus, R représente l'hydrogène et E est un groupe estérifiant aisément éliminable, un groupe acétoxyméthyle, le reste cationique d'une base ou l'hydrogène, avec 1 à 10 équivalents par équivalent d'antibiotique du type β-lactame de formule (IV) d'une base en présence d'un excès de méthanol dans un solvant organique inerte avec addition d'un agent de N-halogènation, et on isole le composé de formule (I) le cas échéant après élimination préalable du groupe protecteur, transformation et un sel ou en un ester pharmaceutiquement utilisable, ou bien

c) on acyle un composé de formule V:

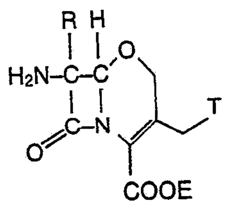

(V)

dans laquelle R, T et E ont la définition indiquée ci-dessus, avec un agent acylant de formule VI

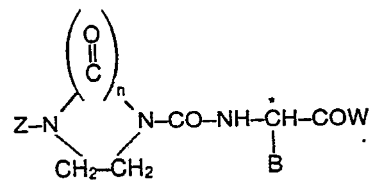

(VI)

dans laquelle Z, n, Ċ et B ont la définition indiquée ci-dessus et W est un groupe fonctionnel réactif, ou bien

d) on fait réagir un composé de formule VIII

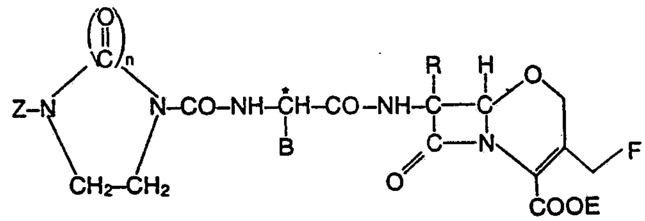

(VIII)

dans laquelle Z, n, Ċ, B, R et E ont la définition indiquée ci-dessus et F représente un groupe éliminable, avec une substance appropriée de formule générale

$R^2H$ (IX)

ou avec une amine tertiaire, de manière à produire les composés de formule (I) mentionnés ci-dessus.

4. Médicament caractérisé par une teneur en au moins un antibiotique du type β-lactame suivant les revendications 1 ou 2.

5. Utilisation de composés de formule (I) suivant la revendication 1 pour la préparation de médicaments.

6. Les composés de formules: